# EUROPEAN PATENT APPLICATION

(11) **EP 4 458 810 A1**
(43) Date of publication of application: **06.11.2024**
(21) Application number: 22916105.4
(22) Date of filing: 27.12.2022
(51) Int. Cl.: C07D 231/56

(54) **INDAZOLE COMPOUND AND PHARMACEUTICAL**

(30) Priority: 28.12.2021 JP 2021215221
(71) Applicant: Nippon Shinyaku Co., Ltd., Kyoto-shi Kyoto 601-8550 (JP)
(72) Inventor: HASHIMOTO, Kosuke, Kyoto-shi, Kyoto 601-8550 (JP); TAKITA, Hirofumi, Kyoto-shi, Kyoto 601-8550 (JP); KAKUTANI, Mai, Kyoto-shi, Kyoto 601-8550 (JP); YAMAGUCHI, Hiroshi, Kyoto-shi, Kyoto 601-8550 (JP); FUKUI, Tomomi, Kyoto-shi, Kyoto 601-8550 (JP)
(74) Representative: Vossius & Partner Patentanwälte Rechtsanwälte mbB
(86) International application number: PCT/JP2022/048213
(87) International publication number: WO 2023/127883

(57) **Abstract**

The present invention provides compounds indicated by general formula [1] and having mPGES-1 inhibitory activity, a pharmaceutically acceptable salt thereof, or a solvate thereof (in the formula, R¹ and R² each independently indicate hydrogen, halogen or substitutable alkyl, R³ indicates -SO₂ R⁵ or -COR⁵, R⁴ indicates substitutable alkyl, cycloalkyl, aryl, heteroaryl, saturated heterocyclic group or arylalkyl, and R⁵ indicates a substitutable alkyl, cycloalkyl, aryl, heteroaryl, saturated heterocyclic group, alkynyl or arylalkyl).

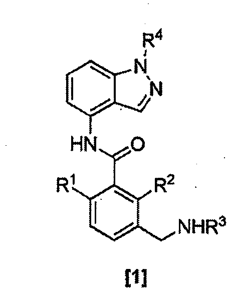

## Description

### [TECHNICAL FIELD]

The present invention relates to a pharmaceutical composition comprising a novel indazole compound or a pharmaceutically acceptable salts thereof, or solvates thereof as an active ingredient.

### [BACKGROUND ART]

Prostaglandins (PGs) are produced in large amounts at the site of inflammation and are involved in the development of inflammation. The production of prostaglandins begins with the release of arachidonic acid from membrane glycerophospholipids by phospholipase A2, which is then converted to prostaglandin H2 (PGH2) by cyclooxygenase (COX). PGH2 is converted to prostaglandins including prostaglandin E2 (PGE2), prostaglandin F2α (PGF2α), prostaglandin D2 (PGD2), prostaglandin I2 (PGI2) and thromboxane A2 (TXA2). These prostaglandins are known to have various physiological or pathophysiological activities, including inflammation-inducing effects. In particular, PGE2 is known to induce inflammation in acute and chronic inflammation, as well as fever and hyperalgesia. PGE2 is also known to be involved in the regulation of urine volume and reabsorption of sodium in kidney (e.g., see Non-Patent Document 1). Nonsteroidal anti-inflammatory drugs (NSAIDs) and selective COX-2 inhibitors exhibit anti-inflammatory and anti-diuretic effects by suppressing the production of PGE2 based on COX-1 and/or COX-2 inhibition. PGE2 synthase (PGES) plays the role in the final step of the synthesis pathway of the inflammatory mediator PGE2. PGES is currently known to include three subtypes: membrane-bound prostaglandin E synthase-1 (mPGES-1) (e.g., see Non-Patent Document 2), mPGES-2 (e.g., see Non-Patent Document 3), and cytosolic PGES (cPGES) (e.g., see Non-Patent Document 4). Among them, mPGES-1, as same to COX-2, is mainly induced during inflammation and is closely involved in production of PGE2 in inflammatory lesions, and especially it is known to be constantly expressed in kidney. In contrast, cPGES is a constitutive PGES that mainly coexists with COX-1 and is involved in the constant production of PGE2 (e.g., see Non-Patent Document 5). mPGES-2 has been shown to be involved in both COX. Studies for mPGES-1 deficient mice have suggested that mPGES-1 is involved in the pathogenesis of various inflammatory models, such as the acetate rising model (e.g., see Non-Patent Document 6), arthritis models (e.g., see Non-Patent Documents 6 and 7), multiple sclerosis models (e.g., see Non-Patent Document 8), and fever models (e.g., see Non-Patent Document 9). Also, it has been suggested that mPGES-1 is involved in the pathogenesis of disease progression in models of polyuria. In addition, deficiency of mPGES-1 has been reported to decrease urine volume in polyuria models, suggesting the involvement of mPGES-1 in the pathogenesis of polyuria (e.g., see Non-Patent Document 10). It is thought that the inhibition of COX-2-depending PGI2 production may contribute to the increased risk of cardiovascular events due to COX-2 inhibitors (e.g., see Non-Patent Document 11). In contrast, mPGES-1 inhibitors do not inhibit PGI2 production and thus do not increase the risk of cardiovascular events which are problematic with COX-2 inhibitors (e.g., see Non-Patent Document 12). Furthermore, mPGES-1 inhibitors inhibit production of PGE2 which is involved in inflammation, while the production of PGI2, which has anti-inflammatory properties (e.g., see Non-Patent Document 13), is increased by them (e.g., see Non-Patent Document 14). Thus, mPGES-1 inhibitors are expected to have strong anti-inflammatory effects. Additionally, since PGE2 promotes cell proliferation (e.g., see Non-Patent Document 15) and PGI2 suppresses cell proliferation (e.g., see Non-Patent Document 16), mPGES-1 inhibitors are also expected to have a strong inhibitory effect on cell proliferation. Therefore, agents that exhibit mPGES-1 inhibitory activity and can selectively inhibit production of PGE2 can be beneficial in the treatment or prevention of diseases involving mPGES-1, such as inflammatory diseases, proliferative diseases, and dysuria diseases.
Although various compounds showing mPGES-1 inhibitory activity are known (e.g., see Patent Documents 1 to 13), compounds showing such activity and having an indazole skeleton are not known.

### [PRIOR ART DOCUMENTS]

### [PATENT DOCUMENTS]

[Patent Document 1] WO2011/048004
[Patent Document 2] WO2012/087771
[Patent Document 3] WO2012/022792
[Patent Document 4] WO2012/161965
[Patent Document 5] WO2013/186692
[Patent Document 6] WO2013/072825
[Patent Document 7] WO2013/118071
[Patent Document 8] WO2014/167444
[Patent Document 9] WO2015/059618
[Patent Document 10] WO2016/199104
[Patent Document 11] WO2016/069374
[Patent Document 12] WO2016/069376
[Patent Document 13] WO2016/097013

### [NON-PATENT DOCUMENTS]

[Non-patent Document 1] Jabs et al. Am. J. Physiol, 1989, Sep 257, F424-30.
[Non-patent Document 2] Jakobsson et al. Proc. Natl. Acad. Sci. USA, 1999, 96, 7220-7225.
[Non-patent Document 3] Tanikawa et al. Biochem. Biophys. Res. Commun. 2002, 291, 884-889.
[Non-patent Document 4] Tanioka et al. J. Biol. Chem. 2000, 275, 32775-32782.
[Non-patent Document 5] Murakami et al. J. Biol. Chem. 2000, 275, 32783-32792.
[Non-patent Document 6] Kamei et al. J. Biol. Chem. 2004, 279, 33684-33695.1.
[Non-patent Document 7] Kojima et al. J. Immunol. 2008, 108, 3861-3868.
[Non-patent Document 8] Kimura et al. Proc. Natl. Acad. Sci. USA, 2000, 106, 21807-21812.
[Non-patent Document 9] Engblom et al. Nat. Neurosci. 2003, 6, 1137-1138.
[Non-patent Document 10] Soodvilai et al. Am. J Physiol Renal Physiol. 2009, 296, F1129-35.
[Non-patent Document 11] Foudi et al. Cardiovasc. Res. 2009, 81, 269-277.
[Non-patent Document 12] Cheng et al. J. Clin. Invest. 2006, 116, 1391-1399.
[Non-patent Document 13] J. Immunol. 2007, 178, 702-710.
[Non-patent Document 14] J. Biol. Chem. 2005, 280, 16579-16585.
[Non-patent Document 15] J. Clin. Endocrinol. Metab., 2003, 88, 4481-4487.
[Non-patent Document 16] Am. J. Respir. Cell Mol. Biol. 2002, 26, 194-201.
[Non-patent Document 17] J. Biol. Chem. 2004, 279, 12647-12658.
[Non-patent Document 18] Biomed. Pharmacother. 2011, 65, 77-84.
[Non-patent Document 19] Acta. Med. Okayama, 2008, 62, 373-378.
[Non-patent Document 20] Proc. Natl. Acad. Sci. USA, 2009, 106, 21807-21812.
[Non-patent Document 21] J. Biol. Chem., 279, 33684-33695.
[Non-patent Document 22] J. Immunol. 2008, 180, 8361-8368.
[Non-patent Document 23] Glia, 2011, 59, 208-218.
[Non-patent Document 24] J. Burn. Care. Res. 2011, 32, 79-90.
[Non-patent Document 25] J. Biol. Chem. 2003, 278, 19396-19405.
[Non-patent Document 26] Oncogene, 2012, 31, 2943-2952.
[Non-patent Document 27] Cancer Res. 2008, 68, 3251-3259.
[Non-patent Document 28] Proc. Natl. Acad. Sci. USA, 2006, 103, 14507-14512.
[Non-patent Document 29] Proc. Natl. Acad. Sci. USA, 2006, 103, 11790-11795.
[Non-patent Document 30] J. Rheumatol. 2005, 32, 887-895.
[Non-patent Document 31] J. Pharmacol. Exp. Ther. 2008, 326, 754-763.
[Non-patent Document 32] Kidney Int., 2011, 79, 77-88.
[Non-patent Document 33] J. Immunol. 2012, 188, 4093-4102.
[Non-patent Document 34] Am. J. Physiol. Lung Cell Mol. Physiol. 2004, 287, L981-L991.
[Non-patent Document 35] Am. J. Physiol. Lung Cell Mol. Physiol. 2005, 288, L1010-L1016.
[Non-patent Document 36] J. Immunol. 2012, 188, 4093-4102.
[Non-patent Document 37] Oncogene, 2012, 31, 2943-2952.
[Non-patent Document 38] Arthritis Res. Ther., 2011, 13, R6.

### [SUMMARY OF INVENTION]

### [PROBLEMS TO BE SOLVED BY THE INVENTION]

The main object of the present invention is providing a novel indazole derivative or pharmaceutically acceptable salts thereof. It is also the object of the present invention to provide a pharmaceutical composition comprising such an indazole derivative or pharmaceutically acceptable salts thereof as active ingredients.

### [MEANS OF SOLVING THE PROBLEMS]

The inventors found that novel indazole derivatives, or pharmaceutically acceptable salts thereof, have excellent mPGES-1 inhibitory activity, as described below.

In other words, the present invention includes the following embodiments.
(I) A compound represented by the general formula [1]: 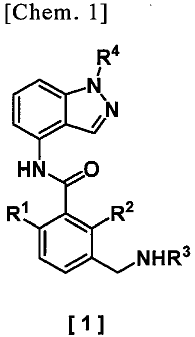 wherein,
   R¹ and R² each independently represent hydrogen, halogen, or optionally substituted alkyl;
   R³ represents -SO₂R⁵ or -COR⁵;
   R⁴ represents optionally substituted alkyl, optionally substituted cycloalkyl, optionally substituted aryl, optionally substituted heteroaryl, optionally substituted saturated heterocyclic group, or optionally substituted arylalkyl; and
   R⁵ represents optionally substituted alkyl, optionally substituted cycloalkyl, optionally substituted aryl, optionally substituted heteroaryl, optionally substituted saturated heterocyclic group, optionally substituted alkynyl, or optionally substituted arylalkyl;
   wherein said heteroaryl or saturated heterocyclic groups is bound at a carbon atom on the ring, or a pharmaceutically acceptable salt thereof, or a solvate thereof.
(II) The compound according to (I),
   wherein
   the alkyl in R¹ and R² is optionally substituted by 1 to 3 halogens;
   the heteroaryl in R⁴ is optionally substituted by 1 to 3 groups independently selected from the group consisting of:
      (1) alkoxy optionally substituted by 1 to 3 halogens,
      (2) cyano,
      (3) halogen,
      (4) alkyl optionally substituted by (i) 1 to 3 halogens, (ii) hydroxy or (iii) alkoxy, and
      (5) cycloalkyl;

   the saturated heterocyclic group in R⁴ is optionally substituted by 1 to 3 groups independently selected from the group consisting of alkyl, alkoxycarbonyl and alkylcarbonyl;
   the alkyl in R⁴ is optionally substituted by a saturated heterocyclic group or cycloalkyl;
   the aryl in R⁴ is optionally substituted by 1 to 3 groups independently selected from the group consisting of:
      (1) alkoxy optionally substituted by 1 to 3 halogens,
      (2) cyano,
      (3) halogen,
      (4) alkyl optionally substituted by (i) 1 to 3 halogens, (ii) hydroxy or (iii) alkoxy, and
      (5) cycloalkyl;
   the arylalkyl in R⁴ is optionally substituted by alkyl or alkoxy optionally substituted by 1 to 3 halogens;
   the saturated heterocyclic group in R⁵ is optionally substituted by 1 to 3 groups independently selected from the group consisting of alkyl and alkoxycarbonyl;
   the alkyl in R⁵ is optionally substituted by 1 to 3 groups independently selected from the group consisting of hydroxy, alkoxy, cycloalkyl, monoalkylamino, dialkylamino and halogen; and
   the cycloalkyl in R⁵ is optionally substituted by 1 to 3 groups independently selected from the group consisting of:
      (1) hydroxy,
      (2) cyano, and
      (3) alkyl optionally substituted by 1 to 3 halogens,
   or a pharmaceutically acceptable salt thereof, or a solvate thereof.
(III) The compound according to (I) or (II),
   wherein
   the aryl in R⁴ is phenyl optionally substituted by 1 to 3 groups independently selected from the group consisting of:
      (1) alkoxy optionally substituted by 1 to 3 halogens,
      (2) cyano,
      (3) halogen,
      (4) alkyl optionally substituted by (i) 1 to 3 halogens, (ii) hydroxy or (iii) alkoxy, and
      (5) cycloalkyl;
   the heteroaryl in R⁴ is imidazolyl, thiazolyl, pyridyl, pyridazinyl or pyrimidinyl, each of which is optionally substituted by 1 to 3 groups independently selected from the group consisting of:
      (1) alkoxy optionally substituted by 1 to 3 halogens,
      (2) cyano,
      (3) halogen,
      (4) alkyl optionally substituted by (i) 1 to 3 halogens, (ii) hydroxy or (iii) alkoxy, and
      (5) cycloalkyl; and
   the saturated heterocyclic group in R⁴ is piperidinyl or tetrahydropyranyl optionally substituted by alkyl, alkoxycarbonyl or alkylcarbonyl,
   or a pharmaceutically acceptable salt thereof, or a solvate thereof.
(IV) The compound according to any one of (I) to (III),
   wherein
   the aryl in R⁵ is phenyl;
   the heteroaryl in R⁵ is furyl; and
   the saturated heterocyclic group in R⁵ is piperidinyl, tetrahydrofuryl or tetrahydropyranyl, each of which is optionally substituted by 1 to 3 groups independently selected from the group consisting of alkyl and alkoxycarbonyl,
   or a pharmaceutically acceptable salt thereof, or a solvate thereof.
(V) The compound according to any one of (I) to (IV), wherein R⁴ is alkyl having 1 to 6 carbons, which is optionally substituted by saturated heterocyclic group or cycloalkyl, or a pharmaceutically acceptable salt, or a solvate thereof
(VI) The compound according to any one of (I) to (IV), wherein R⁴ is cycloalkyl having 3 to 10 carbons, or a pharmaceutically acceptable salt thereof, or a solvate thereof.
(VII) The compound according to any one of (I) to (IV),
   wherein
   R⁴ is phenyl optionally substituted by 1 to 3 groups independently selected from the group consisting of:
   (1) alkoxy optionally substituted by 1 to 3 halogens,
   (2) cyano,
   (3) halogen,
   (4) alkyl optionally substituted by (i) 1 to 3 halogens, (ii) hydroxy or (iii) alkoxy, and
   (5) cycloalkyl,
   or a pharmaceutically acceptable salt thereof, or a solvate thereof.
(VIII) The compound according to any one of (I) to (IV),
   wherein
   R⁴ is imidazolyl, thiazolyl, pyridyl, pyridazinyl or pyrimidinyl, each of which is optionally substituted by 1 to 3 groups independently selected from the group consisting of:
   (1) alkoxy optionally substituted by 1 to 3 halogens,
   (2) cyano,
   (3) halogen,
   (4) alkyl optionally substituted by (i) 1 to 3 halogens, (ii) hydroxy or (iii) alkoxy, and
   (5) cycloalkyl,
   or a pharmaceutically acceptable salt thereof, or a solvate thereof.
(IX) The compound according to any one of (I) to (IV), wherein R⁴ is piperidinyl or tetrahydropyranyl, each of which is optionally substituted by 1 to 3 groups independently selected from the group consisting of alkyl, alkoxycarbonyl and alkylcarbonyl, or a pharmaceutically acceptable salt thereof, or a solvate thereof.
(X) The compound according to any one of (I) to (IV), wherein R⁴ is phenylalkyl, optionally substituted by alkyl or alkoxy having 1 to 6 carbons, each of which is optionally substituted with 1 to 3 halogens, or a pharmaceutically acceptable salt thereof, or a solvate thereof.
(XI) The compound according to any one of (I) to (X), wherein R⁵ is alkyl having 1 to 6 carbons, optionally substituted by 1 to 3 groups independently selected from the group consisting of hydroxy, alkoxy, cycloalkyl, monoalkylamino, dialkylamino, and halogen, or a pharmaceutically acceptable salt thereof, or a solvate thereof.
(XII) The compound according to any one of (I) to (X), wherein R⁵ is cycloalkyl having 3 to 10 carbons optionally substituted by 1 to 3 groups independently selected from the group consisting of (1) hydroxy, (2) cyano and (3) alkyl optionally substituted by 1 to 3 halogens, or a pharmaceutically acceptable salt thereof, or a solvate thereof.
(XIII) The compound according to any one of (I) to (X), wherein R⁵ is optionally substituted phenyl, or a pharmaceutically acceptable salt thereof, or a solvate thereof.
(XIV) The compound according to any one of (I) to (X), wherein R⁵ is optionally substituted furyl, or a pharmaceutically acceptable salt thereof, or a solvate thereof.
(XV) The compound according to any one of (I) to (X), wherein R⁵ is piperidinyl, tetrahydrofuryl or tetrahydropyranyl, each of which is optionally substituted by 1 to 3 groups independently selected from the group consisting of alkyl and alkoxycarbonyl, or a pharmaceutically acceptable salt thereof, or a solvate thereof.
(XVI) The compound according to any one of (I) to (X), wherein R⁵ is optionally substituted alkynyl having 2 to 6 carbons, or a pharmaceutically acceptable salt thereof, or a solvate thereof.
(XVII) The compound according to any one of (I) to (X), wherein R⁵ is optionally substituted phenylalkyl, or a pharmaceutically acceptable salt thereof, or a solvate thereof.
(XVIII) The compound according to any one of (I) to (XVII), wherein R¹ is halogen or alkyl having 1 to 6 carbons optionally substituted by 1 to 3 halogens, or a pharmaceutically acceptable salt thereof, or a solvate thereof.
(XIX) The compound according to any one of (I) to (XVIII), wherein R² is hydrogen or halogen, or a pharmaceutically acceptable salt thereof, or a solvate thereof.
(XX) The compounds of Examples 1-285 or pharmaceutically acceptable salts thereof, or solvates thereof.
(XXI) A pharmaceutical composition comprising the compound according to any one of (I) to (XX) or a pharmaceutically acceptable salt thereof, or a solvate thereof.
(XXII) An inhibitory agent for mPGES-1, comprising the compound according to any one of (I) to (XX) or a pharmaceutically acceptable salt thereof, or a solvate thereof.
(XXIII) A method for inhibiting mPGES-1 activity, comprising administering to a subject in need thereof the compound according to any one of (I) to (XX) or a pharmaceutically acceptable salt thereof, or a solvate thereof.
(XXIV) The compound according to any one of (I) to (XX) or a pharmaceutically acceptable salt thereof, for use in inhibiting mPGES-1.
(XXV) Use of the compound according to any one of (I) to (XX) or a pharmaceutically acceptable salt thereof in the manufacture of an inhibitory agent for mPGES-1.
(XXVI) An inhibitory agent for production of PGE2, comprising the compound according to any one of (I) to (XX) or a pharmaceutically acceptable salt thereof, or a solvate thereof.
(XXVII) A method for inhibiting production of PGE2, comprising administering to a subject in need thereof the compound according to any one of (I) to (XX) or a pharmaceutically acceptable salt thereof, or a solvate thereof.
(XXVIII) The compound according to any one of (I) to (XX) or a pharmaceutically acceptable salt thereof, for use in inhibiting production of PGE2.
(XXIX) Use of the compound according to any one of (I) to (XX) or a pharmaceutically acceptable salt thereof in the manufacture of an inhibitory agent for production of PGE2.
(XXX) A preventive or therapeutic agent for a disease involving mPGES-1, comprising the compound according to any one of (I) to (XX) or a pharmaceutically acceptable salt thereof, or a solvate thereof.
(XXXI) A method for preventing or treating a disease involving mPGES-1, comprising administering to a subject in need thereof the compound according to any one of (I) to (XX) or a pharmaceutically acceptable salt thereof, or a solvate thereof.
(XXXII) A compound or a pharmaceutically acceptable salt thereof of the present invention for use in preventing or treating a disease involving mPGES-1.
(XXXIII) Use of a compound or a pharmaceutically acceptable salt thereof of the present invention in the manufacture of a preventing or treating agent for a disease involving mPGES-1.
(XXXIV) A preventing or therapeutic agent for a disease involving PGE2, comprising the compound according to any one of (I) to (XX) or a pharmaceutically acceptable salt thereof, or a solvate thereof.
(XXXV) A method for preventing or treating a disease involving PGE2, comprising administering to a subject in need thereof a compound according to any one of (I) to (XX) or a pharmaceutically acceptable salt thereof, or a solvate thereof.
(XXXVI) The compound of any one of (I) to (XX) or a pharmaceutically acceptable salt thereof, for use in the prevention or treatment of a disease involving PGE2.
(XXXVII) Use of the compound according to any one of (I) to (XX) or a pharmaceutically acceptable salt thereof in the manufacture of a preventive or therapeutic agent for a disease involving PGE2.
(XXXVIII) A preventive or therapeutic agent comprising the compound according to any one of (I) to (XX) or a pharmaceutically acceptable salt thereof, for a disease which is expected to be prevented or treated by analgesic, anti-inflammatory or cell proliferation inhibitory effect.
(XXXIX) A method for preventing or treating a disease which is expected to be prevented or treated by analgesic, anti-inflammatory or cell proliferation inhibitory effect, comprising administering the compound according to any one of (I) to (XX) or a pharmaceutically acceptable salt thereof to a subject in need thereof.
(XXXX) The compound according to any one of (I) to (XX) or a pharmaceutically acceptable salt thereof, for use in preventing or treating a disease which is expected to be prevented or treated by analgesic, anti-inflammatory or cell proliferation inhibitory effect.
(XXXXI) Use of the compound according to any one of (I) to (XX) or a pharmaceutically acceptable salt thereof in the manufacture of a preventing or treating agent for a disease which is expected to be prevented or treated by analgesic, anti-inflammatory or cell growth inhibitory effect.
(XXXXII) A preventive or therapeutic agent comprising the compound according to (I) to (XX) or a pharmaceutically acceptable salt thereof, or a solvate thereof, which is for inflammatory bowel disease, irritable bowel syndrome, migraine, headache, lumbago, lumbar spinal stenosis, herniated disc, temporomandibular disorder, cervico-omo-brachial syndrome, cervical spondylosis, endometriosis, adenomyosis, premature labor, threatened premature labor, dysmenorrhea, overactive bladder, nocturia, interstitial cystitis, neurodegenerative disease, psoriasis, rheumatoid arthritis, rheumatic fever, fibromyalgia, neuralgia, complex regional pain syndrome, myofascial disorder, viral infections, bacterial infections, fungal infections, burn injury, postoperative, post-trauma, and post-tooth extraction inflammation and pain, malignant tumor, atherosclerosis, stroke, gout, arthritis, osteoarthritis, juvenile arthritis, tenosynovitis, ligament ossification, systemic lupus erythematosus, vasculitis, pancreatitis, nephritis, conjunctivitis, iritis, scleritis, uveitis, wound healing, dermatitis, eczema, osteoporosis, asthma, chronic obstructive pulmonary disease, pulmonary fibrosis, allergic diseases, familial adenomatous polyposis, scleroderma, bursitis, uterine fibroids, prostatitis, depression, neurogenic bladder, nocturnal polyuria, diurnal polyuria, nocturnal enuresis, central diabetes insipidus, nephrogenic diabetes insipidus, urinary incontinence, prostatic hyperplasia, chronic prostatitis, or pain in cancer.
(XXXXIII) A preventive or therapeutic agent comprising the compound according to (I) to (XX) or a pharmaceutically acceptable salt thereof, or a solvate thereof, which is for nocturnal polyuria, nocturia, overactive bladder, neurogenic bladder, nocturnal enuresis, prostatic hyperplasia, central diabetes insipidus, nephrogenic diabetes insipidus, chronic prostatitis, interstitial cystitis, or urinary incontinence.
(XXXXIV) A method for preventing or treating nocturnal polyuria, nocturia, overactive bladder, neurogenic bladder, nocturnal enuresis, prostatic hyperplasia, central diabetes insipidus, nephrogenic diabetes insipidus, chronic prostatitis, interstitial cystitis, or urinary incontinence, comprising administering to a subject in need thereof, the compound according to any one of (I) to (XX) or a pharmaceutically acceptable salt thereof, or a solvate thereof.
(XXXXV) The compound according to any one of (I) to (XX) or a pharmaceutically acceptable salt thereof for use in preventing or treating nocturnal polyuria, nocturia, overactive bladder, neurogenic bladder, nocturnal enuresis, prostatic hyperplasia, central diabetes insipidus, nephrogenic diabetes insipidus, chronic prostatitis, interstitial cystitis, or urinary incontinence.
(XXXXVI) Use of the compound according to any one of (I) to (XX) or a pharmaceutically acceptable salt thereof in the manufacture of a preventive or therapeutic agent for nocturnal polyuria, nocturia, overactive bladder, neurogenic bladder, nocturnal enuresis, prostatic hyperplasia, central diabetes insipidus, nephrogenic diabetes insipidus, chronic prostatitis, interstitial cystitis, or urinary incontinence.

The detailed definitions of the terms used herein are as follows.

"Halogen" refers to a fluorine atom, a chlorine atom, a bromine atom, or an iodine atom. Particularly, a fluorine atom or a chlorine atom is preferred.

"Alkyl" refers to, for example, a linear or branched alkyl chain having 1 to 8, preferably 1 to 6 carbons. Specific examples of "alkyl" include methyl, ethyl, propyl, isopropyl, butyl, isobutyl, sec-butyl, tert-butyl, pentyl, isopentyl, neopentyl, tert-pentyl, isoamyl, -CH(CH₂CH₃)₂, hexyl, isohexyl, -CH₂CH₂C(CH₃)₃, -CH₂CH(CH₂CH₃)₂, heptyl, isoheptyl, octyl, isooctyl and the like.

Examples of the alkyl moiety of "alkylcarbonyl", "alkoxy", "alkoxycarbonyl", "aralkyl", "monoalkylamino", "dialkylamino" and "arylalkyl" include the same "alkyl" as described above.

"Alkoxy" refers to, for example, a linear or branched alkoxy chain having 1 to 8, preferably 1 to 6 carbons. Specific examples of "alkoxy" include methoxy, ethoxy, n-propoxy, isopropoxy, n-butoxy, isobutoxy, sec-butoxy, tert-butoxy, n-pentyloxy, n-hexyloxy, n-heptyloxy, n-octyloxy and the like.

"Cycloalkyl" refers to, for example, a saturated mono-, bi-, or tricyclic hydrocarbon group having 3 to 10 carbons. Specific examples of "cycloalkyl" include cyclopropyl, cyclobutyl, cyclopentyl, cyclohexyl, cycloheptyl, bicyclo[2.1.0]pentyl, bicyclo[2.2.1]heptyl, bicyclo[2.2.2]octyl and bicyclo[3.2.1]octyl, adamantyl (also referred to as tricyclo[3.3.1.1^{3,7}]decanyl) and the like.

"Alkynyl" refers to, for example, a linear or branched alkynyl chain having 2 to 8 carbons with one or two triple bonds. Specific examples of "alkynyl" include ethynyl, 1-propynyl, 2-propynyl, 1-methyl-2-propynyl, 1-butynyl, 2-butynyl, 3-butynyl, 1-pentynyl, 2-pentynyl, 3-pentynyl, 4-pentynyl, 1-methyl-4-pentynyl and the like. Preferably, alkynyl has 2 to 6, more preferably, 2 to 4 carbons.

"Aryl" refers to, for example, a mono-, bi-, or tricyclic aromatic hydrocarbon having 6 to 14 carbons. Specific examples of "aryl" include phenyl, 1-naphthyl, 2-naphthyl, 1-anthryl, 2-anthryl, 9-anthryl, 1-phenanthryl, 2-phenanthryl, 3-phenanthryl, 4-phenanthryl, 10-phenanthryl and the like. Particularly, phenyl is preferred.

Examples of aryl moiety of "arylalkyl" include the same "aryl" as described above. "Arylalkyl" includes phenylmethyl (benzyl), phenylethyl (phenethyl), phenylpropyl, phenylbutyl, phenylpentyl and the like.

"Heteroaryl" refers to a 1- to 2-ring aromatic heterocyclic group having 1 to 4 heteroatoms selected from nitrogen, sulfur, and oxygen atoms in the ring and consisting of 5 to 10 ring atoms. Specific examples of "heteroaryl" include furyl (e.g., 2-furyl, 3-furyl), thienyl (e.g., 2-thienyl, 3-thienyl), pyrrolyl (e.g., 2-pyrrolyl, 3-pyrrolyl), imidazolyl (e.g., 2-imidazolyl, 4-imidazolyl), pyrazolyl (e.g., 3-pyrazolyl, 4-pyrazolyl), triazolyl (e.g., 1,2,4-triazol-3-yl, 1,2,4-triazol-5-yl, 1,2,3-triazol-4-yl), tetrazolyl (e.g., 5-tetrazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), oxadiazolyl (e.g., 1,3,4-oxadiazol-2-yl), thiazolyl (e.g., 2-thiazolyl, 4-thiazolyl, 5-thiazolyl), thiadiazolyl, isothiazolyl (e.g., 3-isothiazolyl, 4-isothiazolyl, 5-isothiazolyl), pyridyl (e.g., 2-pyridyl, 3-pyridyl, 4-pyridyl), pyridazinyl (e.g., 3-pyridazinyl, 4-pyridazinyl), pyrimidinyl (e.g., 2-pyrimidinyl, 4-pyrimidinyl, 5-pyrimidinyl), pyrazinyl (e.g., 2-pyrazinyl), benzimidazolyl (e.g., 2-benzimidazolyl, 4-benzimidazolyl, 5-benzimidazolyl, 6-benzimidazolyl, 7-benzimidazolyl), indazolyl (e.g., 3-indazolyl, 4-indazolyl, 5-indazolyl, 6-indazolyl, 7-indazolyl), and isoquinolyl (e.g., 1-isoquinolyl, 3-isoquinolyl, 4-isoquinolyl, 5-isoquinolyl, 6-isoquinolyl, 7-isoquinolyl, 8-isoquinolyl). More preferably, the examples include furyl (e.g., 2-furyl, 3-furyl), imidazolyl (e.g., 2-imidazolyl, 4-imidazolyl), thiazolyl (e.g., 2-thiazolyl, 4-thiazolyl, 5-thiazolyl), pyridyl (e.g., 2-pyridyl, 3-pyridyl, 4-pyridyl), pyridazinyl (e.g., 3-pyridazinyl, 4-pyridazinyl), and pyrimidinyl (e.g., 2-pyrimidinyl, 4-pyrimidinyl, 5-pyrimidinyl).

As a heteroaryl in R⁴, imidazolyl, thiazolyl, pyridyl, pyridazinyl and pyrimidinyl are preferred.

As a heteroaryl in R⁵, furyl is preferred.

"Saturated heterocyclic group" refers to, for example, a saturated heterocyclic group consisting of 3 to 8 ring atoms and having 1 to 3 heteroatoms optionally selected from the group consisting of nitrogen, sulfur and oxygen atoms which may be identical or different. Specific examples of "saturated heterocyclic group" include oxiranyl, azetidinyl (e.g., 2-azetidinyl, 3-azetidinyl), pyrrolidinyl (e.g., 2-pyrrolidinyl, 3-pyrrolidinyl), piperidinyl (e.g., 2-piperidinyl, 3-piperidinyl, 4-piperidinyl), piperazinyl (e.g., 2-piperazinyl, 3-piperazinyl), morpholinyl (e.g., 2-morpholinyl, 3-morpholinyl), thiomorpholinyl (e.g., 2-thiomorpholinyl, 3-thiomorpholinyl), tetrahydrofuryl (2-tetrahydrofuryl, 3-tetrahydrofuryl), and tetrahydropyranyl (2-tetrahydropyranyl, 3-tetrahydropyranyl, 4-tetrahydropyranyl). More preferably, the examples include piperidinyl (e.g., 2-piperidinyl, 3-piperidinyl, 4-piperidinyl), tetrahydrofuryl (2-tetrahydrofuryl, 3-tetrahydrofuryl), and tetrahydropyranyl (2-tetrahydropyranyl, 3-tetrahydropyranyl, 4-tetrahydropyranyl).

When R⁴ is alkyl group having 1 to 6 carbons, which is optionally substituted by saturated heterocyclic group or cycloalkyl, said saturated heterocyclic group may be the same as defined for "saturated heterocyclic group" above. Also, said cycloalkyl includes the same as defined for "cycloalkyl" above.

When R⁴ is piperidinyl or tetrahydropyranyl, each of which is optionally substituted by 1 to 3 groups independently selected from the group consisting of alkyl, alkoxycarbonyl and alkylcarbonyl, said alkyl may be the same as defined for "alkyl" above. Also, said alkoxycarbonyl may be the same as defined for "alkoxycarbonyl" above, and said alkylcarbonyl may be the same as defined for "alkylcarbonyl" above.

When R⁵ is alkyl having 1 to 6 carbons optionally substituted by 1 to 3 groups independently selected from the group consisting of hydroxy, alkoxy, cycloalkyl, monoalkylamino, dialkylamino and halogen, said alkoxy may be the same as defined for "alkoxy" above. Also, said cycloalkyl may be the same as defined for "cycloalkyl" above, said monoalkylamino may be the same as defined for "monoalkylamino" above, and said dialkylamino may be the same as defined for "dialkylamino" above.

When R⁵ is piperidinyl, tetrahydrofuryl or tetrahydropyranyl, each of which is optionally substituted by 1 to 3 groups independently selected from the group consisting of alkyl and alkoxycarbonyl, sail alkyl may be the same as defined for "alkyl" above. Also, said alkoxycarbonyl may be the same as defined for "alkoxycarbonyl" above.

It is understood that the present invention encompasses all combinations of substituents described herein. That is, the present invention encompasses any combination of substituents independently selected from the groups listed for each of R¹, R², R³ and R⁴ in the general formula [1] and is not limited to the specific combinations as described herein.

### [EFFECT OF INVENTION]

Compounds of the present invention exhibit mPGES-1 inhibitory activity and can selectively inhibit production of PGE2. Furthermore, the compounds of the present invention inhibit the production of PGE2 in renal collecting duct cells, thereby being able to promote reabsorption of sodium and water from urine to blood. Therefore, the compounds of the present invention are expected to have less side effect such as hyponatremia, since they can exhibit anti-diuretic activity without directly affecting the plasma sodium concentration.

### [DESCRIPTION OF EMBODIMENTS]

The compounds of the present invention can be prepared using known compounds or intermediates that can be easily synthesized from known compounds, for example, according to the methods described below, the examples described below, or known methods. In the manufacture of the compounds of the present invention, if a raw material has a substituent that affects the reaction, the reaction is generally carried out after protecting the raw material with an appropriate protecting group by known methods in advance. The protecting group can be deprotected by known methods after the reaction.

The meanings of abbreviations used herein are as follows:
DMF: N,N-dimethylformamide
DMA: N,N-dimethylacetamide
DME: 1,2-dimethoxyethane
DMSO: dimethyl sulfoxide
NMP: N-methylpyrrolidone
THF: tetrahydrofuran
MeOH: methanol
EtOH: ethanol
CHCl₃: chloroform
CH₂Cl₂: methylene chloride
HPLC: high performance liquid chromatography
MS: mass spectrometry
LCMS: liquid chromatograph mass spectrometry
WSCD: 1-ethyl-3-(3-dimethylaminopropyl)carbodiimide hydrochloride
HBTU: O-(benzotriazol-1-yl)-N,N,N',N'-tetramethyluronium hexafluorophosphate
HATU: 2-(1*H*-7-azabenzotriazol-1-yl)-1,1,3,3-tetramethyluronium hexafluorophosphate
PyBOP: 1*H*-benzotriazol-1-yloxytripyrrolidinophosphonium hexafluorophosphate
¹H-NMR: proton nuclear magnetic resonance
CDCl₃: deuterated chloroform
J: coupling constant
s: singlet
d: doublet
t: triplet
q: quartet
quin.: quintet
m: multiplet
v/v: volume/volume

### Preparing method 1

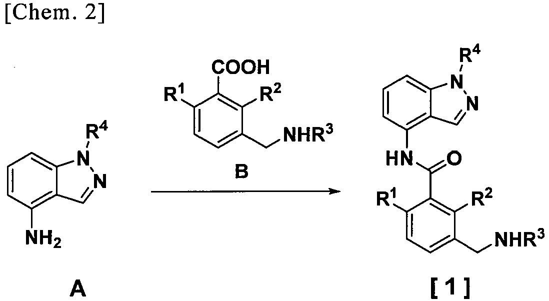

wherein, R¹, R², R³ and R⁴ are as defined above

This reaction is condensation of Compound A with Compound B, and can be carried out as condensation reaction, according to a method known as such. By reacting Compound A with the carboxylic acid Compound B or a reactive derivative thereof, the compound [1] of the present invention can be synthesized. Examples of reactive derivative of compound B can be acid halides (e.g., acid chlorides, acid bromides), mixed acid anhydrides, imidazolides, active amides and others normally used in reaction to form amide condensation. When the carboxylic acid Compound B is used, the reaction can be carried out in range of -20°C to 100°C in the presence or absence of a base, using a condensing agent. Examples of condensing agent which can be used in this reaction include 1,1'-oxalyl diimidazole, 1,1'-carbonyldiimidazole, WSCD, diethyl cyanophosphonic acid, HBTU, HATU and PyBOP. Examples of base which can be used in this reaction include organic bases such as triethylamine, N,N-diisopropylethylamine, N,N-dimethylaniline, pyridine, and 1,8-diazabicyclo[5.4.0]-7-undecene. The solvent which can be used is not limited as long as it is not involved in the reaction. Examples of solvent include ethers such as THF, 1,4-dioxane, diethyl ether and DME, amides such as DMF and DMA, nitriles such as acetonitrile and propionitrile, hydrocarbons such as benzene and toluene, and halogenated hydrocarbons such as CHCl₃ and CH₂Cl₂, or mixture thereof. Also, additives can be used appropriately. Examples of the additives include 1-hydroxybenzotriazole and 1-hydroxy-7-azabenzotriazole. While the reaction time varies depending on the type of material used, reaction temperature, etc., the range from 10 minutes to 24 hours is usually suitable. As for the amount of Compound A and condensing agent used, for example, 1 to 3 mole ratios per mole of Compound B is preferred. The amount of base used is, for example, in the range of 1 to 10 equivalents, preferably 1 to 4 equivalents for Compound B.

Compound A can be prepared, for example, according to the following process. 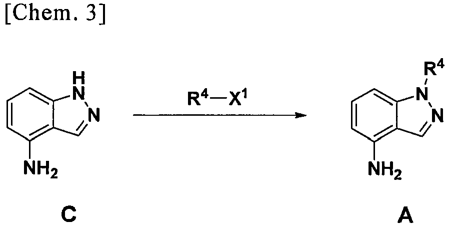 wherein R⁴ is as defined above; X¹ represents a leaving group such as chlorine atom, bromine atom, iodine atom or trifluoromethanesulfonate.

When R⁴ is aryl or heteroaryl, Compound A can be synthesized from a known compound, Compound C (e.g., can be synthesized according to the method described in WO2006/046031) and aryl halide or heteroaryl halide, by Buchwald arylation reaction using copper catalyst such as copper iodide and ligand such as trans-1,2-cyclohexanediamine (J. Org. Chem. 2004, 69, 5578-5587; J. Am. Chem. Soc. 2001, 123, 7727-7729). In this coupling reaction, in addition to the above reagents, the reaction is carried out in the presence of alkali metal carbonate (e.g., sodium carbonate, potassium carbonate, cesium carbonate, etc.), alkali metal phosphate (potassium phosphate, etc.), organic base (triethylamine, diisopropylethylamine, etc.), alkali metal halide (lithium chloride, cesium fluoride, etc.) or alkali metal hydroxide (sodium hydroxide, etc.). Specific examples of solvent should be selected depending on the type of material compounds and reagents used. For example, toluene, THF, 1,4-dioxane, DME, ethyl acetate, acetone, acetonitrile, DMF, DMA, NMP or alcohols such as MeOH, EtOH, isopropanol, tert-butanol, etc. and water can be used alone or as mixture. While the reaction temperature varies depending on the type of material compounds and reagent used, it is usually in the range from 0 to 200°C, preferably from 60 to 150°C. Also, while the reaction time varies depending on the type of materials used and the reaction temperature, the range from 30 minutes to 24 hours is usually suitable.

When R⁴ is alkyl, arylalkyl, cycloalkyl or saturated heterocyclic group, Compound A can be synthesized by N-alkylation reaction. This reaction can be carried out according to conventional methods. For example, the reaction can be carried out in the presence or absence of base in suitable solvent or neat. Examples of base to be used include pyridine, triethylamine, N,N-diisopropylethylamine, potassium carbonate, sodium bicarbonate, etc. The solvent to be used is not limited as long as it is not involved in the reaction. Examples of solvent include ethers such as THF and diethyl ether, amides such as DMF and DMA, nitriles such as acetonitrile and propionitrile, hydrocarbons such as benzene and toluene, water, or mixture thereof. While the reaction temperature varies depending on the type of compound and reagent used, it is usually carried out at 0 to 200°C, preferably 30 to 150°C. The amount of base used is, for example, 1 to 10 equivalents, preferably 1 to 4 equivalents to the amount of Compound C. While the reaction time varies depending on the type of materials used and reaction temperature, the range from 30 minutes to 24 hours is usually suitable.

Compound A can also be prepared according to the following process: 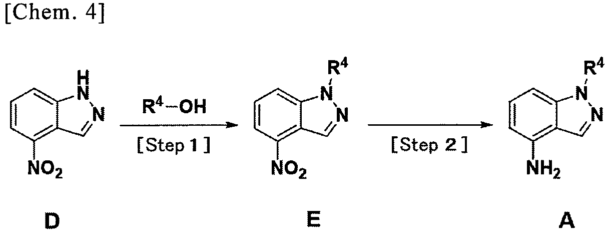 wherein R⁴ represents alkyl, arylalkyl or saturated heterocyclic group.

### Step 1

This reaction uses the typical Mitsunobu method starting from known Compound D (e.g., synthesized according to the method described in J. Med. Chem., 2006, 49, 2339-2352), and the reaction can be carried out according to methods as such known as Mitsunobu reaction. As representative reaction conditions, Compound E can be synthesized by reacting Compound D with alcohol at 0°C to room temperature in the presence of triphenylphosphine and diethyl azodicarboxylate in suitable solvent such as THF, 1,4-dioxane or DMF. Azodicarboxylic acid diesters include di-tert-butyl azodicarboxylate, diisopropyl azodicarboxylate and the like, in addition to diethyl azodicarboxylate. While the reaction temperature varies depending on the type of material compounds and reagents used, it is usually in the range from 0 to 200°C, preferably 0°C to room temperature. While the reaction time varies depending on the type of materials used and the reaction temperature, usually the range from 30 minutes to 24 hours is suitable.

### Step 2

This reaction is the reduction of Compound E to aromatic amine and can be carried out by conventional methods. For example, this reaction can be carried out by the following methods: catalytic hydrogen reduction in suitable solvent using compound E and a catalyst comprising Raney-nickel, palladium, rhodium, platinum, etc.; hydride reduction using lithium aluminum hydride, etc.; iron reduction using a reducing iron reagent and ammonium chloride, etc.; zinc powder and acetic acid, etc. Other methods include the use of sulfides such as sodium hydrosulfite, or reduction with ammonium formate or hydrazine in the presence of a metal catalyst such as palladium-carbon. Examples of solvent include toluene, THF, 1,4-dioxane, DME, ethyl acetate, acetone, acetonitrile, DMF, or alcohols such as MeOH, EtOH, tert-butanol and water, which can be used alone or as mixture. While the reaction temperature varies depending on the type of compounds and reagents used, it is usually in the range from 0 to 300°C, preferably from 20 to 150°C.

Compound B can be prepared according to, for example, the method described in WO2011/048004.

### Preparing method 2

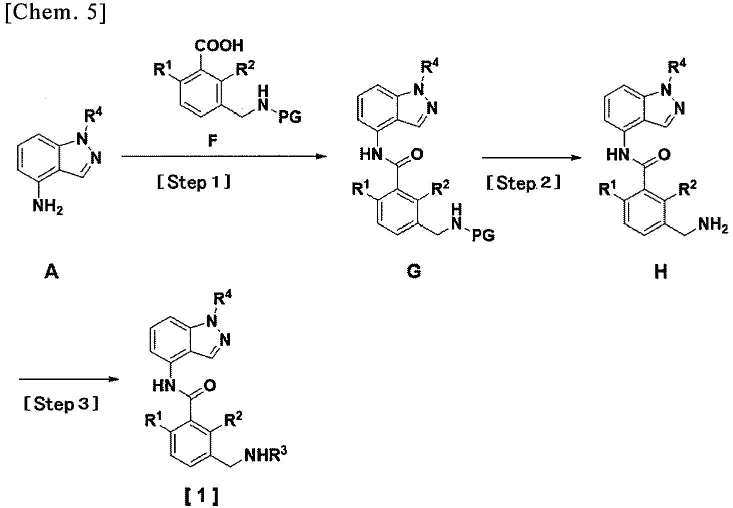

wherein, R¹, R², R³, R⁴ are as defined above; PG represents a protecting group.

### Step 1

This reaction is condensation of Compound A with Compound F. Compound G can be prepared according to Preparing method 1.

### Step 2

This reaction is deprotection of the amino group of Compound G. Compound H can be synthesized according to the methods as such known as deprotection reactions of amino groups. While protecting groups of the amino group are not limited as long as they do not affect the reaction, they include carbamates such as tert-butoxycarbonyl group and benzyloxycarbonyl group, as well as those described in the literature (e.g., Green's Protective Groups in Organic Synthesis, 4th Edition, A John Wiley & Sons, Inc. publication) which are generally used to protect amino group. The deprotection can be carried out in suitable solvent under acidic conditions using hydrochloric acid, Lewis acid, trifluoroacetic acid, etc. Deprotection can also be carried out by hydrogenolysis in the presence of catalyst such as palladium.

### Step 3

This reaction is amidation or sulfonamidation of Compound H. The Compound [1] of the present invention can be prepared according to Step 1 in Preparing method 2.

Compound F can be prepared according to the methods as described in the literatures (e.g., WO2011/048004; Tetrahedron Letters, 1993, 34, 6263-6264).

### Preparing method 3

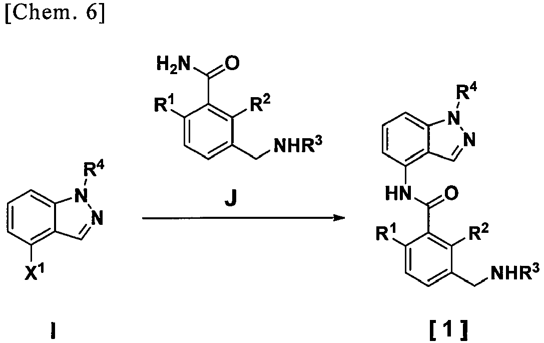

wherein, R¹, R², R³, R⁴ and X¹ are as defined above.

This reaction is palladium- or copper-catalyzed coupling of Compound I with Compound J. The reaction is carried out by known methods as such. While solvents to be used are not limited as long as they are not involved in the reaction, examples of the solvent include hydrocarbons such as toluene and xylene, ethers such as 1,4-dioxane and THF, amides such as DMF, DMA, and NMP, water, or mixture thereof. The reaction is carried out in the presence of base at the temperature ranging from 20 to 200°C, preferably from 60 to 150°C, and microwave can be used, if necessary. Examples of palladium catalyst to be used include tris(dibenzylideneacetone)(chloroform) dipalladium(0), tris(dibenzylideneacetone) dipalladium(0) and palladium(II) acetate etc. The suitable amount of such palladium catalyst is in the range of 0.001-0.3 moles per mole of Compound I. Examples of palladium catalyst ligand to be used include 1,1'-bis(diphenylphosphino)ferrocene, 4,5-bis(diphenylphosphino)-9,9'-dimethylxanthene, 2-dicyclohexylphosphino-2',4',6'-triisopropylbiphenyl, (±)-2,2'-bis(diphenylphosphino)-1,1-biphenyl, 2-(di-t-butylphosphino), bis[2-(diphenylphosphino)phenyl]ether, etc. Examples of copper catalyst to be used include copper iodide, etc. Examples of base to be used include alkali metal carbonates such as sodium carbonate, sodium bicarbonate, potassium carbonate and cesium carbonate, alkali metal hydroxides such as sodium hydroxide, alkali metal phosphates such as potassium phosphate, organic bases such as triethylamine, N,N-diisopropylethylamine, etc., and alkali metal halides such as lithium chloride, cesium fluoride, etc. The amount of base to be used is in the range from 1 to 10 equivalents, preferably 1 to 4 equivalents for Compound I. While the reaction time varies depending on the type of materials used, reaction temperature, etc., usually the range of 10 minutes to 24 hours is suitable.

Compound J can be prepared by, for example, condensation of Compound B described in Preparation method 1 with ammonia.

Compound I can be prepared according to, for example, the method described in the literature (e.g., J. Org. Chem., 2009, 74, 9539; WO 2009/117626), using benzaldehyde derivative K and hydrazine derivative L which is commercially available or can be prepared by known methods as shown below. Examples of solvent to be used are not limited as long as they are not involved in the reaction. For example, the solvents include hydrocarbons such as toluene and xylene, ethers such as 1,4-dioxane and THF, amides such as DMF, DMA, and NMP, or alcohols such as MeOH, EtOH, tert-butanol, etc., which can be used alone or as mixture. While the reaction temperature varies depending on the compounds and reagents used, it is usually in the range from 0 to 300°C, preferably from 20 to 150°C. 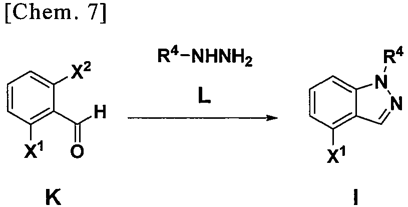 wherein R⁴ and X¹ are as defined above; X² represents a leaving group such as fluorine, chlorine or bromine.

### Preparing method 4

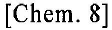

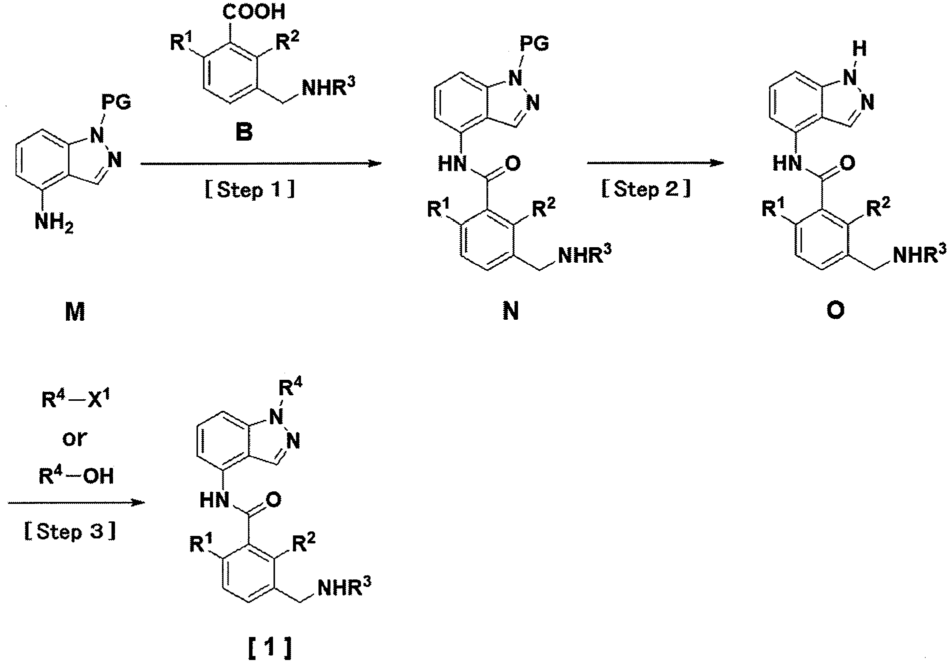

wherein R¹, R², R³, R⁴ and X¹ are as defined above; PG represents a protecting group.

### Step 1

This reaction is condensation of Compound M with Compound B. Compound N can be prepared according to Preparing method 1.

### Step 2

This reaction is deprotection of Compound N and can be carried out according to Step 2 in Preparing method 2.

### Step 3

This reaction is introduction of R⁴ onto the nitrogen atom at 1-position of the indazole ring of Compound O. The compound [1] of the present invention can be prepared according to the method for Compound A described above.

Compound M can be prepared according to the method described in the literatures (e.g., US2010/0063066; WO2004/046133).

While the compounds of the present invention can be used as a pharmaceutical, the compounds can also be used in the form of pharmaceutically acceptable salts by known methods. Examples of such salts include a salt with mineral acids such as hydrochloric acid, hydrobromic acid, sulfuric acid and phosphoric acid, and a salt with organic acids such as acetic acid, citric acid, tartaric acid, maleic acid, succinic acid, fumaric acid, p-toluenesulfonic acid, benzenesulfonic acid and methane sulfonic acid.

For example, a salt of compound with hydrochloric acid can be obtained by dissolving a compound in alcoholic solution, ethyl acetate solution or diethyl ether solution of hydrogen chloride.

The compounds of the present invention or pharmaceutically acceptable salts thereof may form a solvate by incorporating solvent molecules when left in air or recrystallized. Such a solvate is within the scope of the salt of the present invention. Examples of solvate include a solvate with molecules of methanol, ethanol, isopropyl alcohol, butanol, dimethyl sulfoxide, acetonitrile, etc., and a hydrate.

Some of the compounds of the present invention may have an asymmetric carbon, and the respective optical isomers and mixtures thereof are all included in the present invention. The optical isomers can be prepared, for example, by means of optical resolution from the racemate thereof according to a known method using an optically active acid (e.g., tartaric acid, dibenzoyltartaric acid, mandelic acid, 10-camphor sulfonic acid, etc.), utilizing its basicity, or by using an optically active compound prepared in advance as a starting material. In addition, the optical isomers may also be prepared by optical resolution using a chiral column or by asymmetric synthesis.

The compound of the present invention or a pharmaceutically acceptable salt thereof has mPGES-1 inhibitory activity, as shown in the test examples below.

Thus, one embodiment of the present invention provides an mPGES-1 inhibitor comprising the compound of the present invention or a pharmaceutically acceptable salt thereof.

Also, one embodiment of the present invention provides a method for inhibiting mPGES-1 activity, comprising administering the compound of the present invention or a pharmaceutically acceptable salt thereof to a subject in need thereof.

Further, one embodiment of the present invention provides the compound of the present invention or a pharmaceutically acceptable salt thereof for use in inhibiting mPGES-1.

In addition, one embodiment of the present invention provides the use of the compound of the present invention or a pharmaceutically acceptable salt thereof in the manufacture of mPGES-1 inhibitors.

Still furthermore, the compound of the present invention or a pharmaceutically acceptable salt thereof has mPGES-1 inhibitory activity. Thus, one embodiment of the present invention provides an inhibitor of production of PGE2 comprising the compound of the present invention or a pharmaceutically acceptable salt thereof.

Still furthermore, one embodiment of the present invention provides a method for inhibiting production of PGE2, comprising administering the compound of the present invention or a pharmaceutically acceptable salt thereof to a subject in need thereof.

Still furthermore, one embodiment of the present invention provides the compound of the present invention or a pharmaceutically acceptable salt thereof for use in inhibiting production of PGE2.

Still furthermore, one embodiment of the present invention provides the use of the compound of the present invention or a pharmaceutically acceptable salt thereof in the manufacture of an inhibitor of production of PGE2.

Since the compound of the present invention or a pharmaceutically acceptable salt thereof has mPGES-1 inhibitory activity and inhibits production of PGE2, it can be used, for example, for preventing or treating a disease in which mPGES-1 is involved, a disease in which PGE2 is involved, or a disease which is expected to be prevented or treated by analgesic, anti-inflammatory or cell growth inhibitory effect.

Thus, one embodiment of the present invention provides a preventive or therapeutic agent comprising the compound of the present invention or a pharmaceutically acceptable salt thereof, which is for a disease involving mPGES-1.

Furthermore, one embodiment of the present invention provides a method for preventing or treating a disease involving mPGES-1, comprising administering the compound of the present invention or a pharmaceutically acceptable salt thereof to a subject in need thereof.

Still furthermore, one embodiment of the present invention provides the compound of the present invention or a pharmaceutically acceptable salt thereof for use in preventing or treating a disease involving mPGES-1.

Still furthermore, one embodiment of the present invention provides the use of the compound of the present invention or a pharmaceutically acceptable salt thereof in the manufacture of a preventing or treating agent for a disease involving mPGES-1.

Still furthermore, one embodiment of the present invention provides a preventive or therapeutic agent comprising the compound of the present invention or a pharmaceutically acceptable salt thereof, which is for a disease involving PGE2.

Still furthermore, one embodiment of the present invention provides a method for preventing or treating a disease involving PGE2, comprising administering the compound of the present invention or a pharmaceutically acceptable salt thereof to a subject in need thereof.

Still furthermore, one embodiment of the present invention provides the compound of the present invention or a pharmaceutically acceptable salt thereof for use in preventing or treating a disease involving PGE2.

Still furthermore, one embodiment of the present invention provides the use of the compound of the present invention or a pharmaceutically acceptable salt thereof in the manufacture of medicament for preventing or treating a disease involving PGE2.

Still furthermore, one embodiment of the present invention provides a preventive or therapeutic agent comprising the compound of the present invention or a pharmaceutically acceptable salt thereof, which is for a disease that is expected to be prevented or treated by analgesic, anti-inflammatory or cell proliferation inhibitory effect.

Still furthermore, one embodiment of the present invention provides a method for preventing or treating a disease that is expected to be prevented or treated by analgesic, anti-inflammatory, or cell proliferation inhibitory effects, comprising administering the compound of the present invention or a pharmaceutically acceptable salt thereof to a subject in need thereof.

Still furthermore, one embodiment of the present invention provides the compound of the present invention or pharmaceutically acceptable salts thereof for use in preventing or treating a disease which is expected to be prevented or treated by analgesic, anti-inflammatory or cell growth inhibitory effect.

Still furthermore, one embodiment of the present invention provides the use of the compound of the present invention or a pharmaceutically acceptable salt thereof in the manufacture of a preventive or therapeutic agent for a disease which is expected to be prevented or treated by to analgesic, anti-inflammatory or cell growth inhibitory effect.

Examples of diseases to which the compounds of the present invention or pharmaceutically acceptable salts thereof can be applied include a disease involving mPGES-1, such as inflammatory bowel disease (see, e.g., Non-Patent Document 17), irritable bowel syndrome, migraine, headache, lumbago, lumbar spinal canal stenosis, herniated disc, temporomandibular disorder, cervico-omo-brachial syndrome, cervical spondylosis, endometriosis (see, e.g., Non-Patent Document 18), adenomyosis, premature labor, threatened premature labor, dysmenorrhea, overactive bladder, nocturia (see, e.g., Non-Patent Document 19), interstitial cystitis, neurodegenerative disease (e.g., Alzheimer's disease, multiple sclerosis, amyotrophic lateral sclerosis, Parkinson's disease) (e.g., see Non-Patent Document 19), psoriasis, rheumatoid arthritis (e.g., see Non-Patent Documents 21 and 22), rheumatic fever, fibromyalgia, neuralgia (e.g., see Non-Patent Document 23), complex regional pain syndrome, myofascial disorders, viral infections (e.g., influenza, cold, shingles, AIDS), bacterial infections, fungal infections, burn injury (see, e.g., Non-Patent Document 24), postoperative, post-trauma, and post-tooth extraction inflammation and pain, malignant tumors (e.g., colon cancer, breast cancer, lung cancer, prostate cancer) (see, e.g., Non-Patent Documents 25, 26, 27), atherosclerosis (see, e.g., Non-Patent Document 28), stroke (see, e.g., Non-Patent Document 29), gout, osteoarthritis (see, e.g., Non-Patent Documents 30, 31), juvenile arthritis, tenosynovitis, ligament ossification, systemic lupus erythematosus, vasculitis, pancreatitis, nephritis (see, e.g., Non-Patent Document 32), conjunctivitis, iritis, scleritis, uveitis, wound healing, dermatitis, eczema, osteoporosis, asthma (see, e.g., Non-Patent Document 33), chronic obstructive pulmonary disease (see, e.g., Non-Patent Document 34), pulmonary fibrosis (see Non-Patent Document 35), allergic diseases (see Non-Patent Document 36), familial adenomatous polyposis (see, e.g., Non-Patent Document 36), scleroderma (see, e.g., Non-Patent Document 38), bursitis, uterine fibroid, prostatitis, depression, or pain in cancer.

As shown in the test examples below, since the compound of the present invention or a pharmaceutically acceptable salt thereof has suppressive effect on urinary volume in nocturnal polyuria, it can be used for preventing or treating nocturnal polyuria, overactive bladder, neurogenic bladder, nocturnal enuresis, prostatic hyperplasia, central diabetes insipidus, nephrogenic diabetes insipidus, chronic prostatitis, interstitial cystitis, or urinary incontinence.

Thus, one embodiment of the present invention provides a preventive or therapeutic agent for nocturnal polyuria comprising a compound of the present invention or a pharmaceutically acceptable salt thereof.

Also, one embodiment of the present invention provides a method for preventing or treating nocturnal polyuria, comprising administering a compound of the present invention or a pharmaceutically acceptable salt thereof to a subject in need thereof.

Further, one embodiment of the present invention provides a compound of the present invention or a pharmaceutically acceptable salt thereof for use in preventing or treating nocturnal polyuria.

Furthermore, one embodiment of the present invention provides the use of the compound of the present invention or a pharmaceutically acceptable salt thereof in the manufacture of a preventing or therapeutic agent for nocturnal polyuria.

The term "subject" refers to a human or non-human animal that has or is suspected of having a disease involving mPGES-1 or PGE2. In one embodiment of the present invention, the subject is a mammal. In one embodiment of the present invention, the subject is a human.

When the compound of the present invention or a pharmaceutically acceptable salt thereof is administered as a medicament, it is administered to mammals including humans, as it is or as a pharmaceutical composition comprising 0.001% to 99.5%, preferably 0.1% to 90% of the compound or a pharmaceutically acceptable salt thereof in a pharmaceutically acceptable nontoxic and inert carrier.

The carrier may be one or more of pharmaceutically acceptable solid, semi-solid or liquid diluents, fillers, and other auxiliaries for formulations. The pharmaceutical composition according to the present invention is preferably administered in a unit dosage form. The pharmaceutical composition can be administered by tissue administration, oral administration, intravenous administration, local administration (transdermal administration, eye drops, intraperitoneal cavity, intrathoracic cavity, etc.), or transrectally. Of course, the composition is administered in dosage forms suitable for these modes of administration.

The dose as a pharmaceutical is preferably adjusted taking into consideration the conditions such as age, weight, type and severity of disease of the patient, administration route, type of the compound of the present invention, whether or not it is a salt, and the type of the salt. In general, the active ingredient amount of the compound of the present invention or a pharmaceutically acceptable salt thereof for adult, in the case of oral administration, is suitably within a range of 0.01 mg to 5 g/day/adult, preferably 1 mg to 500 mg/day/adult. In some cases, a smaller amount may be sufficient or a larger amount may be required. Usually, the dosage can be administered once a day or can be divided and administered several times a day, or in the case of intravenous administration, the dosage can be administered rapidly or sustainably within 24 hours.

One or more hydrogens, carbons, and/or the other atoms in the compound of the present invention may each be replaced with an isotope thereof. Examples of such isotopes include ²H, ³H, ¹¹C, ¹³C, ¹⁴C, ¹⁵N, ¹⁸O, ¹⁷O, ³¹P, ³²P, ³⁵S, ¹⁸F, ¹²³I and ³⁶Cl, i.e., hydrogen, carbon, nitrogen, oxygen, phosphorus, sulfur, fluorine, iodine, and chlorine. The compounds represented by the general formula [1] include those substituted with such an isotope. The compound substituted with such an isotope is also useful as a pharmaceutical and includes all radiolabeled compounds represented by the general formula [1].

The present invention is described in more detail with reference to, but is not limited to, the following Reference Examples, Examples, and Test Examples.

MS was measured by LCMS. ESI method was used as the ionization method. Observed value of mass spectrometry is expressed in m/z.

The measurement conditions for LCMS are as follows.
Instrument: ACUITY UPLC MS/PDA System (Waters)
Mass Spectrometer: Waters 3100 MS detector
Photodiode array detector: ACUITY PDA detector (UV detection wavelength: 210 to 400 nm)
Column: Acuity BEH C₁₈, 1.7 µm, 2.1 x 50 mm
Flow rate: 0.5 mL/min
Column temperature: 40°C
Solvent;
A: 0.1% formic acid/H₂O (v/v; same below)
B: 0.1% formic acid/acetonitrile
Observed value of mass spectrometry is expressed in m/z.

¹H NMR spectrum was obtained using JNM-ECS400 Nuclear Magnetic Resonance Spectrometer (JEOL RESONANCE Ltd.). The observed peaks are shown as chemical shift values δ (ppm) (s = singlet, d = doublet, t = triplet, q = quartet, brs = broad singlet, m = multiplet, dd = double doublet, dt = double triplet).

The compounds described herein were named using naming software, ACD/NAME (registered trademark, Advanced Chemistry Development Inc.) according to IUPAC rules, or ChemBioDraw (version 14.0, Cambridge Soft), or named according to IUPAC nomenclature.

### Reference Example 1: 2-Chloro-5-(cyclopropanecarbonylamino)methyl]benzoic acid

### Step 1: Methyl 5-(aminomethyl)-2-chlorobenzoate hydrochloride

Methyl 5-(bromomethyl)-2-chlorobenzoate (10.2 g) (synthesized according to the method described in WO2010/132999) was dissolved in MeOH (15 mL), and 20% ammonia/MeOH (v/v, 40 mL) was added and the mixture was stirred at 80°C for 30 min in a sealed tube. After the reaction solution was brought to room temperature, it was concentrated under reduced pressure, and the residue was acidified by adding 2 N hydrochloric acid. The aqueous layer was washed once with ethyl acetate, and the pH was adjusted to 7-8 with aqueous saturated sodium bicarbonate and extracted with ethyl acetate. The organic layer was washed with saturated saline, dried over magnesium sulfate, and concentrated under reduced pressure. The residual yellow oil (2.0 g) was dissolved in MeOH (10 mL), 2 N hydrochloric acid (6 mL) was added at room temperature, and the mixture was stirred for 5 min. MeOH was removed under reduced pressure, and the residue was solidified using hexane-ethyl acetate, filtered, and dried to give the title compound (1.7 g) as pale yellow powder.

### Step 2: Methyl 2-chloro-5-[(cyclopropanecarbonylamino)methyl]benzoate

Methyl 5-(aminomethyl)-2-chlorobenzoate hydrochloride (1.2 g) was suspended in THF (25 mL), N,N-diisopropylethylamine (2.6 mL) was added and the mixture was stirred under ice-cooling. Cyclopropanecarbonyl chloride (0.55 mL) was slowly added dropwise thereto. After stirring at the same temperature for 1 hour, aqueous saturated sodium bicarbonate was added to the reaction solution and the mixture was extracted with ethyl acetate. The organic layer was washed with water and saturated saline in this order, dried over magnesium sulfate, and concentrated. The residue was purified by silica gel column chromatography (hexane-ethyl acetate) to afford the title compound (813 mg) as white powder.

### Step 3 2-Chloro-5-[(cyclopropanecarbonylamino)methyl]benzoic acid

To methyl 2-chloro-5-[(cyclopropanecarbonylamino)methyl]benzoate (813 mg) were added MeOH (15 mL) and 1 N sodium hydroxide solution (15 mL), and the mixture was stirred at 60°C. After 1 hour, the reaction solution was brought to room temperature and MeOH was removed under reduced pressure. Water was added to the residue, then the mixture stirred under ice-cooling, and 2 N hydrochloric acid was used to adjust the pH of the mixture to pH 2-3. The precipitated solid was filtered off and dried under reduced pressure at 80°C to afford the title compound (690 mg) as white powder.

### Reference Example 2: 2-Chloro-5-{[(2,2-dimethylpropanoyl)amino]methyl]benzoic acid

According to the method described in Reference Example 1, using pivaloyl chloride instead of cyclopropane carbonyl chloride in Step 2, the title compound was obtained as white powder,

### Reference Example 3: 3-[(Cyclopropanecarbonylamino)methyl]-2,6-difluorobenzoic acid

To a mixture of N-(hydroxymethyl)cyclopropanecarboxamide (2.76 g) (synthesized according to the method described in Synthesis, 2009, 3, 495) and 2,6-difluorobenzoic acid (1.72 g) was added concentrated sulfuric acid (15 mL). The mixture was stirred at 60°C for 20 hours. The reaction solution was poured into ice-water and extracted with ethyl acetate. The organic layer was washed with saturated saline, dried over magnesium sulfate, and concentrated. The residue was purified by silica gel column chromatography to afford the title compound (0.80 g) as white powder.

### Reference Example 4: 2-Chloro-5-([(2,2,2-trifluoroacetyl)amino]methyl)benzoic acid

2,2,2-Trifluoro-N-(hydroxymethyl)acetamide (3.2 g) (synthesized according to the method described in Synthesis, 2009, 495-501) was dissolved in concentrated sulfuric acid (20 mL) and the mixture was stirred under ice-cooling. 2-Chlorobenzoic acid (3.1 g) was added thereto and the mixture was stirred at room temperature for 3 days. The reaction solution was poured into ice-water (100 mL), stirred vigorously, and the precipitated solid was filtered off and washed well with water. The resulting solid was washed with toluene-methyl ethyl ketone (7:1) to afford the title compound (2.2 g) as white powder.

### Reference Example 5: 4-Amino-1H-indazole

2-Methyl-3-nitro-aniline (30 g) was dissolved in acetic acid (450 mL) and the mixture was stirred under ice-cooling. An aqueous solution (100 mL) of sodium nitrite (20.4 g) was added dropwise, and the mixture was stirred at room temperature for 1 day. After removing acetic acid under reduced pressure, water (100 mL) was added to the resulting solid and the mixture was stirred for a while, and the precipitated solid was filtered off. The solid was dried under reduced pressure, washed with chloroform, and dried to give 4-nitro-1H-indazole (22.1 g). To a solution of 4-nitro-1H -indazole (10 g) in MeOH (100 mL) - THF (50 mL) was added 5% palladium-carbon (1.5 g), and the mixture was stirred vigorously overnight at 0.3 MPa under hydrogen atmosphere. The reaction solution was filtered through celite and the mother liquor was concentrated under reduced pressure. The resulting solid was washed with hexane-ethyl acetate (1:1) and filtered off. The solid was dried under reduced pressure to afford the title compound (6.8 g).

### Reference Example 6: 5-Bromo-2-(methoxymethyl)pyridine

To a solution of (5-bromo-2-pyridyl)methanol (200 mg) in THF (5 mL) was added sodium hydride (60% content, 51 mg) slowly under ice-cooling, and the mixture stirred at the same temperature for 30 minutes. Iodomethane (80 µL) was then added and the mixture was stirred at 60°C overnight. After the reaction solution was brought to room temperature, ice-water was added thereto and the mixture was extracted with ethyl acetate. The organic layer was washed with saturated saline, dried over magnesium sulfate, and concentrated. The residue was purified by silica gel column chromatography to afford the title compound (155 mg) as yellow oil.

### Reference Example 7: 5-Bromo-2-(2-fluoropropan-2-yl)pyridine

A solution of 2-(5-bromo-2-pyridyl)propan-2-ol (133 mg) in CH₂Cl₂ (3 mL) was cooled to -78°C, and bis(2-methoxyethyl)aminosulfatrifluoride (264 µL) was added, then the mixture was stirred at room temperature overnight. Aqueous saturated sodium bicarbonate was slowly added to the reaction solution, and the mixture was extracted with ethyl acetate. The organic layer was washed with saturated saline, dried over magnesium sulfate, and the solvent was removed. The residue was purified by silica gel column chromatography to afford the title compound (88 mg) as colorless oil.

### Example 1: 2-Chloro-5-{[(cyclopropylcarbonyl)amino]methyl}-N-{1-[4-(trifluoromethyl)phenyl]-1H-indazol-4-yl}benzamide

### [Step 1] 4-Amino-1-(4-trifluoromethylphenyl)-1H-indazole

To a suspension of 4-amino-1H-indazole (50 mg), copper(I) iodide (7 mg) and tripotassium phosphate (159 mg) in 1,4-dioxane (1 mL) were added 4-iodobenzotrifluoride (112 mg) and trans-N,N'-dimethylcyclohexane-1,2-diamine (26 mg) and the mixture was stirred at 100°C under argon atmosphere. After 18 hours, the reaction solution was concentrated under reduced pressure and the residue was purified by silica gel column chromatography (hexane-ethyl acetate) to afford the title compound (74 mg).
MS (ESI+) m/z 278 (M+H)+

### [Step 2] 2-Chloro-5-{[(cyclopropylcarbonyl)amino]methyl}-N-{1-[4-(trifluoromethyl)phenyl]-1H-indazol-4-yl}benzamide

To a solution of 2-chloro-5-[(cyclopropanecarbonylamino)methyl]benzoic acid (30 mg), HATU (64 mg) and N,N-diisopropylethylamine (61 µL) in DMF (1 mL) was added 4-amino-1-(4-trifluoromethylphenyl)-1H-indazole (36 mg) at room temperature, and the mixture was stirred overnight at the same temperature. The reaction solution was poured into water and extracted with ethyl acetate. The organic layer was washed with aqueous saturated sodium bicarbonate and saturated saline in this order, dried over magnesium sulfate, and concentrated under reduced pressure. The residue was purified by silica gel column chromatography to afford the title compound (60 mg) as white powder.
MS (ESI+) m/z 513 (M+H)⁺
¹H-NMR (DMSO-d₆) δ: 0.66-0.70 (4H, m), 1.61 (1H, m), 4.35 (2H, d), 7.40 (1H, dd), 7.53-7.57 (3H, m), 7.75 (1H, d), 7.89 (1H, d), 7.95 (2H, d), 8.05 (2H, m), 8.66-8.69 (2H, m), 10.89 (1H, s).

### Example 2: 2-Chloro-5-{[(cyclopropylcarbonyl)aminolmethyl}-N-(1-phenyl-1H-indazol-4-yl)benzamide

According to the method described in Example 1, using iodobenzene instead of 4-iodobenzotrifluoride, the title compound was obtained as white powder.
MS (ESI+) m/z 445 (M+H)⁺
¹H-NMR (DMSO-d₆) δ: 0.67-0.70 (4H, m), 1.61 (1H, m), 4.35 (2H, d), 7.38-7.61 (8H, m), 7.76 (2H, d), 7.83 (1H, d), 8.57 (1H, s), 8.68 (1H, t), 10.84 (1H, s).

### Example 3: 2-Chloro-5-{[(cyclopropylcarbonyl)amino]methyl}-N-{1-[3-(trifluoromethyl)phenyl]-1H-indazol-4-yl}benzamide

According to the method described in Example 1, using 3-iodobenzotrifluoride instead of 4-iodobenzotrifluoride, the title compound was obtained as white powder.
MS (ESI+) m/z 513 (M+H)⁺
¹H-NMR (DMSO-d₆) δ: 0.66-0.70 (4H, m), 1.61 (1H, m), 4.35 (2H, d), 7.40 (1H, dd), 7.53-7.57 (3H, m), 7.67 (1H, d), 7.78 (1H, d), 7.83-7.90 (2H, m), 8.06 (1H, s), 8.14 (1H, d), 8.65-8.69 (2H, m), 10.88 (1H, s).

### Example 4: 2-Chloro-5-{[(cyclopropylcarbonyl)amino]methyl}-N-[1-(pyridin-3-yl)-1H-indazol-4-yllbenzamide hydrochloride

According to the method described in Example 1, using 3-iodopyridine instead of 4-iodobenzotrifluoride, 2-chloro-5-{[(cyclopropylcarbonyl)amino]methyl}-N-[1-(pyridin-3-yl)-1H-indazol-4-yl]benzamide was obtained as white powder. The resulting compound was suspended in MeOH (0.5 mL), and 2 N hydrochloric acid (1 equivalent) was added thereto. The mixture was stirred, then concentrated under reduced pressure. The residue was solidified with diethyl ether-MeOH, filtered and dried to afford the title compound (17 mg) as white powder.
MS (ESI+) m/z 446 (M+H)⁺
¹ H-NMR (DMSO-d₆) δ: 0.66-0.70 (4H, m), 1.61 (1H, m), 4.35 (2H, d), 7.40 (1H, dd), 7.51-7.57 (3H, m), 7.67-7.69 (2H, m), 7.88 (1H, d), 8.28 (1H, m), 8.64 (1H, dd), 8.66 (1H, m), 7.67-7.69 (2H, m), 7.88 (1H, d), 8.28 (1H, m), 8.64 (1H, dd), 8.66 (1H, s), 8.69 (1H, t), 9.06 (1H, d), 10.89 (1H, s).

### Example 5: 2-Chloro-5-{[(cyclopropylcarbonyl)amino]methyl}-N-[1-(pyridin-4-yl)-1H-indazol-4-yl]benzamide hydrochloride

According to the method described in Example 1, using 4-iodopyridine instead of 4-iodobenzotrifluoride, 2-chloro-5-{[(cyclopropylcarbonyl)amino]methyl}-N-[1-(pyridin-4-yl)-1H-indazol-4-yl]benzamide was obtained as white powder. The resulting compound was suspended in MeOH (1.5 mL), and 2 N hydrogen chloride/MeOH (1 equivalent) was added thereto. The mixture was stirred and concentrated under reduced pressure. The residue was solidified with diethyl ether-MeOH, filtered and dried to afford the title compound as pale yellow powder.
MS (ESI+) m/z 446 (M+H)⁺

### Example 6: 2-Chloro-5-{[((cyclopropylcarbonyl)amino]methyl}-N-[1-(4-methylphenyl)-1H-indazol-4-yl]benzamide

According to the method described in Example 1, using 4-iodotoluene instead of 4-iodobenzotrifluoride, the title compound was obtained as white powder.
MS (ESI+) m/z 459 (M+H)⁺

### Example 7: 2-Chloro-5-{[(cyclopropylcarbonyl)aminolmethyl}-N-[1-(3-methylphenyl)-1H-indazol-4-yl]benzamide

According to the method described in Example 1, using 3-iodotoluene instead of 4-iodobenzotrifluoride, the title compound was obtained as white powder.
MS (ESI+) m/z 459 (M+H)⁺
¹ H-NMR (DMSO-d₆) δ: 0.66-0.70 (4H, m), 1.61 (1H, m), 2.43 (3H, s), 4.33 (2H, d), 7.22 (1H, d), 7.40 (1H, dd), 7.45-7.49 (2H, m), 7.53-7.61 (5H, m), 7.84 (1H, d), 8.56 (1H, s), 8.69 (1H, t), 10.84 (1H, s).

### Example 8: 2-Chloro-5-{[(cyclopropylcarbonyl)amino]methyl}-N-[1-(pyridin-2-yl)-1H-indazol-4-yl]benzamide

According to the method described in Example 1, 2-iodopyridine was substituted for 4-iodobenzotrifluoride and the title compound was obtained as white powder.
MS (ESI+) m/z 446 (M+H)⁺
¹ H-NMR (DMSO-d₆) δ: 0.66-0.70 (4H, m), 1.61 (1H, m), 4.35 (2H, d), 7.31-7.35 (1H, m), 7.40 (1H, dd), 7.54-7.58 (3H, m), 7.88 (1H, d), 8.00-8.02 (2H, m), 8.54-8.59 (2H, m), 8.64 (1H, s), 8.69 (1H, t), 10.87 (1H, s).

### Example 9: 2-Chloro-5-{[(cyclopropylcarbonyl)amino]methyl}-N-[1-(2-methylphenyl)-1H-indazol-4-yl]benzamide

According to the method described in Example 1, using 2-iodotoluene instead of 4-iodobenzotrifluoride, the title compound was obtained as white powder.
MS (ESI+) m/z 459 (M+H)⁺

### Example 10: 2-Chloro-5-{[(cyclopropylcarbonyl)amino]methyl}-N-[1-(6-methylpridin-3-yl)-1H-indazol-4-yl]benzamide hydrochloride

According to the method described in Example 1, using 5-bromo-2-methylpyridine instead of 4-iodobenzotrifluoride, 2-chloro-5-{[(cyclopropylcarbonyl)amino]methyl}-N-[1-(6-methylpyridin-3-yl)-1H-indazol-4-yl]benzamide was obtained as white powder. The resulting compound was suspended in MeOH (0.5 mL), and 2 N hydrochloric acid (1 equivalent) was added thereto. The mixture was stirred and concentrated under reduced pressure. The residue was solidified with diethyl ether-MeOH, filtered, dried, and the title compound was obtained as pale yellow powder.
MS (ESI+) m/z 460 (M+H)⁺

### Example 11: 2-Chloro-5-{[(cyclopronylcarbonyl)amino]methyl}-N-{1-[5-(trifluoromethyl)pyridin-3-yl]-1H-indazol-4-yl}benzamide

According to the method described in Example 1, using 3-bromo-5-(trifluoromethyl)pyridine instead of 4-iodobenzotrifluoride, the title compound was obtained as white powder.
MS (ESI+) m/z 514 (M+H)⁺
¹H-NMR (DMSO-d₆) δ: 0.66-0.70 (4H, m), 1.61 (1H, m), 4.35 (2H, d), 7.40 (1H, dd), 7.55-7.59 (3H, m), 7.76 (1H, d), 7.90 (1H, d), 8.55 (1H, t), 8.68 (1H, m), 8.71 (1H, s), 9.02 (1H, d), 9.39 (1H, d), 10.91 (1H, s).

### Example 12: 2-Chloro-5-{[(cyclopropylcarbonyl)amino]methyl}-N-(1-[6-(trifluoromethyl)pyridin-3-yl]-1H-indazol-4-yl}benzamide

According to the method described in Example 1, using 5-bromo-2-(trifluoromethyl)pyridine instead of 4-iodobenzotrifluoride, the title compound was obtained as white powder.
MS (ESI+) m/z 514 (M+H)⁺
¹H-NMR (DMSO-d₆) δ: 0.66-0.70 (4H, m), 1.61 (1H, m), 4.35 (2H, d), 7.40 (1H, dd), 7.55-7.59 (3H, m), 7.83 (1H, d), 7.91 (1H, d), 8.11 (1H, d), 8.52 (1H, d), 8.68 (1H, t), 8.72 (1H, s), 9.28 (1H, d), 10.92 (1H, s).

### Example 13: 2-Chloro-5-{[(cyclopropylcarbonyl)amino]methyl}-N-[1-(2-methoxyphenyl)-1H-indazol-4-yl]benzamide

According to the method described in Example 1, using 2-bromoanisole instead of 4-iodobenzotrifluoride, the title compound was obtained as pale yellow powder.
MS (ESI+) m/z 475 (M+H)⁺
¹H-NMR (DMSO-d₆) δ: 0.66-0.71 (4H, m), 1.61 (1H, m), 3.77 (3H, s), 4.36 (2H, d), 6.97 (1H, d), 7.15 (1H, t), 7.32 (1H, d), 7.33-7.43 (3H, m), 7.51-7.58 (3H, m), 7.75 (1H, d), 8.49 (1H, s), 8.68 (1H, s), 10.79 (1H, s).

### Example 14: N-[1-(4-tert-butylphenyl)-1H-indazol-4-yl]-2-chloro-5-{[(cyclopropylcarbonyl)amino]methyl} benzamide

According to the method described in Example 1, using 1-bromo-4-tert-butyl benzene instead of 4-iodobenzotrifluoride, the title compound was obtained as white powder.
MS (ESI+) m/z 501 (M+H)⁺

### Example 15: 2-Chloro-N-[1-(3-chloro-2-methylphenyl)-1H-indazol-4-yl]-5-[(cyclopropylcarbonyl)amino]methyl}benzamide

According to the method described in Example 1, using 2-chloro-6-iodotoluene instead of 4-iodobenzotrifluoride, the title compound was obtained as pale yellow powder.
MS (ESI+) m/z 493 (M+H)⁺

### Example 16: 3-{[(Cyclopropylcarbonyl)amino]methyl}-2,6-difluoro-N-{1-[3-(trifluoromethyl)phenyl]-1H-indazol-4-yl}benzamide

According to the method described in Example 1, using 3-iodobenzotrifluoride instead of 4-iodobenzotrifluoride in Step 1, and using 3-[(cyclopropanecarbonylamino)methyl]-2,6-difluorobenzoic acid instead of 2-chloro-5-[(cyclopropanecarbonylamino)methyl]benzoic acid in Step 2, the title compound was obtained as white powder.
MS (ESI+) m/z 515 (M+H)⁺

### Example 17: 2-Chloro-5-{[(trifluoroacetyl)amino]methyl}-N-{-1[4-(trifluoromethyl)phenyl)-1H-indazol-4-yl}benzamide

To a suspension of 2-chloro-5-{[(2,2,2-trifluoroacetyl)amino]methyl}benzoic acid (1.01 g) in CH₂Cl₂-THF (2:1) (15 mL) was added catalytic amount of DMF, and then oxalyl chloride (907 µL) was slowly added dropwise with stirring under ice-cooling. The mixture was stirred at room temperature for 1 hour. The solvent was removed under reduced pressure and the residue was dried under reduced pressure. The residue was dissolved in THF (5 mL), and a solution of 4-amino-1-(4-trifluoromethylphenyl)-1H-indazole (777 mg) and N,N-diisopropylethylamine (1.46 mL) in THF (20 mL) was added dropwise under ice-cooling. The mixture was stirred for 1 hour. Aqueous saturated sodium bicarbonate was added to the reaction solution, and the mixture was extracted with ethyl acetate. The organic layer was washed with water and saturated saline in this order, dried over magnesium sulfate, and concentrated under reduced pressure. The residue was purified by silica gel column chromatography (hexane-ethyl acetate) to afford the title compound (1.2 g) as white powder.
MS (ESI+) m/z 541 (M+H)⁺

### Example 18: 5-[(Acetylamino)methyl]-2-chloro-N-{1-[4-(trifluoromethyl)phenyl]-1H-indazol-4-yl}benzamide

### Step 1 5-(Aminomethyl)-2-chloro-N-{1-[4-(trifluoromethyl)phenyl]-1H-indazol-4-yl}benzamide

To a solution of 2-chloro-5-{[(trifluoroacetyl)amino]methyl}-N-{1-[4-(trifluoromethyl)phenyl]indazol-4-yl}benzamide (200 mg) in MeOH (4 mL)-water (1 mL) was added potassium carbonate (256 mg), and the mixture was stirred at 60°C for 4 hours. After the solvent was removed under reduced pressure, water was added to the residue and the mixture was stirred for a while. The insoluble solid was filtered off and dried under reduced pressure to afford the title compound (156 mg) as white powder.
MS (ESI+) m/z 445 (M+H)⁺

### Step 2 5-[(Acetylamino)methyl]-2-chloro-N-{1-[4-(trifluoromethyl)phenyl]-1H-indazol-4-yl}benzamide

To a solution of 5-(aminomethyl)-2-chloro-N-{1-[4-(trifluoromethyl)phenyl]indazol-4-yl}benzamide (20 mg) and N,N-diisopropylethylamine (19 µL) in THF (2 mL) was added acetyl chloride (32 µL) dropwise under ice-cooling, and the mixture was stirred for 10 minutes. Aqueous saturated sodium bicarbonate was added to the reaction solution and the mixture was extracted with ethyl acetate. The organic layer was washed with water and saturated saline in this order, dried over magnesium sulfate, and concentrated under reduced pressure. The residue was solidified with hexane-ethyl acetate, filtered off, and dried to afford the title compound (19 mg) as white powder.
MS (ESI+) m/z 487 (M+H)⁺

### Example 19: 2-Chloro-5-[(propanoylamino)methyl]-N-{1-[4-(trifluoromethyl)phenyl]-1H-indazol-4-yl}benzamide

According to the method described in Example 18, using propionyl chloride instead of acetyl chloride in Step 2, the title compound was obtained as white powder.
MS (ESI+) m/z 501 (M+H)⁺

### Example 20: 2-Chloro-5-{[(3,3-dimethylbutanoyl)amino]methyl)-N-{1-[4-(trifluoromethyl)phenyl]-1H-indazol-4-yl}benzamide

According to the method described in Step 2 of Example 18, using 3,3-dimethylbutyryl chloride instead of acetyl chloride, the title compound was obtained as white powder.
MS (ESI+) m/z 543 (M+H)⁺

### Example 21: 2-Chloro-5-{[(methoxyacetyl)amino]methyl}-N-(1-[4-(trifluoromethyl)phenyl]-1H-indazol-4-yl)benzamide

According to the method described in Step 2 of Example 18, using methoxyacetyl chloride instead of acetyl chloride, the title compound was obtained as white powder.
MS (ESI+) m/z 517 (M+H)⁺

### Example 22: 2-Chloro-5-{[(phenylcarbonyl)amino]methyl}-N-{1-[(trifluoromethyl)phenyl]-1H-indazol-4-yl}benzamide

According to the method described in Step 2 of Example 18, using benzoyl chloride instead of acetyl chloride, the title compound was obtained as white powder.
MS (ESI+) m/z 549 (M+H)⁺

### Example 23: 2-Chloro-5-{[(cyclopropylsulfonyl)amino]methyl}-N-{1-[4-(trifluoromethyl)phenyl]-1H-indazol-4-yl}benzamide

According to the method described in Step 2 of Example 18, using cyclopropanesulfonyl chloride instead of acetyl chloride, the title compound was obtained as white powder.
MS (ESI+) m/z 549 (M+H)⁺

### Example 24: 2-Chloro-5-{(N,N-dimethylglycyl)amino]methyl}-N-{1-[4-(trifluoromethyl)phenyl]-1H-indazol-4-yl}benzamide hydrochloride

To a solution of N,N-dimethylglycine (64 mg), HBTU (26 mg) and N,N-diisopropylethylamine (29 µL) in DMF (0.5 mL) was added 5-(aminomethyl)-2-chloro-N-{1-[4-(trifluoromethyl)phenyl]-1H-indazol-4-yl}benzamide (25 mg), and the mixture was stirred overnight. Aqueous saturated sodium bicarbonate was added to the reaction solution, and the mixture was extracted with ethyl acetate. The organic layer was washed with water and saturated saline in this order, dried over magnesium sulfate, and concentrated under reduced pressure. The residue was purified by silica gel column chromatography (hexane-ethyl acetate) to give 2-chloro-5-{[(N,N-dimethylglycyl)amino]methyl}-N-{1-[4-(trifluoromethyl)phenyl]-1H-indazol-4-yllbenzamide (15 mg) as white powder. To a solution of the resulting compound in MeOH (1 mL) was added aqueous 2 N hydrochloric acid (1 equivalent), and the solvent was removed under reduced pressure. The residue was solidified with diethyl ether-MeOH to afford the title compound (16 mg) as white powder.
MS (ESI+) m/z 530 (M+H)⁺

### Example 25: 2-Chloro-5-({[(1-cyanocyclopropyl)carbonyl]amino}methyl)-N-{1-[4-(trifluoromethyl)phenyl]-1H-indazol-4-yl}benzamide

According to the method described in Example 24, using 1-cyanocyclopropanecarboxylic acid instead of N,N-dimethylglycine, the title compound was obtained as white powder.
MS (ESI+) m/z 538 (M+H)⁺

### Example 26: 2-Chloro-5-[({1-(trifluoromethyl)cyclopropyl]carbonyl}amino)methyl]-N-{1-[4-(trifluoromethyl)phenyl]-1H-indazol-4-yl}benzamide

According to the method described in Example 24, using 1-trifluoromethylcyclopropane-1-carboxylic acid instead of N,N-dimethylglycine, the title compound was obtained as white powder.
MS (ESI+) m/z 581 (M+H)⁺

### Example 27: 2-Chloro-5-({[(1-hydroxycyclopropyl)carbonyl]amino}methyl)-N-{1-[4-(trifluoromethyl)phenyl]-1H-indazol-4-yl}benzamide

According to the method described in Example 24, using 1-hydroxycyclopropanecarboxylic acid instead of N,N-dimethylglycine, the title compound was obtained as white powder.
MS (ESI+) m/z 529 (M+H)⁺

### Example 28: 2-Chloro-5-{[(3-hydroxy-2,2-dimethylpropanoyl)amino]methyl}-N-{1-[4-(trifluoromethyl)phenyl]-1H-indazol-4-yl}benzamide

According to the method described in Example 24, using 2,2-dimethyl-3-hydroxypropionic acid instead of N,N-dimethylglycine, the title compound was obtained as white powder.
MS (ESI+) m/z 545 (M+H)⁺

### Example 29: 2-Chloro-5-{([(cyclobutylcarbonyl)amino]methyl}-N-{1-[4-(trifluoromethyl)phenyl]-1H-indazol-4-yl}benzamide

According to the method described in Step 2 of Example 18, using cyclobutanecarbonyl chloride instead of acetyl chloride, the title compound was obtained as white powder.
MS (ESI+) m/z 527 (M+H)⁺

### Example 30: 2-Chloro-5-{[(cyclopentylcarbonyl)amino]methyl}-N-{1-[4-(trifluoromethyl)phenyl]-1H-indazol-4-yl}benzamide

According to the method described in Step 2 of Example 18, using cyclopentanecarbonyl chloride instead of acetyl chloride, the title compound was obtained as white powder.
MS (ESI+) m/z 541 (M+H)⁺

### Example 31: 2-Chloro-5-{[(cyclohexylcarbonyl)amino]methyl}-N-{1-[4-(trifluoromethyl)phenyl]-1H-indazol-4-yl}benzamide

According to the method described in Step 2 of Example 18, using cyclohexanecarbonyl chloride instead of acetyl chloride, the title compound was obtained as white powder.
MS (ESI+) m/z 555 (M+H)⁺

### Example 32: N-[4-chloro-3-({1-[4-(trifluoromethyl)phenyl]-1H-indazol-4-yl}carbamoyl)benzyl]tricyclo[3.3.1.1^{3,7}]decane-1-carboxamide

According to the method described in Step 1 of Example 24, using 1-adamantanecarboxylic acid instead of N,N-dimethylglycine, the title compound was obtained as white powder.
MS (ESI+) m/z 607 (M+H)⁺

### Example 33: N-[4-chloro-3-({1-[4-(trifluoromethyl)phenyl]-1H-indazol-4-yl}carbamoyl)benzyl]-tetrahydrofuran-2-carboxamide

According to the method described in Step 1 of Example 24, using tetrahydrofuran-2-carboxylic acid instead of N,N-dimethylglycine, the title compound was obtained as white powder.
MS (ESI+) m/z 543 (M+H)⁺

### Example 34: N-[4-chloro-3-({1-[4-(trifluoromethyl]phenyl]-1H-indazol-4-yl}carbamoyl)benzyl]-tetrahydrofuran-3-carboxamide

According to the method described in Step 1 of Example 24, the title compound was obtained as white powder, using tetrahydrofuran-3-carboxylic acid instead of N,N-dimethylglycine.
MS (ESI+) m/z 543 (M+H)⁺

### Example 35: 2-Chloro-5-{[(N,N-dimethyl-β-alanyl)amino]methyl}-N-{1-[4-(trifluoromethyl)phenyl]-1H-indazol-4-yl)benzamide hydrochloride

According to the method described in Example 24, using 3-dimethylaminopropionic acid instead of N,N-dimethylglycine, the title compound was obtained as white powder.
MS (ESI+) m/z 544 (M+H)⁺

### Example 36: tert-Butyl 4-{[4-chloro-3-({1-[4-(trifluoromethyl)phenyl]-1H-indazol-4-yl}carbamoyl)benzyl]carbamoyl}piperidine-1-carboxylate

According to the method described in Example 24, using N-(tert-butoxycarbonyl)isonipecotic acid instead of N,N-dimethylglycine, the title compound was obtained as white powder.
MS (ESI+) m/z 656 (M+H)⁺

### Example 37: 2-Chloro-N-{1-[4-(trifluoromethyl)phenyl]-1H-indazol-4-yl}-5-{[(3,3,3-trifluoropropanoyl)amino]methyl}benzamide

According to the method described in Example 24, using 3,3,3-trifluoropropanoic acid instead of N,N-dimethylglycine, the title compound was obtained as white powder.
MS (ESI+) m/z 555 (M+H)⁺

### Example 38: 2-Chloro-5-{[(cyclopropylacetyl)amino]methyl}-N-{1-[4-(trifluoromethyl)phenyl]-1H-indazol-4-yl}benzamide

According to the method described in Example 24, using cyclopropylacetic acid instead of N,N-dimethylglycine, the title compound was obtained as white powder.
MS (ESI+) m/z 527 (M+H)⁺

### Example 39: 2-Chloro-5-({[trans-(4-hydroxycyclohexyl)carbonyl]amino}methyl)-N-{1-[4-(trifluoromethyl)phenyl]-1H-indazol-4-yl}benzamide

According to the method described in Example 24, using trans-4-hydroxycyclohexanecarboxylic acid instead of N,N-dimethylglycine, the title compound was obtained as white powder.
MS (ESI+) m/z 571 (M+H)⁺

### Example 40: 2-Chloro-5-({[cis-(4-hydroxycyclohexyl)carbonyl]amino}methyl)-N-{1-[4-(trifluoromethyl)phenyl]-1H-indazol-4-yl}benzamide

According to the method described in Example 24, using cis-4-hydroxycyclohexanecarboxylic acid instead of N,N-dimethylglycine, the title compound was obtained as white powder.
MS (ESI+) m/z 571 (M+H)⁺

### Example 41: 2-Chloro-5-{[(2-ethyl-2-hydroxybutanoyl)amino]methyl}-N-{1-[4-(trifluoromethyl)phenyl]-1H-indazol-4-yl}benzamide

According to the method described in Example 24, using 2-ethyl-2-hydroxybutyric acid instead of N,N-dimethylglycine, the title compound was obtained as white powder.
MS (ESI+) m/z 559 (M+H)⁺

### Example 42: 5-[(2-Butynoylamino)methyl]-2-chloro-N-{1-[4-(trifluoromethyl)phenyl]-1H-indazol-4-yl}benzamide

According to the method described in Example 24, using 2-butynoic acid instead of N,N-dimethylglycine, the title compound was obtained as white powder.
MS (ESI+) m/z 511 (M+H)⁺

### Example 43: 5-[(Butanoylamino)methyl]-2-chloro-N-{1-[4-(trifluoromethyl)phenyl]-1H-indazol-4-yl}benzamide

According to the method described in Step 2 of Example 18, using butyryl chloride instead of acetyl chloride, the title compound was obtained as white powder.
MS (ESI+) m/z 515 (M+H)⁺

### Example 44: 2-Chloro-5-{[(phenylacetyl)amino]methyl}-N-{1-(4-(trifluoromethyl)phenyl]-1H-indazol-4-yl}benzamide

According to the method described in Step 2 of Example 18, using phenylacetyl chloride instead of acetyl chloride, the title compound was obtained as white powder.
MS (ESI+) m/z 563 (M+H)⁺

### Example 45: N-[4-chloro-3-({1-[4-(trifluoromethyl)phenyl]-1H-indazol-4-yl}carbamoyl)benzyl]furan-2-carboxamide

According to the method described in Step 2 of Example 18, using 2-furoyl chloride instead of acetyl chloride, the title compound was obtained as white powder.
MS (ESI+) m/z 539 (M+H)⁺

### Example 46: 2-Chloro-5-{[(cyclopentylacetyl)amino]methyl}-N-{1-[4-(trifluoromethyl)phenyl]-1H-indazol-4-yl}benzamide

According to the method described in Example 24, using cyclopentylacetic acid instead of N,N-dimethylglycine, the title compound was obtained as white powder.
MS (ESI+) m/z 555 (M+H)⁺

### Example 47: 2-Chloro-5-{[(3-methoxypropanoyl)amino]methyl)-N-{1-[4-(trifluoromethyl)phenyl]-1H-indazol-4-yl}benzamide

According to the method described in Example 24, using 3-methoxypropionic acid instead of N,N-dimethylglycine, the title compound was obtained as white powder.
MS (ESI+) m/z 531 (M+H)⁺

### Example 48: 2-Chloro-5-{[(2,2-dimethylpropanoyl)amino]methyl}-N-[1-(4-methoxyphenyl)-1H-indazol-4-yl]benzamide

### Step 1: 4-Amino-1-(4-methoxyphenyl)-1H-indazole

According to the method described in Step 1 of Example 1, using 4-bromoanisole instead of 4-iodobenzotrifluoride, the title compound was obtained as colorless powder.

### Step 2: 2-Chloro-5-{[(2,2-dimethylpropanoyl)amino]methyl}-N-[1-(4-methoxyphenyl)-1H-indazol-4-yl]benzamide

To a suspension of 2-chloro-5-{[(2,2-dimethylpropanoyl)amino)methyl]}benzoic acid (53 mg) in CH₂Cl₂ (2 mL) was added catalytic amount of DMF, and then oxalyl chloride (33 µL) was added dropwise under ice-cooling. The mixture was stirred at room temperature for 1 hour. The solvent was removed under reduced pressure and the residue was dried under reduced pressure. The residue was dissolved in THF (1 mL), the solution was added dropwise to a THF (2 mL) solution of 4-amino-1-(4-methoxyphenyl)-1H-indazole (32 mg) and N,N -diisopropylethylamine (144 µL) under ice-cooling, and then the mixture was stirred for 18 hours. Aqueous saturated sodium bicarbonate was added to the reaction solution and the mixture was extracted with ethyl acetate. The organic layer was washed with water and saturated saline in this order, dried over magnesium sulfate, and concentrated under reduced pressure. The residue was purified by silica gel column chromatography (hexane-ethyl acetate) to afford the title compound (47 mg) as white powder.
MS (ESI+) m/z 491 (M+H)⁺
¹H-NMR (DMSO-d₆) δ: 1.13 (9H, s), 3.83 (3H, s), 4.31 (2H, d), 7.12-7.16 (2H, m), 7.37 (1H, dd), 7.42-7.50 (3H, m), 7.53 (1H, d), 7.61-7.65 (2H, m), 7.81 (1H, d), 8.18 (1H, t), 8.51 (1H, s), 10.81 (1H, s).

### Example 49: 2-Chloro-5-{[(2,2-dimethylpropanoyl)amino]methyl]-N-[1-(3-methoxyphenyl)-1H-indazol-4-yl]benzamide

According to the method described in Example 48, using 3-bromoanisole instead of 4-bromoanisole, the title compound was obtained as white powder.
MS (ESI+) m/z 491 (M+H)⁺

### Example 50: 2-Chloro-5-{[(2,2-dimethylpropanoyl)amino]methyl}-N-{1-[4-(trifluoromethyl)phenyl]-1H-indazol-4-yl]benzamide

According to the method described in Step 2 of Example 48, using 4-amino-1-(4-trifluoromethylphenyl)-1H-indazole instead of 4-amino-1-(4-methoxyphenyl)-1H-indazole, the title compound was obtained as white powder.
MS (ESI+) m/z 529 (M+H)⁺

### Example 51: 2-Chloro-5-{[(2,2-dimethylpropanoyl)amino]methyl}-N-{1-[4-(trifluoromethoxy)phenyl]-1H-indazol-4-yl}benzamide

According to the method described in Example 48, using 1-bromo-4-(trifluoromethoxy)benzene instead of 4-bromoanisole, the title compound was obtained as white powder.
MS (ESI+) m/z 545 (M+H)⁺

### Example 52: 2-Chloro-5-{[(2,2-dimethylpropanoyl)amino]methyl}-N-[-(2-methylpyrimidin-5-yl)-1H-indazol-4-yl]benzamide

According to the method described in Example 48, using 5-bromo-2-methylpyrimidine instead of 4-bromoanisole, the title compound was obtained as pale yellow powder.
MS (ESI+) m/z 477 (M+H)⁺
¹H-NMR (DMSO-d₆) δ: 1.13 (9H, s), 2.74 (3H, s), 4.31 (2H, d), 7.37 (1H, d), 7.54 (3H, m), 7.70 (1H, d), 7.88 (1H, d), 8.18 (1H, t), 8.68 (1H, s), 9.17 (1H, s), 10.89 (1H, s).

### Example 53: 2-Chloro-N-[1-(3-chloro-4-methylphenyl)-1H-indazol-4-yl]-5-([(2,2-dimethylpropanoyl)amino]methyl)benzamide

According to the method described in Example 48, using 2-chloro-4-iodotoluene instead of 4-bromoanisole, the title compound was obtained as pale yellow powder.
MS (ESI+) m/z 509 (M+H)⁺

### Example 54: 2-Chloro-5-{[(2,2-dimethylpropanoyl)amino]methyl}-N-[1-(6-methylpyridin-3-yl)-1H-indazol-4-yl]benzamide hydrochloride

According to the method described in Example 48, using 5-bromo-2-methylpyridine instead of 4-bromoanisole, 2-chloro-5-{[(2,2-dimethylpropanoyl)amino]methyl}-N-[1-(6-methylpyridin-3-yl)-1H-indazol-4-yl]benzene (17 mg) was obtained as pale yellow powder. The resulting compound was dissolved in ethyl acetate (0.5 mL), 4 N hydrogen chloride/ethyl acetate (1 equivalent) was added thereto and the mixture was stirred for 5 minutes. The precipitated solid was filtered off and dried to afford the title compound (17 mg) as pale yellow powder.
MS (ESI+) m/z 476 (M+H)⁺

### Example 55: 2-Chloro-N-[1-(4-cyanophenyl)-1H-indazol-4-yl]-5-([(2,2-dimethylpropanoyl)amino]methyl)benzamide

According to the method described in Example 48, using 4-bromobenzonitrile instead of 4-bromoanisole, the title compound was obtained as pale yellow powder.
MS (ESI+) m/z 486 (M+H)⁺

### Example 56: 2-Chloro-5-{[(2,2-dimethylpropanoyl)amino]methyl}-N-[1-(2-methylpyridin-3-yl)-1H-indazol-4-yl]benzamide hydrochloride

According to the method described in Example 54, using 3-bromo-2-methylpyridine instead of 5-bromo-2-methylpyridine, the title compound was obtained as pale yellow powder.
MS (ESI+) m/z 476 (M+H)⁺

### Example 57: 2-Chloro-5-{[(2,2-dimethylpropanoyl)amino]methyl}-N-{1-[5-(trifluoromethyl)pyridin-3-yl]-1H-indazol-4-yl}benzamide

According to the method described in Example 48, using 3-bromo-5-(trifluoromethyl)pyridine instead of 4-bromoanisole, the title compound was obtained as white powder.
MS (ESI+) m/z 530 (M+H)⁺

### Example 58: 2-Chloro-5-{[(2,2-dimethylpropanoyl)amino]methyl}-N-[-(2-methylpyridin-4-yl)-1H-indazol-4-yl]benzamide hydrochloride

According to the method described in Example 54, using 4-bromo-2-methylpyridine instead of 5-bromo-2-methylpyridine, the title compound was obtained as pale yellow powder.
MS (ESI+) m/z 476 (M+H)⁺

### Example 59: 2-Chloro-5-{[(2,2-dimethylpropanoyl)amino]methyl}-N-[1-(6-methylpyridazin-3-yl)-1H-indazol-4-yl]benzamide

According to the method described in Example 48, using 3-iodo-6-methylpyridazine (synthesized according to the method described in EP2080761) instead of 4-bromoanisole, the title compound was obtained as white powder.
MS (ESI+) m/z 477 (M+H)⁺
¹ H-NMR (DMSO-d₆) δ: 1.13 (9H, s), 2.65 (3H, s), 4.31 (2H, d), 7.37 (1H, d), 7.53 (2H, m), 7.62 (1H, t), 7.79 (1H, d), 7.91 (1H, d), 8.16-8.22 (2H, m), 8.52 (1H, d), 8.71 (1H, s), 10.91 (1H, s).

### Example 60: 2-Chloro-5-{[(2,2-dimethylpropanoyl)amino]methyl}-N-[1-(5-methylpyridin-3-yl)-1H-indazol-4-yl]benzamide hydrochloride

According to the method described in Example 54, using 3-bromo-5-methylpyridine instead of 5-bromo-2-methylpyridine, the title compound was obtained as white powder.
MS (ESI+) m/z 476 (M+H)⁺

### Example 61: 2-Chloro-5-{[(2,2-dimethylpropanoyl)amino]methyl]-N-[1-(5-methoxypyridin-3-yl)-1H-indazol-4-yl]benzamide hydrochloride

According to the method described in Example 54, using 3-bromo-5-methoxypyridine instead of 5-bromo-2-methylpyridine, the title compound was obtained as white powder.
MS (ESI+) m/z 492 (M+H)⁺

### Example 62: 2-Chloro-5-{(2,2-dimethylpropanoyl)amino]methyl}-N-{1-[4-methoxy-3-(trifluoromethyl)phenyl]-1H-indazol-4-yl}benzamide

According to the method described in Example 48, using 4-methoxy-3-(trifluoromethyl)bromobenzene instead of 4-bromoanisole, the title compound was obtained as white powder.
MS (ESI+) m/z 559 (M+H)⁺

### Example 63: 2-Chloro-5-{[(2,2-dimethylpropanoyl)amino]methyl}-N-[1-(6-methoxypyridin-3-yl)-1H-indazol-4-yl]benzamide

According to the method described in Example 48, using 5-bromo-2-methoxypyridine instead of 4-bromoanisole, the title compound was obtained as pale yellow powder.
MS (ESI+) m/z 492 (M+H)⁺

### Example 64: 2-Chloro-N-[1-(5-cyclopropylpyridin-3-yl)-1H-indazol-4-yl]-5-{[(2,2-dimethylpropanoyl)amino]methyl}benzamide hydrochloride

According to the method described in Example 54, using 3-bromo-5-cyclopropylpyridine (synthesized according to the method described in WO2008/091681) instead of 5-bromo-2-methylpyridine, the title compound was obtained as white powder.
MS (ESI+) m/z 502 (M+H)⁺

### Example 65: 2-Chloro-5-{[(2,2-dimethylpropanoyl)amino]methyl}-N-[1-(4-ethoxyphenyl)-1H-indazol-4-yl]benzamide

According to the method described in Example 48, using 4-bromophenetole instead of 4-bromoanisole, the title compound was obtained as white powder.
MS (ESI+) m/z 505 (M+H)⁺

### Example 66: 2-Chloro-5-{[(2,2-dimethylpropanoyl)amino]methyl}-N-[1-(3-ethoxyphenyl)-1H-indazol-4-yl]benzamide

According to the method described in Example 48, using 3-bromophenetole instead of 4-bromoanisole, the title compound was obtained as white powder.
MS (ESI+) m/z 505 (M+H)⁺

### Example 67: 2-Chloro-N-(1-(3,4-dimethoxyphenyl)-1H-indazol-4-yl]-5-{[(2,2-dimethylpropanoyl)amino]methyl}benzamide

According to the method described in Example 48, using 4-bromoveratrole instead of 4-bromoanisole, the title compound was obtained as white powder.
MS (ESI+) m/z 521 (M+H)⁺

### Example 68: 2-Chloro-5-{[(2,2-dimethylpropanoyl)amino]methyl}-N-{1-[3-(propan-2-yloxy)phenyl]-1H-indazol-4-yl}benzamide

According to the method described in Example 48, using 1-bromo-3-isopropoxybenzene instead of 4-bromoanisole, the title compound was obtained as light pink powder.
MS (ESI+) m/z 519 (M+H)⁺

### Example 69: 2-Chloro-N-[1-(6-cyclopropylpyridin-3-yl)-1H-indazol-4-yl]-5-{[(2,2-dimethylpropanoyl)amino]methyl}benzamide hydrochloride

According to the method described in Example 54, using 5-bromo-2-cyclopropylpyridine (synthesized according to the method described in WO2006/065215) instead of 5-bromo-2-methylpyridine, the title compound was obtained as white powder.
MS (ESI+) m/z 502 (M+H)⁺

### Example 70: 2-Chloro-N-[1-(2-cyclopropylpyridin-4-yl)-1H-indazol-4-yl]-5-{[(2,2-dimethylpropanoyl)amino]methyl}benzamide hydrochloride

According to the method described in Example 54, using 4-bromo-2-cyclopropylpyridine (synthesized according to the method described in WO2010/125082) instead of 5-bromo-2-methylpyridine, the title compound was obtained as pale yellow powder.
MS (ESI+) m/z 502 (M+H)⁺

### Example 71: 2-Chloro-5-{(2,2-dimethylpropanoyl)amino]methyl}-N-{1-[3-(trifluoromethoxy)phenyl]-1H-indazol-4-yl}benzamide

According to the method described in Example 48, using 1-bromo-3-(trifluoromethoxy)benzene instead of 4-bromoanisole, the title compound was obtained as light pink powder.
MS (ESI+) m/z 545 (M+H)⁺

### Example 72: 2-Chloro-N-[1-(6-cyanopyridin-3-yl)-1H-indazol-4-yl]-5-([(2,2-dimethylpropanoyl)amino]methyl)benzamide

According to the method described in Example 48, using 5-bromo-2-cyanopyridine instead of 4-bromoanisole, the title compound was obtained as light pink powder.
MS (ESI+) m/z 487 (M+H)⁺

### Example 73: 2-Chloro-5-{(2,2-dimethylpropanoyl)amino]methyl}-N-[1-(2-methoxypyridin-4-yl)-1H-indazol-4-yl]benzamide hydrochloride

According to the method described in Example 54, using 4-bromo-2-methoxypyridine instead of 5-bromo-2-methylpyridine, the compound was obtained as light orange powder.
MS (ESI+) m/z 492 (M+H)⁺

### Example 74: 2-Chloro-5-{[(2,2-dimethylpropanoyl)amino]methyl}-N-{1-[6-(methoxymethyl)pyridin-3-yl]-1H-indazol-4-yl}benzamide hydrochloride

According to the method described in Example 54, using 5-bromo-2-(methoxymethyl)pyridine instead of 5-bromo-2-methylpyridine, the title compound was obtained as light orange powder.
MS (ESI+) m/z 506 (M+H)⁺

### Example 75: 2-Chloro-5-{[(2,2-dimethylpropanoyl)amino]methyl}-N-{1-[6-(hydroxymethyl)pyridin-3-yl]-1H-indazol-4-yl}benzamide

According to the method described in Example 54, using 5-bromo-2-hydroxymethylpyridine instead of 5-bromo-2-methylpyridine, the title compound was obtained as light orange powder. MS (ESI+) m/z 492 (M+H)⁺

### Example 76: 2-Chloro-5-{[(2,2-dimethylpropanoyl)amino]methyl]-N-[1-(2,6-dimethylpyridin-4-yl)-1H-indazol-4-yl]benzamide hydrochloride

According to the method described in Example 54, using 4-bromo-2,6-dimethylpyridine instead of 5-bromo-2-methylpyridine, the title compound was obtained as pale yellow powder. MS (ESI+) m/z 490 (M+H)⁺

### Example 77: 2-Chloro-5-{[(2,2-dimethylpropanoyl)amino]methyl}-N-{1-[6-(trifluoromethyl)pyridin-3-yl]-1H-indazol-4-yl}benzamide

According to the method described in Example 48, using 5-bromo-2-(trifluoromethyl)pyridine instead of 4-bromoanisole, the title compound was obtained as light pink powder.
MS (ESI+) m/z 530 (M+H)⁺

### Example 78: 2-Chloro-5-{[(2,2-dimethylpropanoyl)amino]methyl}-N-{1-[5-(methoxymethyl)pyridin-3-yl]-1H-indazol-4-yl}benzamide hydrochloride

According to the method described in Example 54, using 3-bromo-5-(methoxymethyl)pyridine (synthesized according to the method described in US5861423) instead of 5-bromo-2-methylpyridine, the title compound was obtained as pale yellow powder.
MS (ESI+) m/z 506 (M+H)⁺

### Example 79: 2-Chloro-5-{[(2,2-dimethylpronanoyl)amino]methyl}-N-{1-[2-methyl-4-(trifluoromethoxy)phenyl]-1H-indazol-4-yl}benzamide

According to the method described in Example 48, using 1-bromo-2-methyl-4-(trifluoromethoxy)benzene instead of 4-bromoanisole, the title compound was obtained as white powder.
MS (ESI+) m/z 559 (M+H)⁺

### Example 80: 2-Chloro-5-{[(2,2-dimethylpropanoyl)amino]methyl}-N-[1-(1,3-thiazol-2-yl)-1H-indazol-4-yl]benzamide

According to the method described in Example 48, using 2-bromothiazole instead of 4-bromoanisole, the title compound was obtained as white powder.
MS (ESI+) m/z 468 (M+H)⁺
¹H-NMR (DMSO-d₆) δ: 1.13 (9H, s), 4.30 (2H, d), 7.37 (1H, d), 7.53 (2H, m), 7.64 (1H, t), 7.73 (1H, d), 7.93 (1H, d), 8.16 (1H, t), 8.30 (1H, d), 8.66 (1H, s), 10.94 (1H, s).

### Example 81: 2-Chloro-N-{1-[3-(difluoromethoxy)phenyl]-1H-indazol-4-yl}-5-{[(2,2-dimethylpropanoyl)amino]methyl}benzamide

According to the method described in Example 48, using 3-(difluoromethoxy)bromobenzene instead of 4-bromoanisole, the title compound was obtained as white powder.
MS (ESI+) m/z 527 (M+H)⁺

### Example 82: 2-Chloro-5-{[(2,2-dimethylpropanoyl)amino]methyl}-N-{1-[3-methyl-4-(trifluoromethoxy)phenyl]-1H-indazol-4-yl}benzamide

According to the method described in Example 48, using 5-bromo-2-(trifluoromethoxy)toluene instead of 4-bromoanisole, the title compound was obtained as white powder.
MS (ESI+) m/z 559 (M+H)⁺

### Example 83: 2-Chloro-5-{[(2,2-dimethylpropanoyl)amino]methyl}-N-[1-(1,3-thiazol-4-yl)-1H-indazol-4-yl]benzamide

According to the method described in Example 48, using 4-bromothiazole instead of 4-bromoanisole, the title compound was obtained as white powder.
MS (ESI+) m/z 468 (M+H)⁺
¹H-NMR (DMSO-d₆) δ: 1.10 (9H, s), 4.27 (2H, d), 7.33 (1H, d), 7.49 (3H, m), 7.73 (1H, d), 7.82 (1H, d), 8.12 (2H, m), 8.56 (1H, s), 9.26 (1H, d), 10.80 (1H, s).

### Example 84: 2-Chloro-5-{[(2,2-dimethylpropanoyl)amino]methyl}-N-{1-[6-(2-methoxypropan-2-yl)pyridin-3-yl]-1H-Indazol-4-yl)benzamide hydrochloride

According to the method described in Example 54, using 5-bromo-2-(2-methoxypropan-2-yl)pyridine (synthesized according to the method described in WO 2006/048727) instead of 5-bromo-2-methylpyridine, the title compound was obtained as white powder.
MS (ESI+) m/z 534 (M+H)⁺

### Example 85: 2-Chloro-5-{[(2,2-dimethylpropanoyl)amino]methyl}-N-{1-[6-(2-fluoropropan-2-yl)pyridin-3-yl]-1H-indazol-4-yl}benzamide

According to the method described in Example 48, using 5-bromo-2-(2-fluoropropan-2-yl)pyridine instead of 4-bromoanisole, the title compound was obtained as white powder.
MS (ESI+) m/z 522 (M+H)⁺

### Example 86: 2-Chloro-5-{[(2,2-dimethylpropanoyl)amino]methyl}-N-{1-[6-(ethoxymethyl)pyridin-3-yl]-1H-indazol-4-yl}benzamide hydrochloride

According to the method described in Example 54, using 5-bromo-2-(ethoxymethyl)pyridine (synthesized according to the method described in WO2011/051342) instead of 5-bromo-2-methylpyridine, the title compound was obtained as white powder.
MS (ESI+) m/z 520 (M+H)⁺

### Example 87: 2-Chloro-N-[1-(6-methylpyridin-3-yl)-1H-indazol-4-yl]-5-{[(trifluoroacetyl)amino]methyl)benzamide hydrochloride

According to the method described in Example 48, using 5-bromo-2-methylpyridine instead of 4-bromoanisole and using 2-chloro-5-{[(2,2,2-trifluoroacetyl)amino]methyl}benzoic acid instead of 2-chloro-5-{[(2,2-dimethylpropanoyl)amino]methyl}benzoic acid, 2-chloro-N-[1-(6-methylpyridin-3-yl)-1H-indazol-4-yl]-5-([(trifluoroacetyl)amino]methyl}benzamide (15 mg) was obtained. The resulting compound was dissolved in ethyl acetate (0.5 mL), then 4 N hydrogen chloride/ethyl acetate (1 equivalent) was added and the mixture was stirred for 5 minutes. The precipitated solid was filtered off and dried to afford the title compound (11 mg) as white powder.
MS (ESI+) m/z 488 (M+H)⁺

### Example 88: 5-{[(2,2-Dimethylpropanoyl)amino]methyl}-N-[1-(6-methylpyridin-3-yl)-1H-indazol-4-yl]-2-(trifluoromethyl)benzamide hydrochloride

According to the method described in Example 48, using 5-bromo-2-methylpyridine instead of 4-bromoanisole and using 5-{[(2,2-dimethylpropanoyl)amino]methyl}-2-(trifluoromethyl)benzoic acid (synthesized according to the method described in WO2011/048004) instead of 2-chloro-5-{[(2,2-dimethylpropanoyl)amino]methyl]}benzoic acid, 5-{[(2,2-dimethylpropanoyl)amino]methyl}-N-[1-(6-methylpyridin-3-yl)-1H-indazol-4-yl]-2-(trifluoromethyl)benzamide was obtained. The resulting compound was dissolved in ethyl acetate (0.5 mL), 4 N hydrogen chloride/ethyl acetate (1 equivalent) was added thereto, and then the mixture was stirred for 5 minutes. The precipitated solid was filtered off and dried to give the title compound as white powder.
MS (ESI+) m/z 510 (M+H)⁺
Elemental analysis value: C₂₇H₂₆F₃N₅O₂·HCl
Calculated value (%) C: 59.40, H: 4.98, N: 12.83
Measured values (%) C: 59.28, H: 4.68, N: 12.57

### Example 89: 2-Chloro-5-{[(2,2-dimethylpropanoyl)amino]methyl}-N-[1-(3-methoxybenzyl)-1H-indazol-4-yl]benzamide

### Step 1: 1-[(3-Methoxyphenyl)methyl]-4-nitro-1H-indazole

The title compound was prepared according to the method described in WO2011/079076. 4-Nitro-1H-indazole (48 mg), 3-methoxybenzylchloride (69 mg) and potassium carbonate (61 mg) in DMF were stirred at 70°C for 16 hours. After the reaction solution was brought to room temperature, water was added and the mixture was extracted with ethyl acetate. The organic layer was washed with saturated saline, dried over magnesium sulfate, and concentrated under reduced pressure. The residue was purified by silica gel column chromatography (15% ethyl acetate/hexane → 20% ethyl acetate/hexane) to afford 1-[(3-methoxyphenyl)methyl]-4-nitro-1H-indazole (35 mg) from the first eluted fraction and 2-[(3-methoxyphenyl)methyl]-4-nitro-2H-indazole (28 mg) from the second eluted fraction.
1-[(3-methoxyphenyl)methyl]-4-nitro-1H-indazole: ¹H-NMR (CDCl₃): δ 3.74 (3H, s), 5.66 (2H, s), 6.72 (1H, s), 6.79 (2H, m), 7.26 (1H, m), 7.45 (1H, t, J = 7.8 Hz), 7.79 (1H, d, J = 8.3 Hz), 8.14 (1H, d, J = 7.3 Hz), 8.68 (1H, s)
2-[(3-methoxyphenyl)methyl]-4-nitro-2H-indazole: ¹H-NMR (CDCl₃): δ 3.79 (3 H, s), 5.63 (2H, s), 6.87 (1H, s), 6.90 (2H, m), 7.30 (1H, t, J = 7.8 Hz), 7.41 (1H, t, J = 8.3 Hz), 8.10 (1H, d, J = 7.8 Hz), 8.18 (1H, d, J = 7.3 Hz), 8.57 (1H, s)

### Step 2: 4-Amino-1-[(3-methoxyphenyl)methyl]-1H-indazole

A suspension of 1-[(3-methoxyphenyl)methyl]-4-nitro-1H-indazole (35 mg), zinc powder (65 mg), MeOH (1 mL), THF (1 mL) and saturated aqueous ammonium chloride (0.5 mL) was stirred at 50°C for 1.5 hours. After the reaction solution was brought to room temperature, the insoluble material was filtered off and the mother liquor was concentrated. The residue was purified by silica gel column chromatography to afford the title compound (22 mg) as a yellow solid.
MS (ESI+) m/z 254 (M+H)⁺

### Step 3: 2-Chloro-5-{[(2,2-dimethylpropanoyl)amino]methyl}-N-[1-(3-methoxybenzyl)-1H-indazol-4-yl]benzamide

According to the method described in Step 2 of Example 48, using 4-amino-1-[(3-methoxyphenyl)methyl]-1H-indazole instead of 4-amino-1-(4-methoxyphenyl)-1H-indazole, the title compound was obtained as light pink powder.
MS (ESI+) m/z 505 (M+H)⁺
¹H-NMR (DMSO-d₆) δ: 1.12 (9H, s), 3.69 (3H, s), 4.29 (2H, d), 5.61 (2H, s), 6.73 (1H, d), 6.80 (1H, s), 6.81 (1H, d), 7.20 (1H, t), 7.35 (1H, m), 7.50 (3H, m), 7.73 (1H, d), 8.18 (1H, t), 8.32 (1H, s), 10.72 (1H, s).

### Example 90: 2-Chloro-5-{[(2,2-dimethylpropanoyl)amino]methyl}-N-[1-(2-methoxybenzyl)-1H-indazol-4-yl]benzamide

According to the method described in Example 89, using 2-methoxybenzylchloride instead of 3-methoxybenzylchloride, the title compound was obtained as light pink powder.
MS (ESI+) m/z 505 (M+H)⁺

### Example 91: 2-Chloro-5-{[(2,2-dimethylpropanoyl)amino]methyl}-N-[1-(2,4,6-trimethylbenzyl)-1H-indazol-4-yl]benzamide

According to the method described in Example 89, using 2,4,6-trimethylbenzylchloride instead of 3-methoxybenzylchloride, the title compound was obtained as light pink powder.
MS (ESI+) m/z 517 (M+H)⁺

### Example 92: 2-Chloro-5-{[(2,2-dimethylpropanoyl)amino]methyl}-N-{1-[4-(trifluoromethyl)benzyl]-1H-indazol-4-yl}benzamide

According to the method described in Example 89, 4-(trifluoromethyl)benzyl bromide was used instead of 3-methoxybenzyl chloride to afford 1-[4-(trifluoromethyl)benzyl]-4-nitro-1H-indazole (65 mg) and 2-[4-(trifluoromethyl)benzyl]-4-nitro-2H-indazole (63 mg). According to the method described in Steps 2 and 3 in Example 89, using 1-[4-(trifluoromethyl)benzyl]-4-nitro-1H-indazole, the title compound was obtained as white powder.
MS (ESI+) m/z 543 (M+H)⁺

### Example 93: 2-Chloro-5-{[(2,2-dimethylpropanoyl)amino]methyl}-N-{1-[4-(trifluoromethyl)benzyl]-2H-indazol-4-yl}benzamide

The title compound was obtained according to the method described in Example 92.
MS (ESI+) m/z 543 (M+H)⁺

### Example 94: 2-Chloro-5-{[(2,2-dimethylpropanoyl)amino]methyl}-N-{1-[3-(trifluoromethyl)benzyl]-1H-indazol-4-yl}benzamide

According to the method described in Example 89, using 3-(trifluoromethyl)benzyl bromide instead of 3-methoxybenzyl chloride, the title compound was obtained as light pink powder. MS (ESI+) m/z 543 (M+H)⁺

### Example 95: 2-Chloro-5-{[(2,2-dimethylpropanoyl)amino]methyl}-N-[1-(2-phenylethyl)-1H-indazol-4-yl]benzamide

According to the method described in Example 89, using 2-chloroethylbenzene instead of 3-methoxybenzylchloride, 1-(2-phenylethyl)-4-nitro-1H-indazole (32 mg) and 2-(2-phenylethyl)-4-nitro-2H-indazole (26 mg) were obtained. According to the method described in Steps 2 and 3 of Example 89, using 1-(2-phenylethyl)-4-nitro-1H-indazole, the title compound was obtained as white powder.
MS (ESI+) m/z 489 (M+H)⁺

### Example 96: 2-Chloro-5-{[(2,2-dimethylpropanoyl)amino]methyl}-N-[1-(2-phenylethyl-2H-indazol-4-yl]benzamide

The title compound was obtained according to the method described in Example 95.
MS (ESI+) m/z 489 (M+H)⁺

### Example 97: 2-Chloro-N-(1-cyclopentyl-1H-indazol-4-yl)-5- {[(2,2-dimethylpropanoyl)amino]methyl}benzamide

According to the method described in Example 89, using cyclopentyl bromide instead of 3-methoxybenzyl chloride, the title compound was obtained as white powder.
MS (ESI+) m/z 453 (M+H)⁺
¹H-NMR (DMSO-d₆) δ: 1.12 (9H, s), 1.68 (2H, m), 1.85 (2H, m), 1.95 (2H, m), 2.10 (2H, m), 4.28 (2H, d), 5.13 (1H, quin.), 7.33 (2H, m), 7.45 (2H, m), 7.51 (1H, d), 7.70 (1H, d), 8.17 (1H, t), 8.26 (1H, s), 10.68 (1H, s).

### Example 98: 2-Chloro-N-(1-cyclohexyl-1H-indazol-4-yl)-5-{[(2,2-dimethylpropanoyl)amino]methyl}benzamide

According to the method described in Example 89, using cyclohexyl bromide instead of 3-methoxybenzyl chloride, the title compound was obtained as white powder.
MS (ESI+) m/z 467 (M+H)⁺

### Example 99: 2-Chloro-N-[1-(cyclobutylmethyl)-1H-indazol-4-yl]-5-{[(2,2-dimethylpropanoyl)amino]methyl}benzamide

According to the method described in Example 89, using bromomethylcyclobutane instead of 3-methoxybenzyl chloride, the title compound was obtained as white powder.
MS (ESI+) m/z 453 (M+H)⁺

### Example 100: 2-Chloro-N-[1-(cyclohexylmethyl)-1H-indazol-4-yl]-5-{[(2,2-dimethylpropanoyl)amino]methyl}benzamide

According to the method described in Example 89, using bromomethylcyclohexane instead of 3-methoxybenzyl chloride, the title compound was obtained as white powder.
MS (ESI+) m/z 481 (M+H)⁺
¹H-NMR (DMSO-d₆) δ: 1.01 (1H, m), 1.12 (9H, s), 1.10-1.29 (4H, m), 1.45 (2H, m), 1.53-1.67 (3H, m), 1.87 (1H, m), 4.22 (2H, d), 4.20 (2H, d), 7.34 (2H, m), 7.43 (2H, m), 7.51 (1H, d), 7.70 (1H, d), 8.25 (1H, s).

### Example 101: 2-Chloro-5-{[(2,2-dimethylpropanoyl)amino]methyl]-N-[1-(3-phenylpropyl)-1H-indazol-4-yl]benzamide

According to the method described in Example 89, using (3-bromopropyl)benzene instead of 3-methoxybenzylchloride, the title compound was obtained as white powder.
MS (ESI+) m/z 503 (M+H)⁺

### Example 102: 2-Chloro-5-{[(2,2-dimethylpropanoyl)amino]methyl}-N-[1-(propan-2-yl)-1H-indazol-4-yl]benzamide

According to the method described in Example 89, using 2-bromopropane instead of 3-methoxybenzyl chloride, the title compound was obtained as white powder.
MS (ESI+) m/z 427 (M+H)⁺
¹H-NMR (DMSO-d₆) δ: 1.12 (9H, s), 1.46 (6H, d), 2.29 (2H, d), 4.96 (1H, quin.), 7.34 (2H, m), 7.45 (2H, m), 7.51 (1H, d), 7.70 (1H, d), 8.17 (1H, t), 8.27 (1H, d), 10.67 (1H, s).

### Example 103: tert-Butyl 4-(4-{[(2-chloro-5-{[(2,2-dimethylpropanoyl)amino]methyl}phenyl)carbonyl]amino}-1H-indazol-1-yl)piperidine-1-carboxylate

### Step 1: tert-Butyl 4-(4-nitro-1H-indazol-1-yl)piperidine-1-carboxylate

A solution of 4-nitro-1H-indazole (100 mg), tert-butyl 4-hydroxypiperidine-1-carboxylate (148 mg) and triphenylphosphine (241 mg) in THF (6 mL) was stirred under ice-cooling, and 40% diethyl azodicarboxylate/toluene solution (w/w, 0.42 mL) was added dropwise, then the mixture was stirred overnight at room temperature. The solvent was removed under reduced pressure and the residue was purified by silica gel column chromatography to afford the title compound (51 mg) as pale yellow powder.

### Step 2: tert-Butyl 4-(4-amino-1H-indazol-1-yl)piperidine-1-carboxylate

To a solution of tert-butyl 4-(4-nitro-1H-indazol-1-yl)piperidine-1-carboxylate (51 mg) in MeOH (5 mL) - THF (5 mL) was added 5% palladium carbon (20 mg), and the mixture was stirred at 0.3 MPa under hydrogen atmosphere. After 4 h, the reaction solution was filtered through celite, and the mother liquor was concentrated. The residue was purified by silica gel column chromatography to afford the title compound (43 mg) as pale yellow powder.

### Step 3: tert-Butyl 4-(4-{[(2-chloro-5-{[(2,2-dimethylpropanoyl)amino]methyl}phenyl)carbonyl]amino}-1H-indazol-1-yl)piperidine-1-carboxylate

According to the method described in Step 2 of Example 48, using tert-butyl 4-(4-amino-1H-indazol-1-yl)piperidine-1-carboxylate instead of 4-amino-1-(4-methoxyphenyl)-1H-indazole, the title compound was obtained as white powder.
MS (ESI+) m/z 568 (M+H)⁺
¹H-NMR (DMSO-d₆) δ: 1.12 (9H, s), 1.42 (9H, s), 1.91 (4H, m), 3.23 (2H, m), 4.10 (2H, m), 4.29 (2H, d), 4.84 (1H, quin.), 7.35 (2H, m), 7.44 (1H, s), 7.51 (2H, m), 8.17 (1H, m), 8.28 (1H, s), 10.68 (1H, s).

### Example 104: 2-Chloro-5-{[(2,2-dimethylpropanoyl)amino]methyl}-N-[1-(tetrahydro-2H-pyran-4-yl)-1H-indazol-4-yl]benzamide

According to the method described in Example 103, using tetrahydro-2H-pyran-4-ol instead of tert-butyl 4-hydroxypiperidine-1-carboxylate, the title compound was obtained as white powder.
MS (ESI+) m/z 469 (M+H)⁺
¹H-NMR (DMSO-d₆) δ: 1.12 (9H, s), 1.89 (2H, m), 2.10 (2H, m), 3.56 (2H, t), 4.01 (2H, m), 4.30 (2H, d), 4.87 (1H, quin.), 7.36 (2H, m), 7.45 (1H, s), 7.51 (2H, m), 7.71 (1H, d), 8.17 (1H, t), 8.29 (1H, s), 10.69 (1H, s).

### Example 105: 2-Chloro-5-{[(2,2-dimethylpropanoyl)amino]methyl}-N-[1-(tetrahydro-2H-pyran-2-ylmethyl)-1H-indazol-4-yl]benzamide

According to the method described in Example 89, using 2-(bromomethyl)tetrahydro-2H-pyran instead of 3-methoxybenzylchloride, the title compound was obtained as white powder. MS (ESI+) m/z 483 (M+H)⁺

### Example 106: N-[1-(1-acetylpiperidin-4-yl)-1H-indazol-4-yl]-2-chloro-5-{[(2,2-dimethylpropanoyl)amino]methyl}benzamide

### Step 1: 2-Chloro-5-{[(2,2-dimethylpropanoyl)amino]methyl}-N-[1-(piperidin-4-yl)-1H-indazol-4-yl]benzamide hydrochloride

A solution of tert-butyl 4-(4-{[(2-chloro-5-{[(2,2-dimethylpropanoyl)amino]methyl}phenyl)carbonyl]amino}-1H-indazol-1-yl)piperidine-1-carboxylate (41 mg) in ethyl acetate (2 mL) was stirred at room temperature, and 4 N hydrogen chloride/ethyl acetate (2 mL) was added thereto, and then the mixture was stirred for 1 hour. After removing the solvent under reduced pressure, the residue was solidified with diethyl ether-MeOH to afford the title compound (32 mg) as white powder.
MS (ESI+) m/z 468 (M+H)⁺

### Step 2: N-[1-(1-acetylpiperidin-4-yl)-1H-indazol-4-yl]-2-chloro-5-{[(2,2-dimethylpropanoyl)amino]methyl}benzamide

To a solution of 2-chloro-5-{[(2,2-dimethylpropanoyl)amino]methyl}-N-[1-(piperidin-4-yl)-1H-indazol-4-yl]benzamide hydrochloride (13 mg) in THF (0.5 mL) was added pyridine (7 µL) and the mixture was stirred under ice-cooling. To this solution was added acetyl chloride (3 µL) dropwise, and the mixture was stirred at room temperature for 2 hours. Aqueous saturated sodium bicarbonate was added to the reaction solution, and the mixture was extracted with ethyl acetate. The organic layer was washed with water and saturated saline in this order, dried over magnesium sulfate, and concentrated. The residue was purified by silica gel column chromatography to afford the title compound (7 mg) as white powder.
MS (ESI+) m/z 510 (M+H)⁺

### Example 107: 2-Chloro-5-{[(2,2-dimethylpropanoyl)amino]methyl}-N-{1-[2-(morpholin-4-yl)ethyl]-1H-indazol-4-yl)benzamide hydrochloride

According to the method described in Example 103, using 2-morpholinoethanol instead of tert-butyl 4-hydroxypiperidine-1-carboxylate, 2-chloro-5-{[(2,2-dimethylpropanoyl)amino]methyl}-N-{1-[2-(morpholin-4-yl)ethyl]-1H-indazol-4-yl}benzamide (33 mg) was obtained. The product was dissolved in THF (1 mL), 2 N hydrogen chloride/MeOH (1 equivalent) was added, and then the solvent was removed under reduced pressure. The residue was powdered from diethyl ether-THF to afford the title compound (29 mg) as white powder.
MS (ESI+) m/z 498 (M+H)⁺

### Example 108: 2-Chloro-5-{[(2,2-dimethylpropanoyl)aminolmethyl}-N-[1-(4-phenylbutyl)-1H-indazol-4-yllbenzamide

According to the method described in Example 103, using 4-phenylbutane-1-ol instead of tert-butyl 4-hydroxypiperidine-1-carboxylate, the title compound was obtained as white powder. MS (ESI+) m/z 517 (M+H)⁺

### Example 109: N-(1-benzyl-1H-indazol-4-yl)-2-chloro-5-{[(cyclopropylcarbonyl)amino]methyl}benzamide

### Step 1: 1-Benzyl-4-nitro-1H-indazole

1-Benzyl-4-nitro-1H-indazole and 2-benzyl-4-nitro-2H-indazole were obtained according to the method described in Example 89, using benzyl bromide instead of 3-methoxybenzyl chloride.

### Step 2: 4-Amino-1-benzyl-1H-indazole

The title compound was obtained according to the method described in Step 2 of Example 89, using 1-benzyl-4-nitro-1H-indazole instead of 1-[(3-methoxyphenyl)methyl]-4-nitro-1H-indazole.

### Step 3: N-(1-benzyl-1H-indazol-4-yl)-2-chloro-5-{[(cyclopropylcarbonyl)amino]methyl} benzamide

According to the method described in Step 2 of Example 1, using 4-amino-1-benzyl-1H-indazole instead of 4-amino-1-(4-trifluoromethylphenyl)-1H-indazole, the title compound was obtained as white powder.
MS (ESI+) m/z 459 (M+H)⁺

### Example 110: N-(1-benzyl-2H-indazol-4-yl)-2-chloro-5-{(cyclopropylcarbonyl)aminolmethyl}benzamide

The title compound was obtained according to the method described in Example 109.
MS (ESI+) m/z 459 (M+H)⁺

### Example 111: 2-Chloro-5-{(2,2-dimethylpropanoyl)amino]methyl}-N-(1-methyl-1H-indazol-4-yl)benzamide

According to the method described in Example 89, using iodomethane instead of 3-methoxybenzylchloride, the title compound was obtained as white powder.
MS (ESI+) m/z 399 (M+H)⁺
¹H-NMR (DMSO-d₆) δ: 1.12 (9H, s), 4.01 (3H, s), 4.29 (2H, d), 7.36 (3H, m), 7.46 (1H, s), 7.51 (1H, d), 7.73 (1H, d), 8.16 (1H, t), 8.24 (1H, s), 10.68 (1H, s).

### Example 112: 2-Chloro-5-{[(cyclopropylcarbonyl)amino]methyl}-N-(1-methyl-1H-indazol-4-yl)benzamide

According to the method described in Step 2 of Example 1, using 4-amino-1-methyl-1H-indazole (synthesized according to the method described in WO2004/046133) instead of 4-amino-1-(4-trifluoromethylphenyl)-1H-indazole, the title compound was obtained as white powder.
MS (ESI+) m/z 383 (M+H)⁺

### Example 113: 2-Chloro-5-{[(2,2-dimethylpropanoyl)amino]methyl]-N-(1-ethyl-1H-indazol-4-yl)benzamide

According to the method described in Example 89, using iodoethane instead of 3-methoxybenzylchloride, the title compound was obtained as white powder.
MS (ESI+) m/z 413 (M+H)⁺
¹H-NMR (DMSO-d₆) δ: 1.12 (9H, s), 1.38 (3H, t), 4.29 (2H, d), 4.41 (2H, q), 7.36 (2H, m), 7.45 (2H, m), 7.51 (1H, d), 7.70 (1H, d), 8.16 (1H, t), 8.25 (1H, s), 10.67 (1H, s).

### Example 125: 2-Chloro-5-{[(3-hydroxy-2,2-dimethylpropanoyl)amino]methyl]-N-{1-[3-(trifluoromethoxy)phenyl]-1H-indazol-4-yl[benzamide

### [Step 1] 4-Amino-1-(3-(trifluoromethoxy)phenyl)-1H-indazole

The title compound was obtained according to the method described in Step 1 of Example 1, using 1-bromo-3-(trifluoromethoxy)benzene instead of 4-iodobenzotrifluoride.
MS (ESI+) m/z 294 (M+H)⁺

### [Step 2] tert-Butyl-N-[4-chloro-3-({1-[3-(trifluoromethoxy)phenyl]-1H-indazol-4-yl}carbamoyl)benzyl]carbamate

5-[(tert-Butoxycarbonyl)amino]methyl-2-chlorobenzoic acid (300 mg) and 4-amino-1-[3-(trifluoromethoxy)phenyl]-1H-indazole (323 mg) were suspended in acetonitrile (5 ml) and the mixture was stirred at room temperature. To this suspension were added chloro-N,N,N',N'-tetramethylformamidinium hexafluorophosphate (442 mg) and 1-methylimidazole (431 mg) and the mixture was stirred overnight. The reaction solution was poured into water and extracted with ethyl acetate. The organic layer was separated, then washed with water and saturated saline in this order, dried over magnesium sulfate, and concentrated under reduced pressure. The residue was purified by silica gel column chromatography to afford the title compound (539 mg) as pale yellow solid.
MS (ESI-) m/z 559 (M-H)⁺

### [Step 3] 2-Chloro-5-{[(3-hydroxy-2,2-dimethylpropanoyl)amino]methyl}-N-{1-[3-(trifluoromethoxy)phenyl]-1H-indazol-4-yl}benzamide

t-Butyl-N-[4-chloro-3-({1-[3-(trifluoromethoxy)phenyl]-1H-indazol-4-yl}carbamoyl)benzyl]carbamate (530 mg) was suspended in ethyl acetate (5 ml) and the mixture was stirred at room temperature. To this suspension was added 4 N hydrochloric acid/ethyl acetate (5 ml) and the mixture was stirred for 3 hours. The solvent was removed under reduced pressure and the residue was suspended in ethyl acetate. The solid was filtered off and dried to give white solid (430 mg). This solid was dissolved in DMF (8 ml), and 3-hydroxy-2,2-dimethylpropanoic acid (133 mg), HATU (493 mg) and N,N-diisopropylethylamine (0.449 ml) were added while stirring at room temperature overnight. The reaction solution was diluted with water and extracted with ethyl acetate. The organic layer was separated, washed with water and saturated saline in this order, dried over magnesium sulfate, and concentrated under reduced pressure. The residue was purified by silica gel column chromatography to afford the title compound (451 mg) as white solid.
MS (ESI+) m/z 561 (M+H)⁺

### Example 276: 5-{[(2,2-Dimethylpropanoyl)amino]methyl}-N-[-(6-methoxvpyridin-3-yl)-1H-indazol-4-yll-2-(trifluoromethyl)benzamide

### [Step 1] 4-Amino-[1-(6-methoxypyridin-3-yl)-1H-indazole

The title compound was obtained according to the method described in Step 1 of Example 1, using 5-bromo-2-methoxypyridine instead of 4-iodobenzotrifluoride.
MS (ESI+) m/z 241 (M+H)⁺

### [Step 2] 5-{[(2,2-Dimethylpropanoyl)amino]methyl}-N-[1-(6-methoxypyridin-3-yl)-1H-indazol-4-yl]-2-(trifluoromethyl)benzamide

The title compound was obtained according to the method described in Step 2 of Example 125, using 5-[(2,2-dimethylpropanoylamino)methyl]-2-(trifluoromethyl)benzoic acid instead of 5-[(tert-butoxycarbonyl)amino]methyl-2-chlorobenzoic acid and using 4-amino-[1-(6-(methoxypyridin-3-yl)-1H-indazole instead of 4-amino-1-[3-(trifluoromethoxy)phenyl]-1H-indazole.
MS (ESI+) m/z 526 (M+H)⁺

The following compounds were prepared by using the same reaction and treatment as described in Example 18 or Example 89, using a corresponding starting material.

### Example 114: 2-Chloro-5-{[(3,3-dimethylbutanoyl)amino]methyl)-N-(1-[4-(trifluoromethoxy)phenyl]-1H-indazol-4-yl}benzamide

The title compound was obtained by using the reaction and treatment as described in Example 18.
MS (ESI+) m/z 559 (M+H)⁺

### Example 115: 2-Chloro-5-{[(cyclopentylcarbonyl)amino]methyl}-N-{1-[4-(trifluoromethoxy)phenyl]-1H-indazol-4-yl}benzamide

The title compound was obtained by using the reaction and treatment as described in Example 18.
MS (ESI+) m/z 557 (M+H)⁺

### Example 116: 2-Chloro-N-{1-[4-(trifluoromethoxy)phenyl]-1H-indazol-4-yl}-5-[(([1-(trifluoromethyl)cyclopropyl]carbonyl)amino)methyl]benzamide

The title compound was obtained by using the reaction and treatment as described in Example 18.
MS (ESI+) m/z 597 (M+H)⁺

### Example 117: 2-Chloro-5-({[(1-hydroxycyclopropyl)carbonyl]amino}methyl)-N-{1-[4-(trifluoromethoxy)phenyl]-1H-indazol-4-yl}benzamide

The title compound was obtained by using the reaction and treatment as described in Example 18.
MS (ESI+) m/z 545 (M+H)⁺

### Example 118: 2-Chloro-5-{[(methoxyacetyl)aminolmethyl)-N-{1-[4-(trifluoromethoxy)phenyl]-1H-indazol-4-yl}benzamide

The title compound was obtained by using the reaction and treatment as described in Example 18.
MS (ESI+) m/z 533 (M+H)⁺

### Example 119: 2-Chloro-5-{[(3-hydroxy-2,2-dimethylpropanoyl)amino]methyl)-N-{1-[4-(trifluoromethoxy)phenyl]-1H-indazol-4-yl}benzamide

The title compound was obtained by using the reaction and treatment as described in Example 18.
MS (ESI+) m/z 561 (M+H)⁺

### Example 120: 2-Chloro-5-{[(cyclopropylsulfonyl)amino]methyl}-N-{1-[4-(trifluoromethoxy)phenyl]-1H-indazol-4-yl}benzamide

The title compound was obtained by using the reaction and treatment as described in Example 18.
MS (ESI+) m/z 565 (M+H)⁺

### Example 121: 2-Chloro-5-{[(3,3-dimethylbutanoyl)amino]methyl)-N-{1-[3-(trifluoromethoxy)phenyl]-1H-indazol-4-yl}benzamide

The title compound was obtained by using the reaction and treatment as described in Example 18.
MS (ESI+) m/z 559 (M+H)⁺

### Example 122: 2-Chloro-5-{[(cyclopentylcarbonyl)amino]methyl)-N-{1-[3-(trifluoromethoxy)phenyl]-1H-indazol-4-yl}benzamide

The title compound was obtained by using the reaction and treatment as described in Example 18.
MS (ESI+) m/z 557 (M+H)⁺

### Example 123: 2-Chloro-N-{1-[3-(trifluoromethoxy)phenyl]-1H-indazol-4-yl}-5-[({[1-(trifluoromethyl)cyclopropyl]carbonyl}amino)methyl]benzamide

The title compound was obtained by using the reaction and treatment as described in Example 18.
MS (ESI+) m/z 597 (M+H)⁺

### Example 124: 2-Chloro-5-{[(methoxyacetyl)amino]methyl}-N-(1-[3-(trifluoromethoxy)phenyl]-1H-indazol-4-yl}benzamide

The title compound was obtained by using the reaction and treatment as described in Example 18.
MS (ESI+) m/z 533 (M+H)⁺

### Example 126: 2-Chloro-5-{[(cyclopropylsulfonyl)amino]methyl}-N-{1-[3-(trifluoromethoxy)phenyl]-1H-indazol-4-yl}benzamide

The title compound was obtained by using the reaction and treatment as described in Example 18.
MS (ESI+) m/z 565 (M+H)⁺

### Example 127: 2-Chloro-N-[1-(3-chloro-4-methylphenyl)-1H-indazol-4-yl]-5-{[(3,3-dimethylbutanoyl)amino]methyl}benzamide

The title compound was obtained by using the reaction and treatment as described in Example 18.
MS (ESI+) m/z 523 (M+H)⁺

### Example 128: 2-Chloro-N-[1-(3-chloro-4-methylphenyl)-1H-indazol-4-yl]-5-{[(cyclopentylcarbonyl)amino]methyl)benzamide

The title compound was obtained by using the reaction and treatment as described in Example 18.
MS (ESI+) m/z 521 (M+H)⁺

### Example 129: 2-Chloro-N-[1-(3-chloro-4-methylphenyl)-1H-indazol-4-yl]-5-[({[1-(trifluoromethyl)cyclopropyl]carbonyl}amino)methyl]benzamide

The title compound was obtained by using the reaction and treatment as described in Example 18.
MS (ESI+) m/z 561 (M+H)⁺

### Example 130: 2-Chloro-N-[1-(3-chloro-4-methylphenyl)-1H-indazol-4-yl]-5-({[(1-hydroxycyclopropyl)carbonyl]amino}methyl)benzamide

The title compound was obtained by using the reaction and treatment as described in Example 18.
MS (ESI+) m/z 509 (M+H)⁺

### Example 131: 2-Chloro-N-[1-(3-chloro-4-methylphenyl)-1H-indazol-4-yl]-5-{[(methoxyacetyl)amino]methyl}benzamide

The title compound was obtained by using the reaction and treatment as described in Example 18.
MS (ESI+) m/z 497 (M+H)⁺

### Example 132: 2-Chloro-N-[1-(3-chloro-4-methylphenyl)-1H-indazol-4-yl]-5-{[(cyclopropylsulfonyl)amino]methyl}benzamide

The title compound was obtained by using the reaction and treatment as described in Example 18.
MS (ESI+) m/z 529 (M+H)⁺

### Example 133: 2-Chloro-5-{[(3,3-dimethylbutanoyl)amino]methyl)-N-{1-[3-methyl-4-(trifluoromethoxy)phenyl]-1H-indazol-4-yl}benzamide

The title compound was obtained by using the reaction and treatment as described in Example 18.
MS (ESI+) m/z 573 (M+H)⁺

### Example 134: 2-Chloro-5-{[(cyclopentylcarbonyl)amino]methyl}-N-{1-[3-methyl-4-(trifluoromethoxy)phenyl]-1H-indazol-4-yl)benzamide

The title compound was obtained by using the reaction and treatment as described in Example 18.
MS (ESI+) m/z 571 (M+H)⁺

### Example 135: 2-Chloro-5-{[(methoxyacetyl)amino]methyl}-N-{1-[3-methyl-4-(trifluoromethoxy)phenyl]-1H-indazol-4-yl}benzamide

The title compound was obtained by using the reaction and treatment as described in Example 18.
MS (ESI+) m/z 547 (M+H)⁺

### Example 136: 2-Chloro-5-{[(3-hydroxy-2,2-dimethylpropanoyl)amino]methyl)-N-(1-[3-methyl-4-(trifluoromethoxy)phenyl]-1H-indazol-4-yl}benzamide

The title compound was obtained by using the reaction and treatment as described in Example 18.
MS (ESI+) m/z 575 (M+H)⁺

### Example 137: 2-Chloro-5-{[(cyclopropylsulfonyl)amino]methyl}-N-{1-[3-methyl-4-(trifluoromethoxy)phenyl]-1H-indazol-4-yl}benzamide

The title compound was obtained by using the reaction and treatment as described in Example 18.
MS (ESI+) m/z 579 (M+H)⁺

### Example 138: 2-Chloro-N-{1-[3-(difluoromethoxy)phenyl]-1H-indazol-4-yl}-5-{[(3,3-dimethylbutanoyl)amino]methyl)benzamide

The title compound was obtained by using the reaction and treatment as described in Example 18.
MS (ESI+) m/z 541 (M+H)⁺

### Example 139: 2-Chloro-5-{[(cyclopentylcarbonyl)amino]methyl)-N-{1-[3-(difluoromethoxy)phenyl]-1H-indazol-4-yl}benzamide

The title compound was obtained by using the reaction and treatment as described in Example 18.
MS (ESI+) m/z 539 (M+H)⁺

### Example 140: 2-Chloro-N-{1-[3-(difluoromethoxyl)phenyl]-1H-indazol-4-yl}-5-[({[1-(trifluoromethyl)cyclopropyl]carbonyl)amino)methyl]benzamide

The title compound was obtained by using the reaction and treatment as described in Example 18.
MS (ESI+) m/z 579 (M+H)⁺

### Example 141: 2-Chloro-N-{1-[3-(difluoromethoxy)phenyl]-1H-indazol-4-yl}-5-({[(1-hydroxycyclopropyl)carbonyl]amino}methyl)benzamide

The title compound was obtained by using the reaction and treatment as described in Example 18.
MS (ESI+) m/z 527 (M+H)⁺

### Example 142: 2-Chloro-N-{1-[3-(difluoromethoxy)phenyl]-1H-indazol-4-yl)-5-{[(methoxyacetyl)amino]methyl}benzamide

The title compound was obtained by using the reaction and treatment as described in Example 18.
MS (ESI+) m/z 515 (M+H)⁺

### Example 143: 2-Chloro-N-{1-[3-(difluoromethoxy)phenyl]-1H-indazol-4-yl}-5-{[(3-hydroxy-2,2-dimethylpropanoyl)amino]methyl} benzamide

The title compound was obtained by using the reaction and treatment as described in Example 18.
MS (ESI+) m/z 543 (M+H)⁺

### Example 144: 2-Chloro-5-{[(cyclopropylsulfonyl)amino]methyl)-N-{1-[3-(difluoromethoxy)phenyl]-1H-indazol-4-yl}benzamide

The title compound was obtained by using the reaction and treatment as described in Example 18.
MS (ESI+) m/z 547 (M+H)⁺

### Example 145: 2-Chloro-N-[1-(4-chlorophenyl)-1H-indazol-4-yl]-5-([(3,3-dimethylbutanoyl)amino 1methyl)benzamide

The title compound was obtained by using the reaction and treatment as described in Example 18.
MS (ESI+) m/z 509 (M+H)⁺

### Example 146: 2-Chloro-N-[1-(4-chlorophenyl)-1H-indazol-4-yl]-5-([(cyclopentylcarbonyl)amino]methyl)benzamide

The title compound was obtained by using the reaction and treatment as described in Example 18.
MS (ESI+) m/z 507 (M+H)⁺

### Example 147: 2-Chloro-N-[1-(4-chlorophenyl)-1H-indazol-4-yl]-5-[({[1-(trifluoromethyl)cyclopropyl]carbonyl]amino)methyl]benzamide

The title compound was obtained by using the reaction and treatment as described in Example 18.
MS (ESI+) m/z 547 (M+H)⁺

### Example 148: 2-Chloro-N-[1-(4-chlorophenyl)-1H-indazol-4-yl]-5-(r(methoxyacetyl)aminolmethyl)benzamide

The title compound was obtained by using the reaction and treatment as described in Example 18.
MS (ESI+) m/z 483 (M+H)⁺

### Example 149: 2-Chloro-N-[1-(4-chlorophenyl)-1H-indazol-4-yl]-5-{[(3-hydroxy-2,2-dimethylpropanoyl)amino]methyl}benzamide

The title compound was obtained by using the reaction and treatment as described in Example 18.
MS (ESI+) m/z 511 (M+H)⁺

### Example 150: 2-Chloro-N-[1-(4-chlorophenyl)-1H-indazol-4-yl]-5-([(cyclopropylsulfonyl)amino]methyl)benzamide

The title compound was obtained by using the reaction and treatment as described in Example 18.
MS (ESI+) m/z 515 (M+H)⁺

### Example 151: 2-Chloro-5-{[(3,3-dimethylbutanoyl)amino]methyl}-N-[1-(4-fluorophenyl)-1H-indazol-4-yl]benzamide

The title compound was obtained by using the reaction and treatment as described in Example 18.
MS (ESI+) m/z 493 (M+H)⁺

### Example 152: 2-Chloro-5-{[(cyclopentylcarbonyl)aminolmethyl]-N-[1-(4-fluorophenyl)-1H-indazol-4-yl]benzamide

The title compound was obtained by using the reaction and treatment as described in Example 18.
MS (ESI+) m/z 491 (M+H)⁺

### Example 153: 2-Chloro-N-[1-(4-fluorophenyl)-1H-indazol-4-yl]-5-[({[1-(trifluoromethyl)cyclopropyl]carbonyl}amino)methyl]benzamide

The title compound was obtained by using the reaction and treatment as described in Example 18.
MS (ESI+) m/z 531 (M+H)⁺

### Example 154: 2-Chloro-N-[1-(4-fluorophenyl)-1H-indazol-4-yl]-5-({[(1-hydroxycyclopropyl)carbonyllaminolmethyl)benzamide

The title compound was obtained by using the reaction and treatment as described in Example 18.
MS (ESI+) m/z 479 (M+H)⁺

### Example 155: 2-Chloro-N-[1-(4-fluorophenyl)-1H-indazol-4-yl]-5-([(methoxyacetyl)amino]methyl)benzamide

The title compound was obtained by using the reaction and treatment as described in Example 18.
MS (ESI+) m/z 467 (M+H)⁺

### Example 156: 2-Chloro-N-[1-(4-fluorophenyl)-1H-indazol-4-yl]-5-([(3-hydroxy-2,2-dimethylpropanoyl)aminolmethyl)benzamide

The title compound was obtained by using the reaction and treatment as described in Example 18.
MS (ESI+) m/z 495 (M+H)⁺

### Example 157: 2-Chloro-5-{[(cyclopropylsulfonyl)amino]methyl}-N-[1-(4-fluorophenyl)-1H-indazol-4-yl]benzamide

The title compound was obtained by using the reaction and treatment as described in Example 18.
MS (ESI+) m/z 499 (M+H)⁺

### Example 158: 2,6-Dichloro-3-{[(2,2-dimethylpropanoyl)amino]methyl}-N-[1-(4-fluorophenyl)-1H-indazol-4-yl]benzamide

The title compound was obtained by using the reaction and treatment as described in Example 18.
MS (ESI+) m/z 513 (M+H)⁺

### Example 159: 2-Chloro-5-{[(3,3-dimethylbutanoyl)amino]methyl}-N-[1-(2-methoxypyridin-4-yl)-1H-indazol-4-yl]benzamide

The title compound was obtained by using the reaction and treatment as described in Example 18.
MS (ESI+) m/z 506 (M+H)⁺

### Example 160: 2-Chloro-5-{[(cyclopentylcarbonyl)amino]methyl}-N-[1-(2-methoxypyridin-4-yl)-1H-indazol-4-yl]benzamide

The title compound was obtained by using the reaction and treatment as described in Example 18.
MS (ESI+) m/z 504 (M+H)⁺

### Example 161: 2-Chloro-N-[1-(2-methoxypyridin-4-yl)-1H-indazol-4-yl]-5-[({[1-(trifluoromethyl)cyclopropyl]carbonyl]amino)methyl]benzamide

The title compound was obtained by using the reaction and treatment as described in Example 18.
MS (ESI+) m/z 544 (M+H)⁺

### Example 162: 2-Chloro-5-({[(1-hydroxycyclopropyl)carbonyl]amino]methyl)-N-[1-(2-methoxypyridin-4-yl)-1H-indazol-4-yllbenzamide

The title compound was obtained by using the reaction and treatment as described in Example 18.
MS (ESI+) m/z 492 (M+H)⁺

### Example 163: 2-Chloro-5-{[(methoxyacetyl)amino]methyl}-N-[1-(2-methoxypyridin-4-yl)-1H-indazol-4-yl]benzamide

The title compound was obtained by using the reaction and treatment as described in Example 18.
MS (ESI+) m/z 480 (M+H)⁺

### Example 164: 2-Chloro-5-{[(3-hydroxy-2,2-dimethylnropanoyl)amino]methyl}-N-[1-(2-methoxypyridin-4-yl)-1H-indazol-4-yl]benzamide

The title compound was obtained by using the reaction and treatment as described in Example 18.
MS (ESI+) m/z 508 (M+H)⁺

### Example 165: 2-Chloro-5-([(cyclopropylsulfonyl)amino]methyl}-N-[1-(2-methoxypyridin-4-yl)-1H-indazol-4-yl]benzamide

The title compound was obtained by using the reaction and treatment as described in Example 18.
MS (ESI+) m/z 512 (M+H)⁺

### Example 166: 2-Chloro-5-([(3,3-dimethylbutanoyl)amino]methyl)-N-(1-[5-(trifluoromethyl)pyridin-3-yl]-1H-indazol-4-yl}benzamide

The title compound was obtained by using the reaction and treatment as described in Example 18.
MS (ESI+) m/z 544 (M+H)⁺

### Example 167: 2-Chloro-5-{[(cyclopentylcarbonyl)amino]methyl)-N-{1-[5-(trifluoromethyl)pyridin-3-yl]-1H-indazol-4-yl}benzamide

The title compound was obtained by using the reaction and treatment as described in Example 18.
MS (ESI+) m/z 542 (M+H)⁺

### Example 168: 2-Chloro-5-[({ri-(trifluoromethyl)cyclopropyl]carbonyl]amino)methyl]-N-{1-[5-(trifluoromethyl)pyridin-3-yl]-1H-indazol-4-yl]benzamide

The title compound was obtained by using the reaction and treatment as described in Example 18.
MS (ESI+) m/z 582 (M+H)⁺

### Example 169: 2-Chloro-5-({[(1-hydroxycyclopropyl)carbony]amino}methyl)-N-{1-[5-(trifluoromethyl)pyridin-3-yl]-1H-indazol-4-yl}benzamide

The title compound was obtained by using the reaction and treatment as described in Example 18.
MS (ESI+) m/z 530 (M+H)⁺

### Example 170: 2-Chloro-5-{[(methoxyacetyl)amino]methyl)-N-{1-[5-(trifluoromethyl)pyridin-3-yl]-1H-indazol-4-yl}benzamide

The title compound was obtained by using the reaction and treatment as described in Example 18.
MS (ESI+) m/z 518 (M+H)⁺

### Example 171: 2-Chloro-5-{[(3-hydroxy-2.2-dimethylpropanoyl)amino]methyl}-N-{1-[5-(trifluoromethyl)pyridin-3-yl]-1H-indazol-4-yl]benzamide

The title compound was obtained by using the reaction and treatment as described in Example 18.
MS (ESI+) m/z 546 (M+H)⁺

### Example 172: 2-Chloro-5-{[(cyclopropylsulfonyl)amino]methyl)-N-{1-[5-(trifluoromethyl)pyridin-3-yl]-1H-indazol-4-yl}benzamide

The title compound was obtained by using the reaction and treatment as described in Example 18.
MS (ESI+) m/z 550 (M+H)⁺

### Example 173: 2-Chloro-5-{[(3,3-dimethylbutanoyl)amino]methyl}-N-{1-[6-(trifluoromethyl)pyridin-3-yl]-1H-indazol-4-yl}benzamide

The title compound was obtained by using the reaction and treatment as described in Example 18.
MS (ESI+) m/z 544 (M+H)⁺

### Example 174: 2-Chloro-5-{[(cyclopentylcarbonyl)amino]methyl}-N-{1-[6-(trifluoromethyl)pyridin-3-yl]-1H-indazol-4-yl}benzamide

The title compound was obtained by using the reaction and treatment as described in Example 18.
MS (ESI+) m/z 542 (M+H)⁺

### Example 175: 2-Chloro-5-[({[1-(trifluoromethyl)cyclopropyl]carbonyl]amino)methyl]-N-{1-[6-(trifluoromethyl)pyridin-3-yl]-1H-indazol-4-yl]benzamide

The title compound was obtained by using the reaction and treatment as described in Example 18.
MS (ESI+) m/z 582 (M+H)⁺

### Example 176: 2-Chloro-5-({[(1-hydroxycyclopropyl)carbonyl]amino}methyl)-N-{1-[6-(trifluoromethyl)pyridin-3-yl]-1H-indazol-4-yl}benzamide

The title compound was obtained by using the reaction and treatment as described in Example 18.
MS (ESI+) m/z 530 (M+H)⁺

### Example 177: 2-Chloro-5-{[(methoxyacetyl)aminolmethyl]-N-{1-[6-(trifluoromethyl)pyridin-3-yl]-1H-indazol-4-yl}benzamide

The title compound was obtained by using the reaction and treatment as described in Example 18.
MS (ESI+) m/z 518 (M+H)⁺

### Example 178: 2-Chloro-5-{[(3-hydroxy-2,2-dimethylpronanoyl)amino]methyl}-N-{1-[6-(trifluoromethyl)pyridin-3-yl]-1H-indazol-4-yl}benzamide

The title compound was obtained by using the reaction and treatment as described in Example 18.
MS (ESI+) m/z 546 (M+H)⁺

### Example 179: 2-Chloro-5-(r(cyclopropylsulfonyl)amino]methyl)-N-{1-[6-(trifluoromethyl)pyridin-3-yl]-1H-indazol-4-yl}benzamide

The title compound was obtained by using the reaction and treatment as described in Example 18.
MS (ESI+) m/z 550 (M+H)⁺

### Example 180: 2-Chloro-5-{(cyclopentylcarbonyl)amino]methyl}-N-[1-(propan-2-yl)-1H-indazol-4-yl]benzamide

The title compound was obtained by using the reaction and treatment as described in Example 89.
MS (ESI+) m/z 439 (M+H)⁺

### Example 181: 2-Chloro-N-[1-(propan-2-yl)-1H-indazol-4-yl]-5-[({[1-(trifluoromethyl)cyclopropyl]carbonyl]amino)methyl]benzamide

The title compound was obtained by using the reaction and treatment as described in Example 89.
MS (ESI+) m/z 479 (M+H)⁺

### Example 182: 2-Chloro-5-{[(methoxyacetyl)amino]methyl}-N-[1-(propan-2-yl)-1H-indazol-4-yllbenzamide

The title compound was obtained by using the reaction and treatment as described in Example 89.
MS (ESI+) m/z 415 (M+H)⁺

### Example 183: 2-Chloro-5-{[(3-hydroxy-2,2-dimethylpropanoyl)amino]methyl}-N-[1-(propan-2-yl)-1H-indazol-4-yl]benzamide

The title compound was obtained by using the reaction and treatment as described in Example 89.
MS (ESI+) m/z 443 (M+H)⁺

### Example 184: 2-Chloro-5-{[(cyclopropylsulfonyl)amino]methyl}-N-[1-(propan-2-yl)-1H-indazol-4-yl]benzamide

The title compound was obtained by using the reaction and treatment as described in Example 89.
MS (ESI+) m/z 447 (M+H)⁺

### Example 185: 2-Chloro-5-({[(1-hydroxycyclopropyl)carbonyl]amino}methyl)-N-[1-(propan-2-yl)-1H-indazol-4-yl]benzamide

The title compound was obtained by using the reaction and treatment as described in Example 89.
MS (ESI+) m/z 427 (M+H)⁺

### Example 186: 2-Chloro-5-{[(3,3-dimethylbutanoyl)amino]methyl}-N-[1-(propan-2-yl)-1H-indazol-4-yl]benzamide

The title compound was obtained by using the reaction and treatment as described in Example 89.
MS (ESI+) m/z 441 (M+H)⁺

### Example 187: 2-Chloro-5-{[(3,3-dimethylbutanoyl)amino]methyl}-N-[1-(1,3-thiazol-4-yl)-1H-indazol-4-yl]benzamide

The title compound was obtained by using the reaction and treatment as described in Example 18.
MS (ESI+) m/z 482 (M+H)⁺

### Example 188: 2-Chloro-5-{[(cyclopentylcarbonyl)amino]methyl}-N-[1-(1,3-thiazol-4-yl)-1H-indazol-4-yl]benzamide

The title compound was obtained by using the reaction and treatment as described in Example 18.
MS (ESI+) m/z 480 (M+H)⁺

### Example 189: 2-Chloro-N-[1-(1,3-thiazol-4-yl)-1H-indazol-4-yl]-5-[({[1-(trifluoromethyl)cyclopropyl]carbonyl]amino)methyl]benzamide

The title compound was obtained by using the reaction and treatment as described in Example 18.
MS (ESI+) m/z 520 (M+H)⁺

### Example 190: 2-Chloro-5-({[(1-hydroxycyclopropyl)carbonyl]amino}methyl)-N-[1-(1,3-thiazol-4-yl)-1H-indazol-4-yl]benzamide

The title compound was obtained by using the reaction and treatment as described in Example 18.
MS (ESI+) m/z 468 (M+H)⁺

### Example 191: 2-Chloro-5-{[(methoxyacetyl)amino]methyl}-N-[1-(1,3-thiazol-4-yl)-1H-indazol-4-yllbenzamide

The title compound was obtained by using the reaction and treatment as described in Example 18.
MS (ESI+) m/z 456 (M+H)⁺

### Example 192: 2-Chloro-5-{[(3-hydroxy-2,2-dimethylpropanoyl)amino]methyl}-N-[1-(1,3-thiazol-4-yl]-1H-indazol-4-yl]benzamide

The title compound was obtained by using the reaction and treatment as described in Example 18.
MS (ESI+) m/z 484 (M+H)⁺

### Example 193: 2-Chloro-5-{[(cyclopropylsulfonyl)amino]methyl}-N-[1-(1,3-thiazol-4-yl)-1H-indazol-4-yl]benzamide

The title compound was obtained by using the reaction and treatment as described in Example 18.
MS (ESI+) m/z 488 (M+H)⁺

### Example 194: 5-{[(2,2-Dimethylpropanoyl)amino]methyl]-N-{1-[4-(trifluoromethoxy)phenyl]-1H-indazol-4-yl)-2-(trifluoromethyl)benzamide

The title compound was obtained by using the reaction and treatment as described in Example 18.
MS (ESI+) m/z 579 (M+H)⁺

### Example 195: 5-{[(2,2-Dimethylpropanoyl)amino]methyl}-N-{1-[3-(trifluoromethoxy)phenyl]-1H-indazol-4-yl}-2-(trifluoromethyl)benzamide

The title compound was obtained by using the reaction and treatment as described in Example 18.
MS (ESI+) m/z 579 (M+H)⁺

### Example 196: 5-{[(2,2-Dimethylpropanoyl)amino]methyl}-2-(trifluoromethyl)-N-{11-[6-(trifluoromethyl)pyridin-3-yl1-1H-indazol-4-yl}benzamide

The title compound was obtained by using the reaction and treatment as described in Example 18.
MS (ESI+) m/z 564 (M+H)⁺

### Example 197: 5-{[(2,2-Dimethylpropanoyl)amino]methyl}-N-[1-(2-methoxypyridin-4-yl)-1H-indazol-4-yl]-2-(trifluoromethyl)benzamide

The title compound was obtained by using the reaction and treatment as described in Example 18.
MS (ESI+) m/z 526 (M+H)⁺

### Example 198: N-[1-(4-chlorophenyl)-1H-indazol-4-yl]-5-{[(2,2-dimethylpropanoyl)amino]methyl}-2-(trifluoromethyl)benzamide

The title compound was obtained by using the reaction and treatment as described in Example 18.
MS (ESI+) m/z 529 (M+H)⁺

### Example 199: 5-{[(2,2-Dimethylpropanoyl)amino]methyl}-N-{1-[3-methyl-4-(trifluoromethoxy)phenyl]-1H-indazol-4-yl)-2-(trifluoromethyl)benzamide

The title compound was obtained by using the reaction and treatment as described in Example 18.
MS (ESI+) m/z 593 (M+H)⁺

### Example 200: N-[1-(3-chloro-4-methylphenyl)-1H-indazol-4-yl]-5-{[(2,2-dimethylpropanoyl)amino]methyl}-2-(trifluoromethyl)benzamide

The title compound was obtained by using the reaction and treatment as described in Example 18.
MS (ESI+) m/z 543 (M+H)⁺

### Example 201: 5-{[(2,2-Dimethylpropanoyl)amino]methyl}-N-[1-(1,3-thiazol-4-yl)-1H-indazol-4-yl]-2-(trifluoromethyl)benzamide

The title compound was obtained by using the reaction and treatment as described in Example 18.
MS (ESI+) m/z 502 (M+H)⁺

### Example 202: 5-{[(2,2-Dimethylpropanoyl)amino]methyl}-N-[1-(propan-2-yl)-1H-indazol-4-yl]-2-(trifluoromethyl)benzamide

The title compound was obtained by using the reaction and treatment as described in Example 89.
MS (ESI+) m/z 461 (M+H)⁺

### Example 203: 2-Chloro-5-{[(3,3-dimethylbutanoyl)amino]methyl}-N-(1-methyl-1H-indazol-4-yl)benzamide

The title compound was obtained by using the reaction and treatment as described in Example 89.
MS (ESI+) m/z 413 (M+H)⁺

### Example 204: 2-Chloro-5-{[(cyclopentylcarbonyl)amino]methyl}-N-(1-methyl-1H-indazol-4-yl)benzamide

The title compound was obtained by using the reaction and treatment as described in Example 89.
MS (ESI+) m/z 411 (M+H)⁺

### Example 205: 2-Chloro-N-(1-methyl-1H-indazol-4-yl)-5-[({[1-(trifluoromethyl)cyclopropyl]carbonyl}amino)methyl]benzamide

The title compound was obtained by using the reaction and treatment as described in Example 89.
MS (ESI+) m/z 451 (M+H)⁺

### Example 206: 2-Chloro-5-({[(1-hydroxycyclopropyl)carbonyl]amino}methyl)-N-(1-methyl-1H-indazol-4-yl)benzamide

The title compound was obtained by using the reaction and treatment as described in Example 89.
MS (ESI+) m/z 399 (M+H)⁺

### Example 207: 2-Chloro-5-{[(methoxyacetyl)amino]methyl}-N-(1-methyl-1H-indazol-4-yl)benzamide

The title compound was obtained by using the reaction and treatment as described in Example 89.
MS (ESI+) m/z 387 (M+H)⁺

### Example 208: 2-Chloro-5-{[(cyclopropylsulfonyl)amino]methyl)-N-(1-methyl-1H-indazol-4-yl)benzamide

The title compound was obtained by using the reaction and treatment as described in Example 89.
MS (ESI+) m/z 419 (M+H)⁺

### Example 209: 5-{[(2,2-Dimethylpropanoyl)amino]methyl}-N-(1-methy-1H-indazol-4-yl)-2-(trifluoromethyl)benzamide

The title compound was obtained by using the reaction and treatment as described in Example 89.
MS (ESI+) m/z 433 (M+H)⁺

### Example 210: 2-Chloro-5-{[(3,3-dimethylbutanoyl)amino]methyl}-N-(1-ethyl-1H-indazol-4-yl)benzamide

The title compound was obtained by using the reaction and treatment as described in Example 89.
MS (ESI+) m/z 427 (M+H)⁺

### Example211: 2-Chloro-5-{[(cyclopentylcarbonyl)amino]methyl}-N-(1-ethyl-1H-indazol-4-yl)benzamide

The title compound was obtained by using the reaction and treatment as described in Example 89.
MS (ESI+) m/z 425 (M+H)⁺

### Example 212: 2-Chloro-N-(1-ethyl-1H-indazol-4-yl)-5-[({[1-(trifluoromethyl)cyclopropyllcarbonyllamino)methyllbenzamide

The title compound was obtained by using the reaction and treatment as described in Example 89.
MS (ESI+) m/z 465 (M+H)⁺

### Example 213: 2-Chloro-N-(1-ethyl-1H-indazol-4-yl)-5-({[(1-hydroxycyclopropyl)carbonyll amino I methyl)benzamide

The title compound was obtained by using the reaction and treatment as described in Example 89.
MS (ESI+) m/z 413 (M+H)⁺

### Example 214: 2-Chloro-N-(1-ethyl-1H-indazol-4-yl)-5-([(methoxyacetyl)amino]methyl)benzamide

The title compound was obtained by using the reaction and treatment as described in Example 89.
MS (ESI+) m/z 401 (M+H)⁺

### Example 215: 2-Chloro-N-(1-ethyl-1H-indazol-4-yl)-5-{[(3-hydroxy-2,2-dimethylpropanoyl)amino]methyl}benzamide

The title compound was obtained by using the reaction and treatment as described in Example 89.
MS (ESI+) m/z 429 (M+H)⁺

### Example 216: 2-Chloro-5-{[(cyclopropylsulfonyl)amino]methyl}-N-(1-ethyl-1H-indazol-4-yl)benzamide

The title compound was obtained by using the reaction and treatment as described in Example 89.
MS (ESI+) m/z 433 (M+H)⁺

### Example 217: 5-{[(2,2-Dimethylpropanoyl)amino]methyl}-N-(1-ethyl-1H-indazol-4-yl)-2-(trifluoromethyl)benzamide

The title compound was obtained by using the reaction and treatment as described in Example 89.
MS (ESI+) m/z 447 (M+H)⁺

### Example 218: 2-Chloro-5-{[(3,3-dimethylbutanoyl)amino]methyl)-N-{1-[3-(trifluoromethyl)phenyl]-1H-indazol-4-yl}benzamide

The title compound was obtained by using the reaction and treatment as described in Example 18.
MS (ESI+) m/z 543 (M+H)⁺

### Example 219: 2-Chloro-5-{[(cyclopentylcarbonyl)amino]methyl}-N-(1-[3-(trifluoromethyl)phenyl]-1H-indazol-4-yl}benzamide

The title compound was obtained by using the reaction and treatment as described in Example 18.
MS (ESI+) m/z 541 (M+H)⁺

### Example 220: 2-Chloro-5-[({[1-(trifluoromethyl)cyclopropyl]carbonyl)amino)methyl]-N-{1-[3-(trifluoromethyl)phenyl]-1H-indazol-4-yl}benzamide

The title compound was obtained by using the reaction and treatment as described in Example 18.
MS (ESI+) m/z 581 (M+H)⁺

### Example 221: 2-Chloro-5-({[(1-hydroxycyclopropyl)carbonyl]amino}methyl)-N-{1-[3-(trifluoromethyl)phenyl]-1H-indazol-4-yl}benzamide

The title compound was obtained by using the reaction and treatment as described in Example 18.
MS (ESI+) m/z 529 (M+H)⁺

### Example 222: 2-Chloro-5-{[(methoxyacetyl)amino]methyl)-N-{1-[3-(trifluoromethyl)phenyl]-1H-indazol-4-yl}benzamide

The title compound was obtained by using the reaction and treatment as described in Example 18.
MS (ESI+) m/z 517 (M+H)⁺

### Example 223: 2-Chloro-5-{[(3-hydroxy-2,2-dimethylpropanoyl)amino]methyl}-N-{1-[3-(trifluoromethyl)phenyl]-1H-indazol-4-yl}benzamide

The title compound was obtained by using the reaction and treatment as described in Example 18.
MS (ESI+) m/z 545 (M+H)⁺

### Example 224: 2-Chloro-5-{[(cyclopropylsulfonyl)amino]methyl}-N-{1-[3-(trifluoromethyl)phenyl]-1H-indazol-4-yl}benzamide

The title compound was obtained by using the reaction and treatment as described in Example 18.
MS (ESI+) m/z 549 (M+H)⁺

### Example 225: 5-{[(2,2-Dimethylpropanoyl)amino]methyl}-2-(trifluoromethyl)-N-{1-[3-(trifluoromethyl)phenyl]-1H-indazol-4-yl}benzamide

The title compound was obtained by using the reaction and treatment as described in Example 18.
MS (ESI+) m/z 563 (M+H)⁺

### Example 226: 2-Chloro-5-{[(3,3-dimethylbutanoyl)amino]methyl}-N-[1-(1,3-thiazol-2-yl)-1H-indazol-4-yl]benzamide

The title compound was obtained by using the reaction and treatment as described in Example 18.
MS (ESI+) m/z 482 (M+H)⁺

### Example 227: 2-Chloro-5-{[(cyclopentylcarbonyl)amino]methyl}-N-[1-(1,3-thiazol-2-yl)-1H-indazol-4-yl]benzamide

The title compound was obtained by using the reaction and treatment as described in Example 18.
MS (ESI+) m/z 480 (M+H)⁺

### Example 228: 2-Chloro-N-[1-(1,3-thiazol-2-yl)-1H-indazol-4-yl]-5-[({[1-(trifluoromethyl)cyclopropyl]carbonyl]amino)methyl]benzamide

The title compound was obtained by using the reaction and treatment as described in Example 18.
MS (ESI+) m/z 520 (M+H)⁺

### Example 229: 2-Chloro-5-({[(1-hydroxycyclopropyl)carbonyl]amino}methyl)-N-[1-(1,3-thiazol-2-yl)-1H-indazol-4-yl]benzamide

The title compound was obtained by using the reaction and treatment as described in Example 18.
MS (ESI+) m/z 468 (M+H)⁺

### Example 230: 2-Chloro-5-([(methoxyacetyl)amino]methyl)-N-[1-(1,3-thiazol-2-yl)-1H-indazol-4-yl]benzamide

The title compound was obtained by using the reaction and treatment as described in Example 18.
MS (ESI+) m/z 456 (M+H)⁺

### Example 231: 2-Chloro-5-{[(3-hydroxy-2,2-dimethylpropanoyl)amino]methyl}-N-[1-(1,3-thiazol-2-yl)-1H-indazol-4-yl]benzamide

The title compound was obtained by using the reaction and treatment as described in Example 18.
MS (ESI+) m/z 484 (M+H)⁺

### Example 232: 2-Chloro-5-{[(cyclopropylsulfonyl)amino]methyl}-N-[1-(1,3-thiazol-2-yl)-1H-indazol-4-yl]benzamide

The title compound was obtained by using the reaction and treatment as described in Example 18.
MS (ESI+) m/z 488 (M+H)⁺

### Example 233: 5-{[(2,2-Dimethylpropanoyl)amino]methyl)-N-[1-(1,3-thiazol-2-yl)-1H-indazol-4-yll-2-(trifluoromethyl)benzamide

The title compound was obtained by using the reaction and treatment as described in Example 18.
MS (ESI+) m/z 502 (M+H)⁺

### Example 234: 2-Chloro-5-{[(3,3-dimethylbutanoyl)aminolmethyl}-N-[1-(2-methylpyridin-4-yl)-1H-indazol-4-yl]benzamide

The title compound was obtained by using the reaction and treatment as described in Example 18.
MS (ESI+) m/z 490 (M+H)⁺

### Example 235: 2-Chloro-5-{[(cyclopentylcarbonyl)amino]methyl}-N-[1-(2-methylpyridin-4-yl)-1H-indazol-4-yl]benzamide

The title compound was obtained by using the reaction and treatment as described in Example 18.
MS (ESI+) m/z 488 (M+H)⁺

### Example 236: 2-Chloro-5-{[(methoxyacetyl)amino]methyl}-N-[1-(2-methylpyridin-4-yl)-1H-indazol-4-yl]benzamide

The title compound was obtained by using the reaction and treatment as described in Example 18.
MS (ESI+) m/z 464 (M+H)⁺

### Example 237: 2-Chloro-5-{[(cyclopropylsulfonyl)amino]methyl}-N-[1-(2-methylpyridin-4-yl)-1H-indazol-4-yl]benzamide

The title compound was obtained by using the reaction and treatment as described in Example 18.
MS (ESI+) m/z 496 (M+H)⁺

### Example 238: 5-{[(2,2-dimethylpropanoyl)amino]methyl)-N-[1-(2-methylpyridin-4-yl)-1H-indazol-4-yl]-2-(trifluoromethyl)benzamide

The title compound was obtained by using the reaction and treatment as described in Example 18.
MS (ESI+) m/z 510 (M+H)⁺

### Example 239: 2-Chloro-5-{[(3,3-dimethylbutanoyl)amino]methyl}-N-[1-(5-methylpyridin-3-yl)-1H-indazol-4-yl]benzamide

The title compound was obtained by using the reaction and treatment as described in Example 18.
MS (ESI+) m/z 490 (M+H)⁺

### Example 240: 2-Chloro-5-{[(cyclopentylcarbonyl)amino]methyl}-N-[1-(5-methylpyridin-3-yl)-1H-indazol-4-yl]benzamide

The title compound was obtained by using the reaction and treatment as described in Example 18.
MS (ESI+) m/z 488 (M+H)⁺

### Example 241: 2-Chloro-N-[1-(5-methylpyridin-3-yl)-1H-indazol-4-yl]-5-[({[1-(trifluoromethyl)cyclopropyl]carbonyl}amino)methyl]benzamide

The title compound was obtained by using the reaction and treatment as described in Example 18.
MS (ESI+) m/z 528 (M+H)⁺

### Example 242: 2-Chloro-5-({[(1-hydroxycyclopropyl)carbonyl]amino}methyl)-N-[1-(5-methylpyridin-3-yl)-1H-indazol-4-yl]benzamide

The title compound was obtained by using the reaction and treatment as described in Example 18.
MS (ESI+) m/z 476 (M+H)⁺

### Example 243: 2-Chloro-5-{[(methoxyacetyl)amino]methyl}-N-[1-(5-methylpyridin-3-yl)-1H-indazol-4-yl]benzamide

The title compound was obtained by using the reaction and treatment as described in Example 18.
MS (ESI+) m/z 464 (M+H)⁺

### Example 244: 2-Chloro-5-{[(3-hydroxy-2,2-dimethylpropanoyl)amino]methyl}-N-[1-(5-methylpyridin-3-yl)-1H-indazol-4-yl]benzamide

The title compound was obtained by using the reaction and treatment as described in Example 18.
MS (ESI+) m/z 492 (M+H)⁺

### Example 245: 2-Chloro-5-{[(cyclopropylsulfonyl)amino]methyl}-N-[1-(5-methylpyridin-3-yl)-1H-indazol-4-yl]benzamide

The title compound was obtained by using the reaction and treatment as described in Example 18.
MS (ESI+) m/z 496 (M+H)⁺

### Example 246: 5-{[(2,2-dimethylpropanoyl)amino]methyl}-N-[1-(5-methylpyridin-3-yl)-1H-indazol-4-yll-2-(trifluoromethyl)benzamide

The title compound was obtained by using the reaction and treatment as described in Example 18.
MS (ESI+) m/z 510 (M+H)⁺

### Example 247: 2-Chloro-5-{[(3,3-dimethylbutanoyl)amino]methyl}-N-[1-(6-methylpyridazin-3-yl)-1H-indazol-4-yl]benzamide

The title compound was obtained by using the reaction and treatment as described in Example 18.
MS (ESI+) m/z 491 (M+H)⁺

### Example 248: 2-Chloro-5-{[(cyclopentylcarbonyl)amino]methyl}{-N-[1-(6-methylpyridazin-3-yl)-1H-indazol-4-yl]benzamide

The title compound was obtained by using the reaction and treatment as described in Example 18.
MS (ESI+) m/z 489 (M+H)⁺

### Example 249: 2-Chloro-N-[1-(6-methylpyridazin-3-yl)-1H-indazol-4-yl]-5-[({[1-(trifluoromethyl)cyclopropyl]carbonyl]amino)methyl]benzamide

The title compound was obtained by using the reaction and treatment as described in Example 18.
MS (ESI+) m/z 489 (M+H)⁺

### Example 250: 2-Chloro-5-({[(1-hydroxycyclopropyl)carbonyl]amino}methyl)-N-[1-(6-methylpyridazin-3-yl)-1H-indazol-4-yl]benzamide

The title compound was obtained by using the reaction and treatment as described in Example 18.
MS (ESI+) m/z 477 (M+H)⁺

### Example 251: 2-Chloro-5-{[(methoxyacetyl)amino]methyl}-N-[1-(6-methylpyridazin-3-yl)-1H-indazol-4-yl]benzamide

The title compound was obtained by using the reaction and treatment as described in Example 18.
MS (ESI+) m/z 465 (M+H)⁺

### Example 252: 2-Chloro-5-{[(3-hydroxy-2,2-dimethylpropanoyl)aminolmethyl}-N-[1-(6-methylpyridazin-3-yl)-1H-indazol-4-yl]benzamide

The title compound was obtained by using the reaction and treatment as described in Example 18.
MS (ESI+) m/z 493 (M+H)⁺

### Example 253: 2-Chloro-5-{[(cyclopropylsulfonyl)amino]methyl}-N-[1-(6-methylpyridazin-3-yl)-1H-indazol-4-yl]benzamide

The title compound was obtained by using the reaction and treatment as described in Example 18.
MS (ESI+) m/z 497 (M+H)⁺

### Example 254: 2-Chloro-5-{[(3,3-dimethylbutanoyl)amino]methyl)-N-{1-[4-methyl-3-(trifluoromethyl)phenyl]-1H-indazol-4-yl}benzamide

The title compound was obtained by using the reaction and treatment as described in Example 18.
MS (ESI+) m/z 557 (M+H)⁺

### Example 255: 2-Chloro-5-{[(cyclopentylcarbonyl)amino]methyl)-N-{1-[4-methyl-3-(trifluoromethyl)phenyl]-1H-indazol-4-yl}benzamide

The title compound was obtained by using the reaction and treatment as described in Example 18.
MS (ESI+) m/z 555 (M+H)⁺

### Example 256: 2-Chloro-N-{1-[4-methyl-3-(trifluoromethyl)phenyt]-1H-indazol-4-yl}-5-[({[1-(triftuoromethyl)cyclopropyl]carbonyl}amino)methyl]benzamide

The title compound was obtained by using the reaction and treatment as described in Example 18.
MS (ESI+) m/z 495 (M+H)⁺

### Example 257: 2-Chloro-5-({[(1-hydroxycyclopropyl)carbonyl]amino}methyl)-N-{1-[4-methyl-3-(trifluoromethyl)phenyl]-1H-indazol-4-yl}benzamide

The title compound was obtained by using the reaction and treatment as described in Example 18.
MS (ESI+) m/z 543 (M+H)⁺

### Example 258: 2-Chloro-5-{[(methoxyacetyl)amino]methyl}-N-{1-[4-methyl-3-(trifluoromethyl)phenyl]-1H-indazol-4-yl]benzamide

The title compound was obtained by using the reaction and treatment as described in Example 18.
MS (ESI+) m/z 513 (M+H)⁺

### Example 259: 2-Chloro-5-{[(3-hydroxy-2,2-dimethylpropanoyl)amino]methyl}-N-{1-[4-methyl-3-(trifluoromethyl)phenyl]-1H-indazol-4-yl)benzamide

The title compound was obtained by using the reaction and treatment as described in Example 18.
MS (ESI+) m/z 559 (M+H)⁺

### Example 260: 2-Chloro-5-{[(cyclopropylsulfonyl)amino]methyl}-N-{1-[4-methyl-3-(trifluoromethyl)phenyl]-1H-indazol-4-yl)benzamide

The title compound was obtained by using the reaction and treatment as described in Example 18.
MS (ESI+) m/z 563 (M+H)⁺

### Example 261: 2-Chloro-5-{[(3,3-dimethylbutanoyl)aminol]methyl}-N-{1-[4-methoxy-3-(trifluoromethyl)phenyl]-1H-indazol-4-yl}benzamide

The title compound was obtained by using the reaction and treatment as described in Example 18.
MS (ESI+) m/z 573 (M+H)⁺

### Example 262: 2-Chloro-5-([(cyclopentylcarbonyl)amino]methyl}-N-{1-[4-methoxy-3-(trifluoromethyl)phenyl]-1H-indazol-4-yl)benzamide

The title compound was obtained by using the reaction and treatment as described in Example 18.
MS (ESI+) m/z 571 (M+H)⁺

### Example 263: 2-Chloro-N-{1-[4-methoxy-3-(trifluoromethyl)phenyl]-1H-indazol-4-yl)-5-[({[1-(trifluoromethyl)cyclopropyl]carbonyl}amino)methyl]benzamide

The title compound was obtained by using the reaction and treatment as described in Example 18.
MS (ESI+) m/z 611 (M+H)⁺

### Example 264: 2-Chloro-5-({[(1-hydroxycyclopropyl)carbonyl]aminolmethyl)-N-{1-[4-methoxy-3-(trifluoromethyl)phenyl]-1H-indazol-4-yl}benzamide

The title compound was obtained by using the reaction and treatment as described in Example 18.
MS (ESI+) m/z 559 (M+H)⁺

### Example 265: 2-Chloro-5-{[(methoxyacetyl)amino]methyl}-N-{1-[4-methoxy-3-(trifluoromethyl)phenyl]-1H-indazol-4-yl)benzamide

The title compound was obtained by using the reaction and treatment as described in Example 18.
MS (ESI+) m/z 547 (M+H)⁺

### Example 266: 2-Chloro-5-{[(3-hydroxy-2,2-dimethylpropanoyl)amino]methyl}-N-{1-[4-methoxy-3-(trifluoromethyl)phenyl]-1H-indazol-4-yl}benzamide

The title compound was obtained by using the reaction and treatment as described in Example 18.
MS (ESI+) m/z 575 (M+H)⁺

### Example 267: 2-Chloro-5-{[(cyclopropylsulfonyl)amino]methyl}-N-{1-[4-methoxy-3-(trifluoromethyl)phenyl]-1H-indazol-4-yl}benzamide

The title compound was obtained by using the reaction and treatment as described in Example 18.
MS (ESI+) m/z 579 (M+H)⁺

### Example 268: 5-{[(2,2-Dimethylpropanoyl)amino]methyl}-N-{1-[4-methoxy-3-(trifluoromethyl)phenyl]-1H-indazol-4-yl}-2-(trifluoromethyl)benzamide

The title compound was obtained by using the reaction and treatment as described in Example 18.
MS (ESI+) m/z 593 (M+H)⁺

### Example 269: 2-Chloro-5-{[(3,3-dimethylbutanoyl)amino]methyl}-N-[1-(6-methoxypyridin-3-yl)-1H-indazol-4-yl]benzamide

The title compound was obtained by using the reaction and treatment as described in Example 18.
MS (ESI+) m/z 506 (M+H)⁺

### Example 270: 2-Chloro-5-{[(cyclopentylcarbonyl)amino]methyl)-N-[1-(6-methoxypyridin-3-yl)-1H-indazol-4-yl]benzamide

The title compound was obtained by using the reaction and treatment as described in Example 18.
MS (ESI+) m/z 504 (M+H)⁺

### Example 271: 2-Chloro-N-[1-(6-methoxypyridin-3-yl)-1H-indazol-4-yl]-5-[({[1-(trifluoromethyl)cyclopropyl]carbonyl)amino)methyl]benzamide

The title compound was obtained by using the reaction and treatment as described in Example 18.
MS (ESI+) m/z 544 (M+H)⁺

### Example 272: 2-Chloro-5-({[(1-hydroxycyclopropyl)carbonyl]amino}methyl)-N-[1-(6-methoxypyridin-3-yl)-1H-indazol-4-yl]benzamide

The title compound was obtained by using the reaction and treatment as described in Example 18.
MS (ESI+) m/z 492 (M+H)⁺

### Example 273: 2-Chloro-5-{[(methoxyacetyl)amino]methyl}-N-[1-(6-methoxypyridin-3-yl)-1H-indazol-4-yl]benzamide

The title compound was obtained by using the reaction and treatment as described in Example 18.
MS (ESI+) m/z 480 (M+H)⁺

### Example 274: 2-Chloro-5-{[(3-hydroxy-2,2-dimethylpropanoyl)aminolmethyl}-N-[1-(6-methoxypyridin-3-yl)-1H-indazol-4-yl]benzamide

The title compound was obtained by using the reaction and treatment as described in Example 18.
MS (ESI+) m/z 508 (M+H)⁺

### Example 275: 2-Chloro-5-{[(cyclopropylsulfonyl)amino]methyl)-N-[1-(6-methoxypyridin-3-yl)-1H-indazol-4-yl]benzamide

The title compound was obtained by using the reaction and treatment as described in Example 18.
MS (ESI+) m/z 512 (M+H)⁺

### Example 277: 2-Chloro-5-{[(3,3-dimethylbutanoyl)amino]methyl}-N-[1-(2,6-dimethylpyridin-4-yl)-1H-indazol-4-yl]benzamide

The title compound was obtained by using the reaction and treatment as described in Example 18.
MS (ESI+) m/z 504 (M+H)⁺

### Example 278: 2-Chloro-5-{[(cyclopentylcarbonyl)amino]methyl-N-[1-(2,6-dimethyipyridin-4-yl)-1H-indazol-4-vllbenzamide

The title compound was obtained by using the reaction and treatment as described in Example 18.
MS (ESI+) m/z 502 (M+H)⁺

### Example 279: 2-Chloro-N-[1-(2,6-dimethylpyridin-4-yl)-1H-indazol-4-yl]-5-[({1-(trifluoromethyl)cyclopropyl]carbonyl]amino)methyl]benzamide

The title compound was obtained by using the reaction and treatment as described in Example 18.
MS (ESI+) m/z 542 (M+H)⁺

### Example 280: 2-Chloro-N-[1-(2,6-dimethylpyridin-4-yl)-1H-indazol-4-yl]-5-{[(methoxyacetyl)amino]methyl}benzamide

The title compound was obtained by using the reaction and treatment as described in Example 18.
MS (ESI+) m/z 478 (M+H)⁺

### Example 281: 2-Chloro-N-[1-(2,6-dimethylpyridin-4-yl)-1H-indazol-4-yl]-5-{(3-hydroxy-2,2-dimethylpropanoyl)amino]methyl}benzamide

The title compound was obtained by using the reaction and treatment as described in Example 18.
MS (ESI+) m/z 506 (M+H)⁺

### Example 282: 2-Chloro-5-{[(cyclopropylsulfonyl)amino]methyl}-N-[1-(2,6-dimethylpyridin-4-yl)-1H-indazol-4-yl]benzamide

The title compound was obtained by using the reaction and treatment as described in Example 18.
MS (ESI+) m/z 510 (M+H)⁺

### Example 283: 5-{[(2,2-Dimethylpropanoyl)amino]methyl}-N-[1-(2,6-dimethylpyridin-4-yl)-1H-indazol-4-yll-2-(trifluoromethyl)benzamide

The title compound was obtained by using the reaction and treatment as described in Example 18.
MS (ESI+) m/z 524 (M+H)⁺

### Example 284: 2-Chloro-5-{[(2,2-dimethylpropanoyl)amino]methyl}-N-(1-cyclopropyl-1H-indazol-4-yl)benzamide

The title compound was obtained by using the reaction and treatment as described in Example 89.
MS (ESI+) m/z 425 (M+H)⁺

### Example 285: 5-{[(2,2-Dimethylpropanoyl)amino]methyl}-N-(1-cyclopropyl-1H-indazol-4-yl)-2-(trifluoromethyl)benzamide

The title compound was obtained by using the reaction and treatment as described in Example 89.
MS (ESI+) m/z 459 (M+H)⁺

The compounds listed in the following 1-55 can be prepared according to any method as described in Example 1, Example 48, Example 88, or Example 89.
1. 2,6-Dichloro-3-{[(2,2-dimethylpropanoyl)amino]methyl}-N-{1-[4-(trifluoromethoxy)phenyl]-1H-indazol-4-yl}benzamide
2. 2-Chloro-5-({[(1-hydroxycyclopropyl)carbonyl]amino}methyl)-N-{1-[3-(trifluoromethoxy)phenyl]-1H-indazol-4-yl}benzamide
3. 2,6-Dichloro-3-{[(2,2-dimethylpropanoyl)amino]methyl}-N-{1-[3-(trifluoromethoxy)phenyl]-1 H-indazol-4-yl} benzamide
4. 2-Chloro-N-[1-(3-chloro-4-methylphenyl)-1H-indazol-4-yl]-5-([(3-hydroxy-2,2-dimethylpropanoyl)amino]methyl} benzamide
5. 2,6-Dichloro-N-[1-(3-chloro-4-methylphenyl)-1H-indazol-4-yl]-3-{[(2,2-dimethylpropanoyl)amino]methyl}benzamide
6. 2-Chloro-N-{1-[3-methyl-4-(trifluoromethoxy)phenyl]-1H-indazol-4-yl}-5-[({[1-(trifluoromethyl)cyclopropyl]carbonyl}amino)methyl]benzamide
7. 2-Chloro-5-({[(1-hydroxycyclopropyl)carbonyl]amino}methyl)-N-{1-[3-methyl-4-(trifluoromethoxy)phenyl]-1H-indazol-4-yl}benzamide
8. 2,6-Dichloro-3-{[(2,2-dimethylpropanoyl)amino]methyl}-N-{1-[3-methyl-4-(trifluoromethoxy)phenyl]-1H-indazol-4-yl[benzamide
9. 2,6-Dichloro-N-{ 1-[3-(difluoromethoxy)phenyl]-1H-indazol-4-yl}-3-{[(2,2-dimethylpropanoyl)amino]methyl}benzamide
10. 2-Chloro-N-[1-(4-chlorophenyl)-1H-indazol-4-yl]-5-({[(1-hydroxycyclopropyl)carbonyl]amino}methyl)benzamide
11. 2,6-Dichloro-N-[1-(4-chlorophenyl)-1H-indazol-4-yl]-3-{[(2,2-dimethylpropanoyl)amino]methyl}benzamide
12. 2,6-Dichloro-3-{[(2,2-dimethylpropanoyl)amino]methyl}-N-[1-(2-methoxypyridin-4-yl)-1H-indazol-4-yl]benzamide
13. 2,6-Dichloro-3-{ [(2,2-dimethylpropanoyl)amino]methyl}-N-{ 1-[5-(trifluoromethyl)pyridin-3-yl]-1H-indazol-4-yl}benzamide
14. 2,6-Dichloro-3-{ [(2,2-dimethylpropanoyl)amino]methyl}-N-{ 1-[6-(trifluoromethyl)pyridin-3-yl]-1H-indazol-4-yl}benzamide
15. 2,6-Dichloro-3-{[(2,2-dimethylpropanoyl)amino]methyl}-N-[1-(propan-2-yl)-1H-indazol-4-yl]benzamide
16. 2,6-Dichloro-3-{[(2,2-dimethylpropanoyl)amino]methyl}-N-[1-(1,3-thiazol-4-yl)-1H-indazol-4-yl]benzamide
17. 5-{[(2,2-Dimethylpropanoyl)amino]methyl}-2-(trifluoromethyl)-N-{1-[5-(trifluoromethyl)pyridin-3-yl]-1H-indazol-4-yl}benzamide
18. 5-{[(2,2-Dimethylpropanoyl)amino]methyl}-N-[1-(4-fluorophenyl)-1H-indazol-4-yl]-2-(trifluoromethyl)benzamide
19. N-{1-[3-(difluoromethoxy)phenyl]-1H-indazol-4-yl}-5-{[(2,2-dimethylpropanoyl)amino]methyl}-2-(trifluoromethyl)benzamide
20. 2-Chloro-5-{[(3-hydroxy-2,2-dimethylpropanoyl)amino]methyl}-N-(1-methyl-1H-indazol-4-yl)benzamide
21. 2,6-Dichloro-3-{[(2,2-dimethylpropanoyl)amino]methyl}-N-(1-methyl-1H-indazol-4-yl)benzamide
22. 2,6-Dichloro-3-{[(2,2-dimethylpropanoyl)amino]methyl}-N-(1-ethyl-1H-indazol-4-yl)benzamide
23. 2,6-Dichloro-3-{[(2,2-dimethylpropanoyl)amino]methyl}-N-{1-[3-(trifluoromethyl)phenyl]-1H-indazol-4-yl}benzamide
24. 2-Chloro-5-{[(3,3-dimethylbutanoyl)amino]methyl}-N-{1-[2-methyl-4-(trifluoromethoxy)phenyl]-1H-indazol-4-yl}benzamide
25. 2-Chloro-5-{[(cyclopentylcarbonyl)amino]methyl}-N-11-[2-methyl-4-(trifluoromethoxy)phenyl]-1H-indazol-4-yl}benzamide
26. 2-Chloro-N-{1-[2-methyl-4-(trifluoromethoxy)phenyl]-1H-indazol-4-yl}-5-[({[1-(trifluoromethyl)cyclopropyl]carbonyl}amino)methyl]benzamide
27. 2-Chloro-5-({[(1-hydroxycyclopropyl)carbonyl]amino}methyl)-N-{1-[2-methyl-4-(trifluoromethoxy)phenyl]-1H-indazol-4-yl}benzamide
28. 2-Chloro-5-{[(methoxyacetyl)amino]methyl}-N-{1-[2-methyl-4-(trifluoromethoxy)phenyl]-1H-indazol-4-yl}benzamide
29. 2-Chloro-5-{[(3-hydroxy-2,2-dimethylpropanoyl)amino]methyl}-N-{1-[2-methyl-4-(trifluoromethoxy)phenyl]-1H-indazol-4-yl}benzamide
30. 2-Chloro-5-{[(cyclopropylsulfonyl)amino]methyl}-N-{1-[2-methyl-4-(trifluoromethoxy)phenyl]-1H-indazol-4-yl}benzamide
31. 2,6-Dichloro-3-{[(2,2-dimethylpropanoyl)amino]methyl}-N-{1-[2-methyl-4-(trifluoromethoxy)phenyl]-1H-indazol-4-yl}benzamide
32. 5-{[(2,2-Dimethylpropanoyl)amino]methyl}-N-{1-[2-methyl-4-(trifluoromethoxy)phenyl]-1H-indazol-4-yl)-2-(trifluoromethyl)benzamide
33. 2,6-Dichloro-3-{[(2,2-dimethylpropanoyl)amino]methyl}-N-[1-(1,3-thiazol-2-yl)-1H-indazol-4-yl]benzamide
34. 2-Chloro-N-[1-(2-methylpyridin-4-yl)-1H-indazol-4-yl]-5-[({[1-(trifluoromethyl)cyclopropyl]carbonyl}amino)methyl]benzamide
35. 2-Chloro-5-({[(1-hydroxycyclopropyl)carbonyl]amino}methyl)-N-[1-(2-methylpyridin-4-yl)-1H-indazol-4-yl]benzamide
36. 2-Chloro-5-{[(3-hydroxy-2,2-dimethylpropanoyl)amino]methyl}-N-[1-(2-methylpyridin-4-yl)-1H-indazol-4-yl]benzamide
37. 2,6-Dichloro-3-{[(2,2-dimethylpropanoyl)amino]methyl}-N-[1-(2-methylpyridin-4-yl)-1H-indazol-4-yl]benzamide
38. 2,6-Dichloro-3-{[(2,2-dimethylpropanoyl)amino]methyl}-N-[1-(5-methylpyridin-3-yl)-1H-indazol-4-yl]benzamide
39. 2,6-Dichloro-3-{[(2,2-dimethylpropanoyl)amino]methyl}-N-[1-(6-methylpyridazin-3-yl)-1H-indazol-4-yl]benzamide
40. 5-{[(2,2-Dimethylpropanoyl)amino]methyl}-N-[1-(6-methylpyridazin-3-yl)-1H-indazol-4-yl]-2-(trifluoromethyl)benzamide
41. 2,6-Dichloro-3-{[(2,2-dimethylpropanoyl)amino]methyl}-N-{1-[4-methyl-3-(trifluoromethyl)phenyl]-1H-indazol-4-yl}benzamide
42. 5-{[(2,2-Dimethylpropanoyl)amino]methyl}-N-{1-[4-methyl-3-(trifluoromethyl)phenyl]-1H-indazol-4-yl} -2-(trifluoromethyl)benzamide
43. 2,6-Dichloro-3-{[(2,2-dimethylpropanoyl)amino]methyl}-N-{1-[4-methoxy-3-(trifluoromethyl)phenyl]-1H-indazol-4-yl}benzamide
44. 2,6-Dichloro-3-{[(2,2-dimethylpropanoyl)amino]methyl}-N-[1-(6-methoxypyridin-3-yl)-1H-indazol-4-yl]benzamide
45. 2-Chloro-N-[1-(2,6-dimethylpyridin-4-yl)-1H-indazol-4-yl]-5-({[(1-hydroxycyclopropyl)earbonyl]amino}methyl)benzamide
46. 2,6-Dichloro-3-{[(2,2-dimethylpropanoyl)amino]methyl}-N-[1-(2,6-dimethylpyridin-4-yl)-1H-indazol-4-yl]benzamide
47. 2-Chloro-N-[1-(2-cyclopropylpyridin-4-yl)-1H-indazol-4-yl]-5-{[(3,3-dimethylbutanoyl)amino]methyl}benzamide
48. 2-Chloro-5-{[(cyclopentylcarbonyl)amino]methyl)-N-[1-(2-cyclopropylpyridin-4-yl)-1H-indazol-4-yl]benzamide
49. 2-Chloro-N-[1-(2-cyclopropylpyridin-4-yl)-1H-indazol-4-yl]-5-[({[1-(trifluoromethyl)cyclopropyl]carbonyl}amino)methyl]benzamide
50. 2-Chloro-N-[1-(2-cyclopropylpyridin-4-yl)-1H-indazol-4-yl]-5-({[(1-hydroxycyclopropyl)carbonyl]amino}methyl)benzamide
51. 2-Chloro-N-[1-(2-cyclopropylpyridin-4-yl)-1H-indazol-4-yl]-5-([(methoxyacetyl)amino]methyl)benzamide
52. 2-Chloro-N-[1-(2-cyclopropylpyridin-4-yl)-1H-indazol-4-yl]-5-{[(3-hydroxy-2,2-dimethylpropanoyl)amino]methyl}benzamide
53. 2-Chloro-N-[1-(2-cyclopropylpyridin-4-yl)-1H-indazol-4-yl]-5-{[(cyclopropylsulfonyl)amino]methyl}benzamide
54. 2,6-Dichloro-N-[1-(2-cyclopropylpyridin-4-yl)-1H-indazol-4-yl]-3-{[(2,2-dimethylpropanoyl)amino]methyl}benzamide
55. N-[1-(2-cyclopropylpyridin-4-yl)-1H-indazol-4-yl]-5-{[(2,2-dimethylpropanoyl)amino]methyl}-2-(trifluoromethyl)benzamide

The structural formulas for Examples 1 to 285 are shown in Tables 1 to 24.

**[Table 1]**

| **Example** | **Structure** | **Example** | **Structure** | **Example** | **Structure** |
|---|---|---|---|---|---|
| **1** | 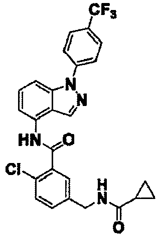 | **2** | 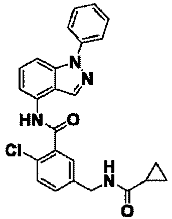 | **3** | 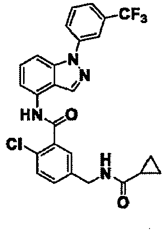 |
| **4** | 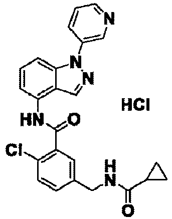 | **5** | 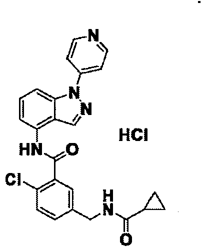 | **6** | 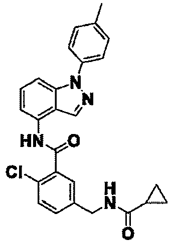 |
| **7** | 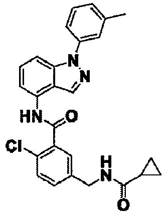 | **8** | 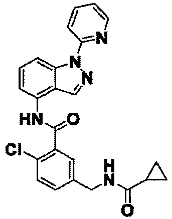 | **9** | 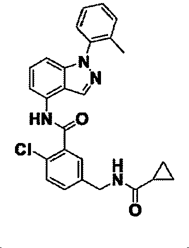 |
| **10** | 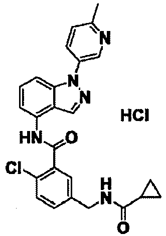 | **11** | 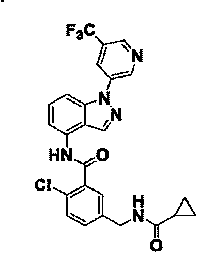 | **12** | 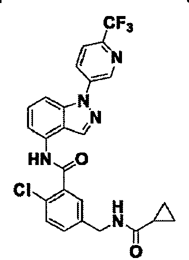 |

**[Table 2]**

| **Example** | **Structure** | **Example** | **Structure** | **Example** | **Structure** |
|---|---|---|---|---|---|
| **13** | 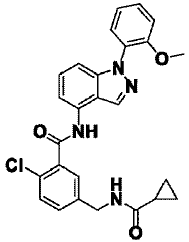 | **14** | 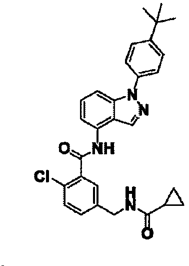 | **15** | 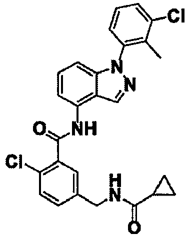 |
| **16** | 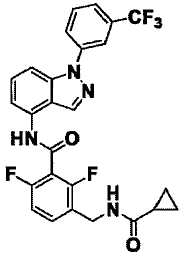 | **17** | 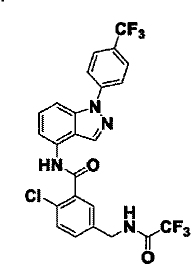 | **18** | 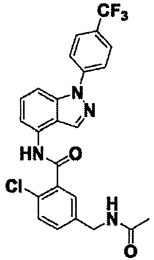 |
| **19** | 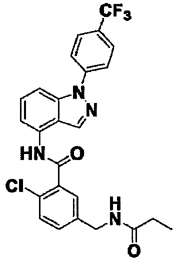 | **20** | 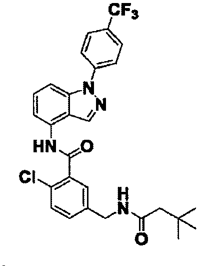 | **21** | 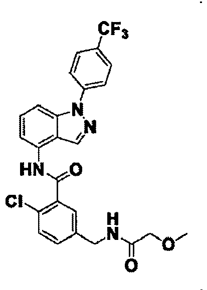 |
| **22** | 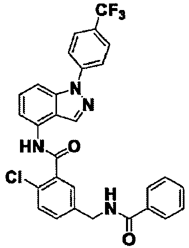 | **23** | 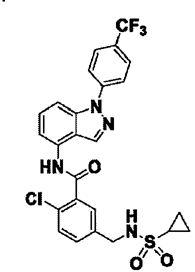 | **24** | 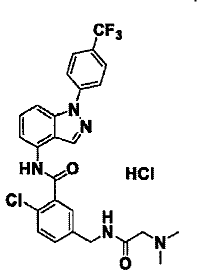 |

**[Table 3]**

| **Example** | **Structure** | **Example** | **Structure** | **Example** | **Structure** |
|---|---|---|---|---|---|
| **25** | 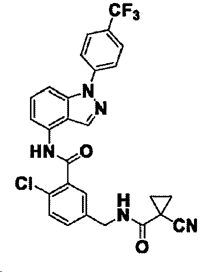 | **26** | 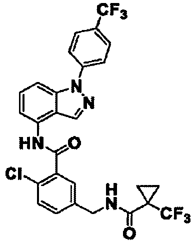 | **27** | 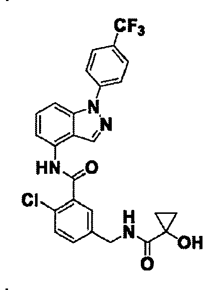 |
| **28** | 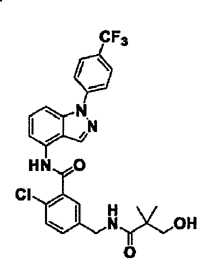 | **29** | 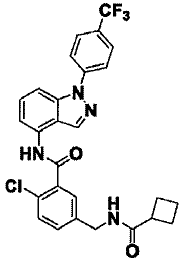 | **30** | 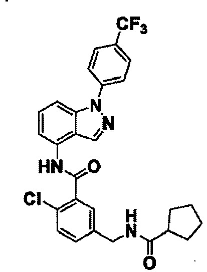 |
| **31** | 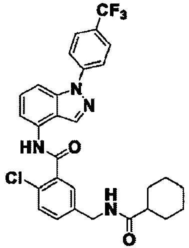 | **32** | 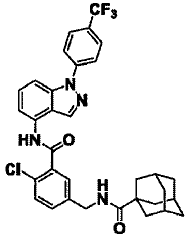 | **33** | 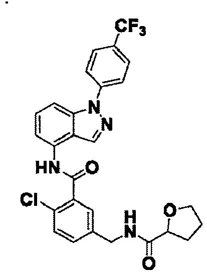 |
| **34** | 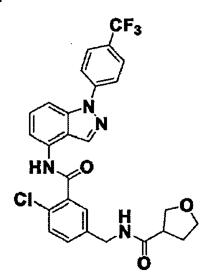 | **35** | 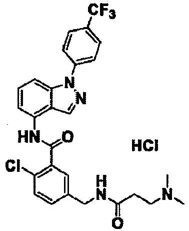 | **36** | 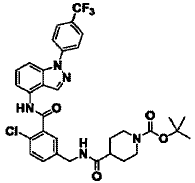 |

**[Table 4]**

| **Example** | **Structure** | **Example** | **Structure** | **Example** | **Structure** |
|---|---|---|---|---|---|
| **37** | 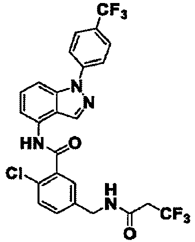 | **38** | 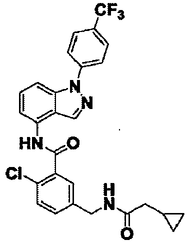 | **39** | 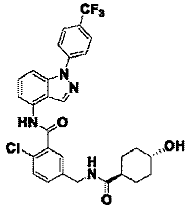 |
| **40** | 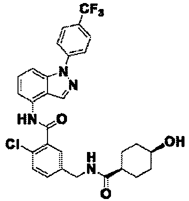 | **41** | 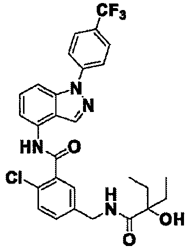 | **42** | 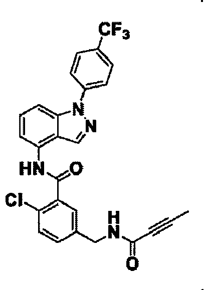 |
| **43** | 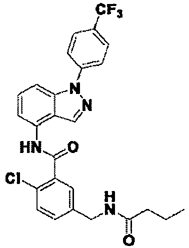 | **44** | 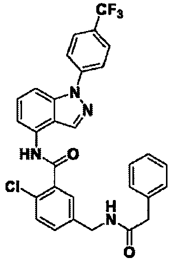 | **45** | 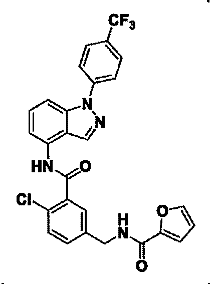 |
| **46** | 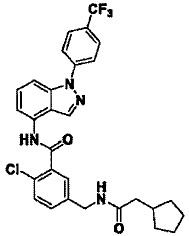 | **47** | 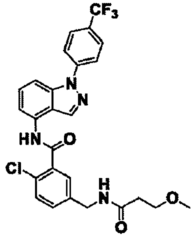 | **48** | 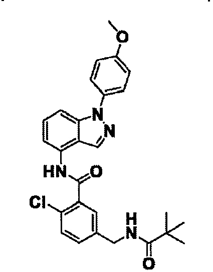 |

**[Table 5]**

| **Example** | **Structure** | **Example** | **Structure** | **Example** | **Structure** |
|---|---|---|---|---|---|
| **49** | 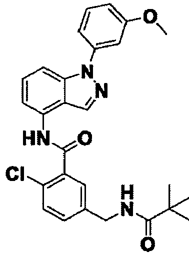 | **50** | 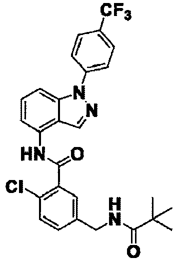 | **51** | 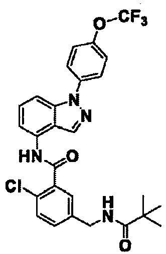 |
| **52** | 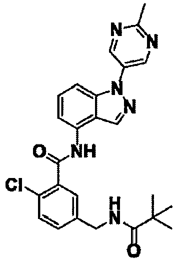 | **53** | 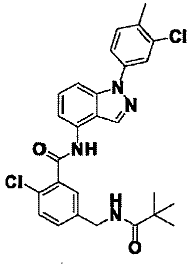 | **54** | 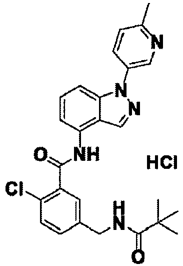 |
| **55** | 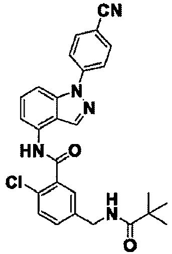 | **56** | 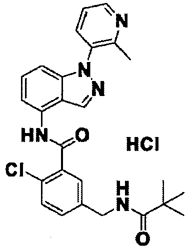 | **57** | 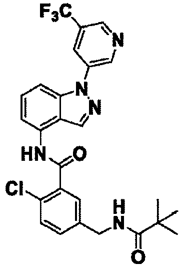 |
| **58** | 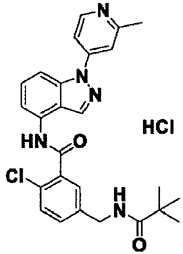 | **59** | 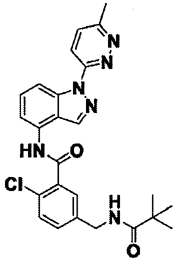 | **60** | 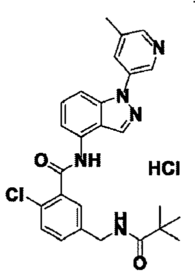 |

**[Table 6]**

| **Example** | **Structure** | **Example** | **Structure** | **Example** | **Structure** |
|---|---|---|---|---|---|
| **61** | 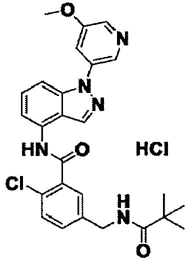 | **62** | 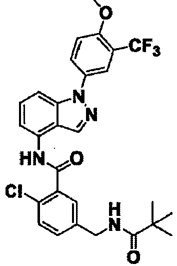 | **63** | 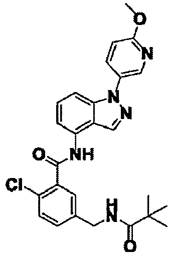 |
| **64** | 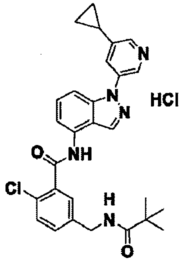 | **65** | 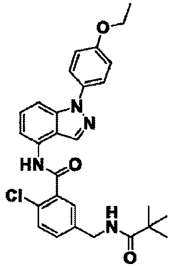 | **66** | 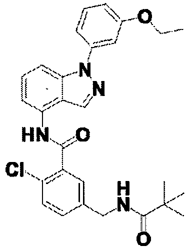 |
| **67** | 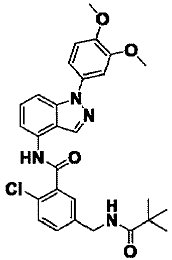 | **68** | 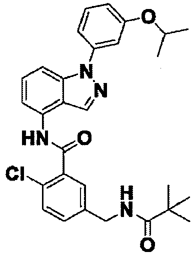 | **69** | 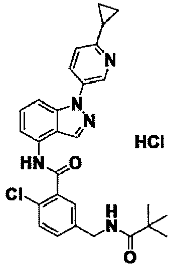 |
| **70** | 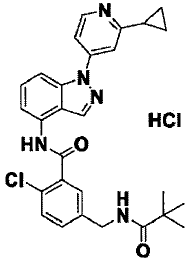 | **71** | 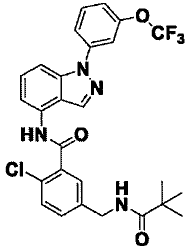 | **72** | 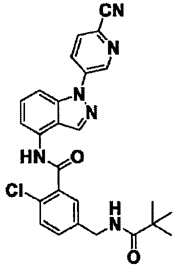 |

**[Table 7]**

| **Example** | **Structure** | **Example** | **Structure** | **Example** | **Structure** |
|---|---|---|---|---|---|
| **73** | 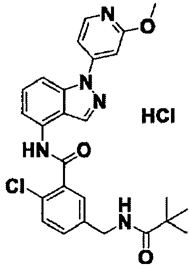 | **74** | 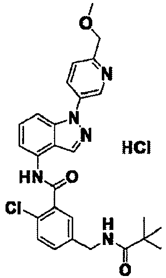 | **75** | 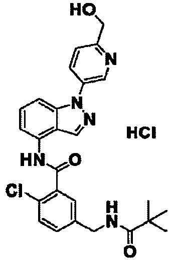 |
| **76** | 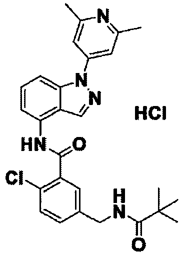 | **77** | 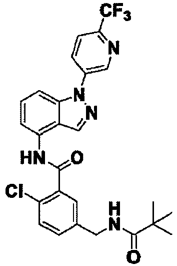 | **78** | 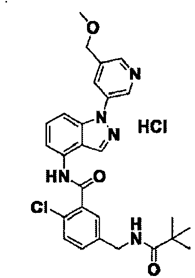 |
| **79** | 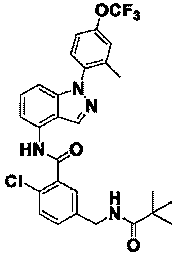 | **80** | 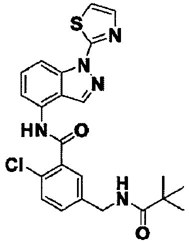 | **81** | 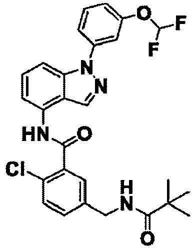 |
| **82** | 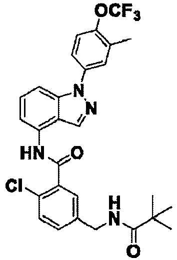 | **83** | 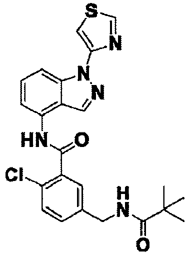 | **84** | 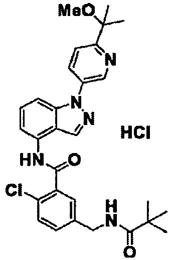 |

**[Table 8]**

| **Example** | **Structure** | **Example** | **Structure** | **Example** | **Structure** |
|---|---|---|---|---|---|
| **85** | 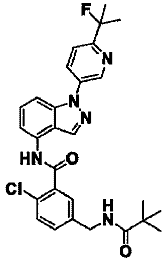 | **86** | 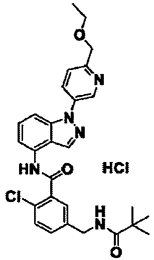 | **87** | 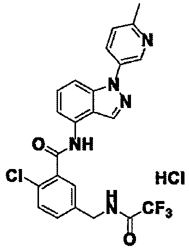 |
| **88** | 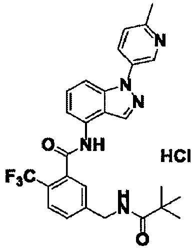 | **89** | 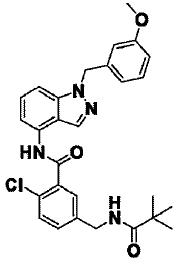 | **90** | 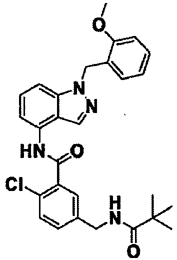 |
| **91** | 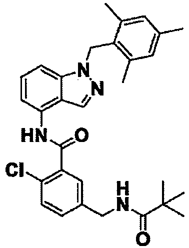 | **92** | 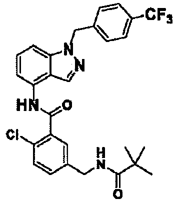 | **93** | 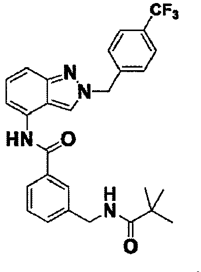 |
| **94** | 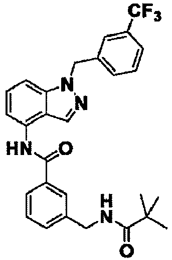 | **95** | 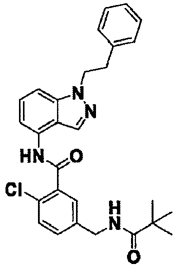 | **96** | 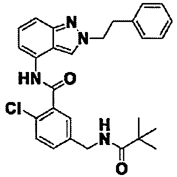 |

**[Table 9]**

| **Example** | **Structure** | **Example** | **Structure** | **Example** | **Structure** |
|---|---|---|---|---|---|
| **97** | 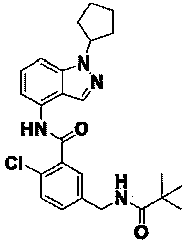 | **98** | 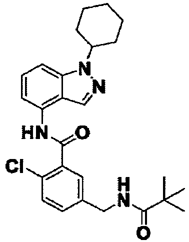 | **99** | 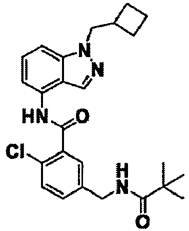 |
| **100** | 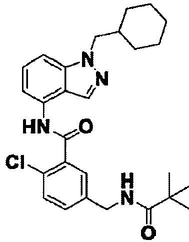 | **101** | 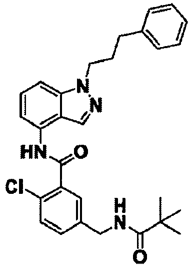 | **102** | 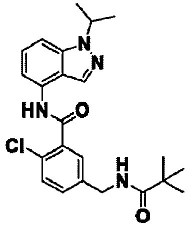 |
| **103** | 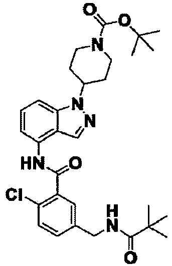 | **104** | 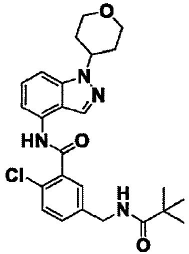 | **105** | 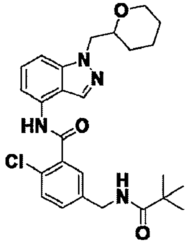 |
| **106** | 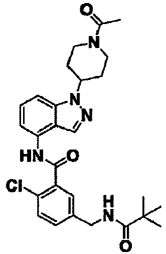 | **107** | 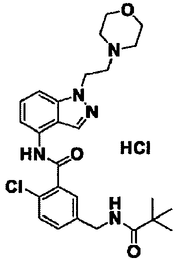 | **108** | 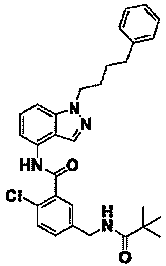 |

**[Table 10]**

| **Example** | **Structure** | **Example** | **Structure** | **Example** | **Structure** |
|---|---|---|---|---|---|
| **109** | 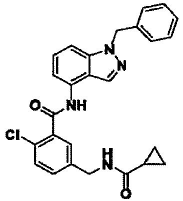 | **110** | 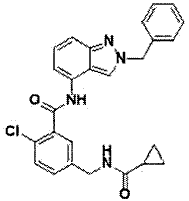 | **111** | 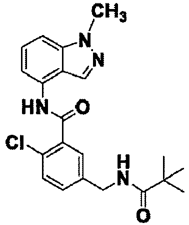 |
| **112** | 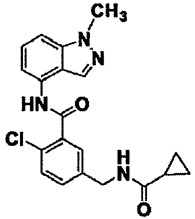 | **113** | 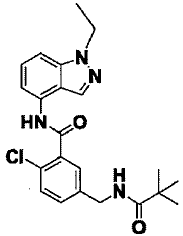 | **114** | 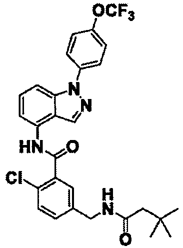 |
| **115** | 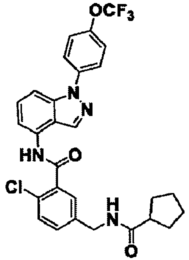 | **116** | 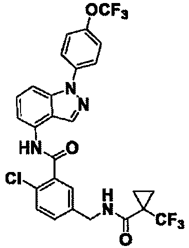 | **117** | 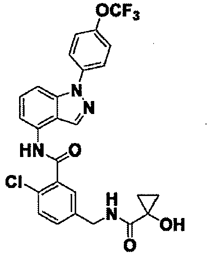 |
| **118** | 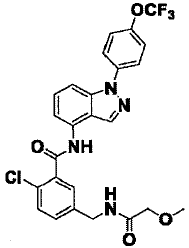 | **119** | 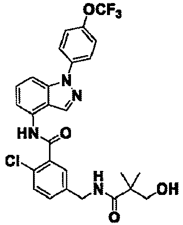 | **120** | 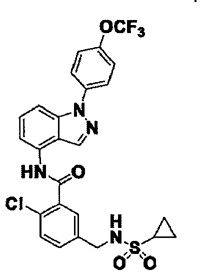 |

**[Table 11]**

| **Example** | **Structure** | **Example** | **Structure** | **Example** | **Structure** |
|---|---|---|---|---|---|
| **121** | 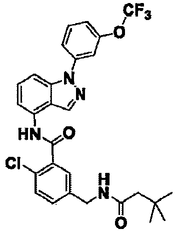 | **122** | 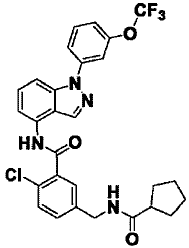 | **123** | 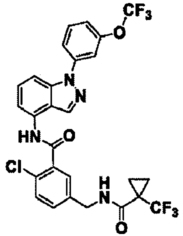 |
| **124** | 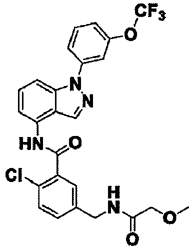 | **125** | 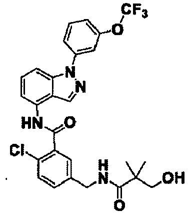 | **126** | 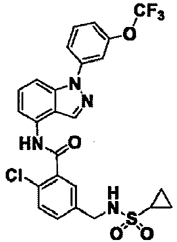 |
| **127** | 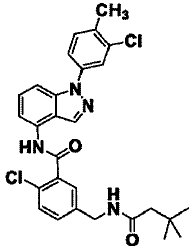 | **128** | 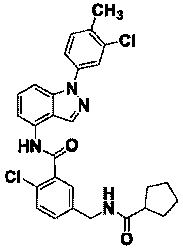 | **129** | 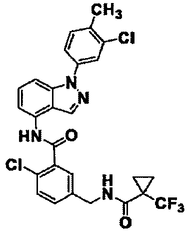 |
| **130** | 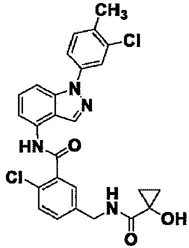 | **131** | 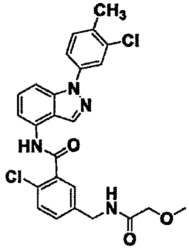 | **132** | 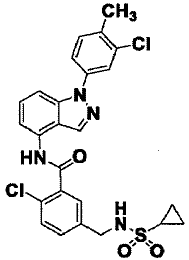 |

**[Table 12]**

| **Example** | **Structure** | **Example** | **Structure** | **Example** | **Structure** |
|---|---|---|---|---|---|
| **133** | 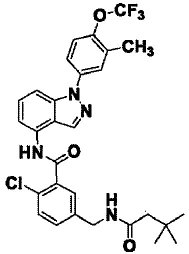 | **134** | 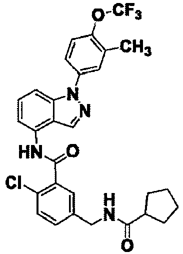 | **135** | 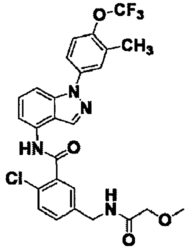 |
| **136** | 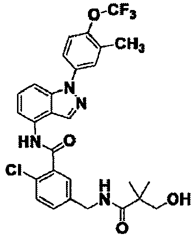 | **137** | 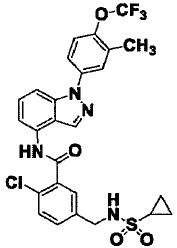 | **138** | 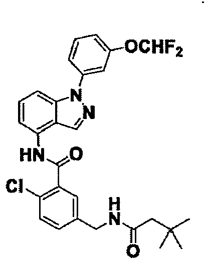 |
| **139** | 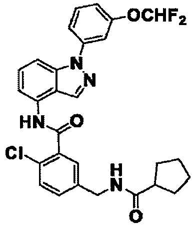 | **140** | 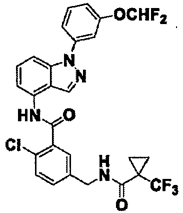 | **141** | 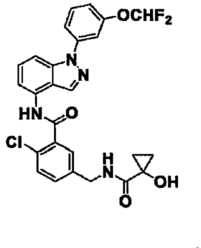 |
| **142** | 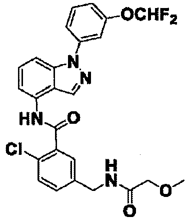 | **143** | 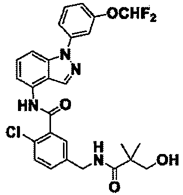 | **144** | 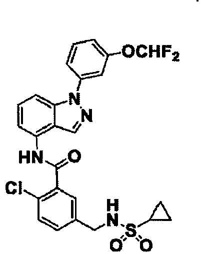 |

**[Table 13]**

| **Example** | **Structure** | **Example** | **Structure** | **Example** | **Structure** |
|---|---|---|---|---|---|
| **145** | 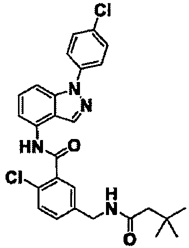 | **146** | 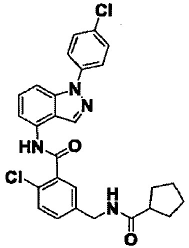 | **147** | 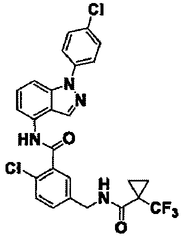 |
| **148** | 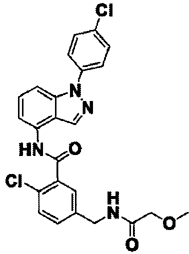 | **149** | 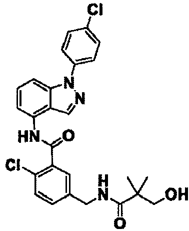 | **150** | 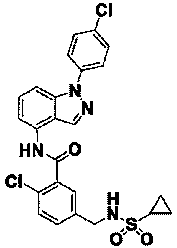 |
| **151** | 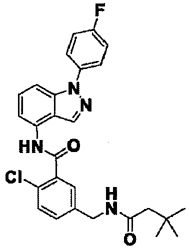 | **152** | 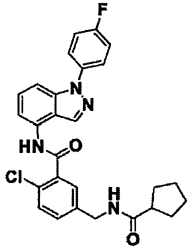 | **153** | 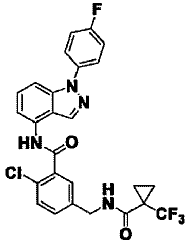 |
| **154** | 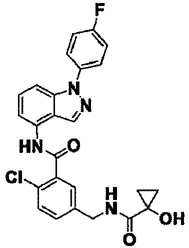 | **155** | 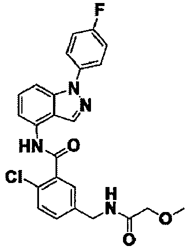 | **156** | 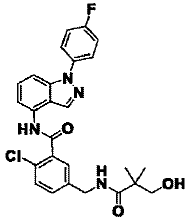 |

**[Table 14]**

| **Example** | **Structure** | **Example** | **Structure** | **Example** | **Structure** |
|---|---|---|---|---|---|
| **157** | 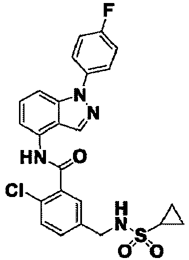 | **158** | 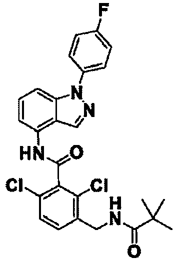 | **159** | 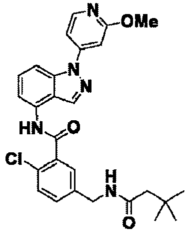 |
| **160** | 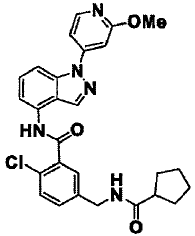 | **161** | 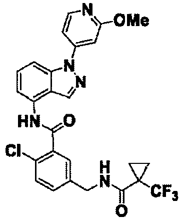 | **162** | 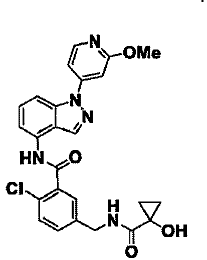 |
| **163** | 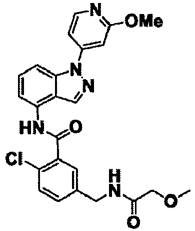 | **164** | 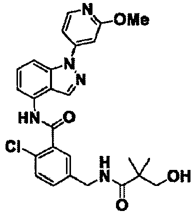 | **165** | 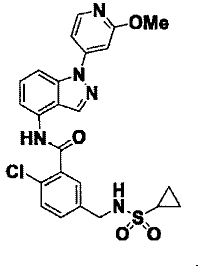 |
| **166** | 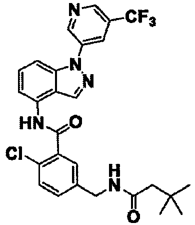 | **167** | 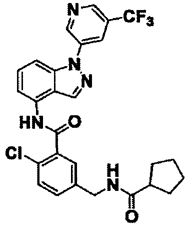 | **168** | 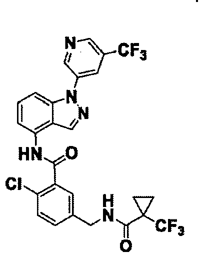 |

**[Table 15]**

| **Example** | **Structure** | **Example** | **Structure** | **Example** | **Structure** |
|---|---|---|---|---|---|
| **169** | 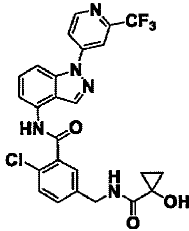 | **170** | 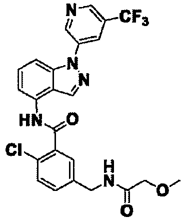 | **171** | 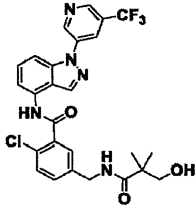 |
| **172** | 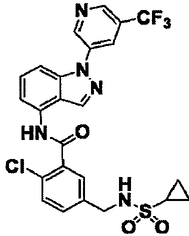 | **173** | 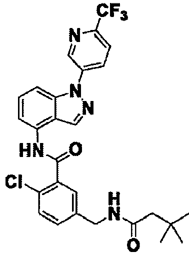 | **174** | 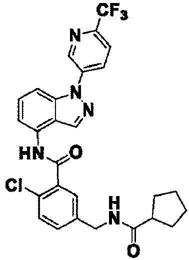 |
| **175** | 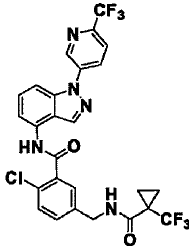 | **176** | 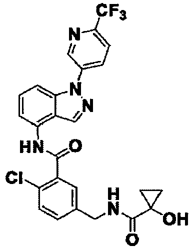 | **177** | 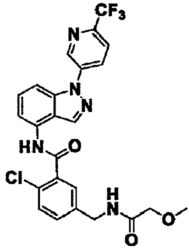 |
| **178** | 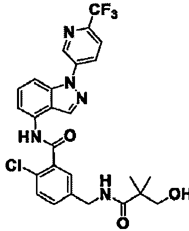 | **179** | 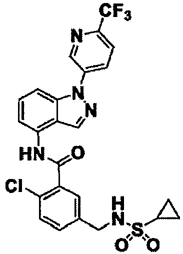 | **180** | 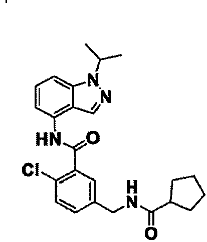 |

**[Table 16]**

| **Example** | **Structure** | **Example** | **Structure** | **Example** | **Structure** |
|---|---|---|---|---|---|
| **181** | 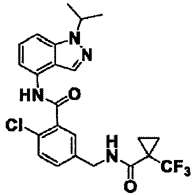 | **182** | 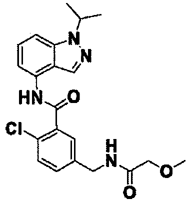 | **183** | 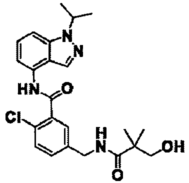 |
| **184** | 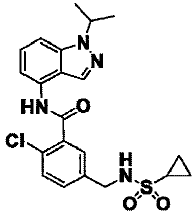 | **185** | 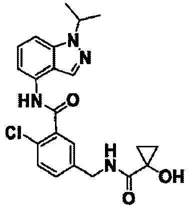 | **186** | 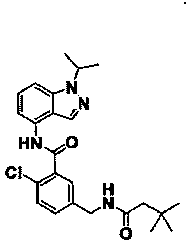 |
| **187** | 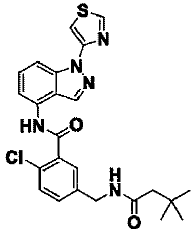 | **188** | 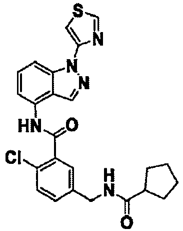 | **189** | 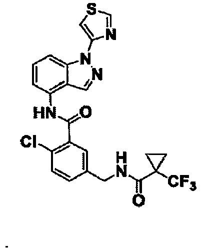 |
| **190** | 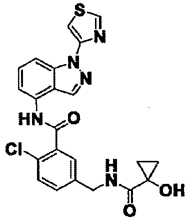 | **191** | 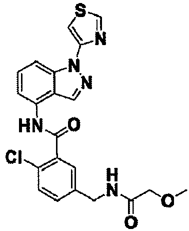 | **192** | 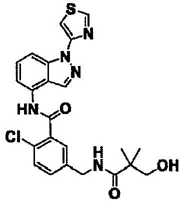 |

**[Table 17]**

| **Example** | **Structure** | **Example** | **Structure** | **Example** | **Structure** |
|---|---|---|---|---|---|
| **193** | 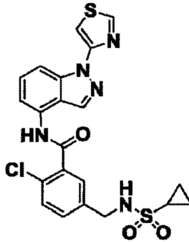 , | **194** | 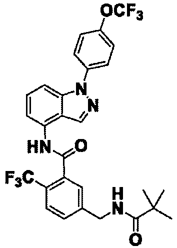 | **195** | 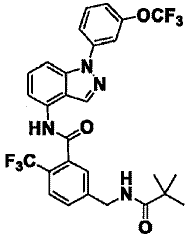 |
| **196** | 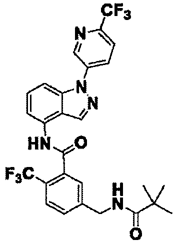 | **197** | 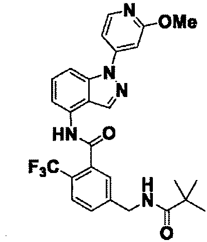 | **198** | 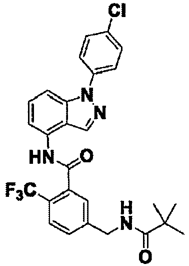 |
| **199** | 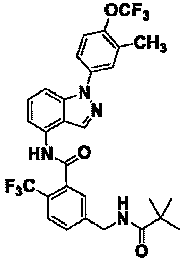 | **200** | 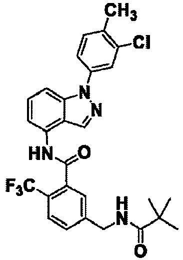 | **201** | 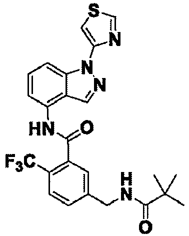 |
| **202** | 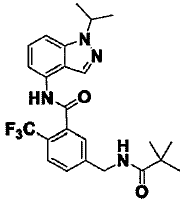 | **203** | 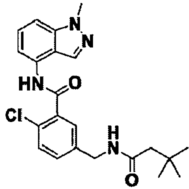 | **204** | 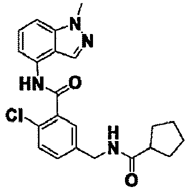 |

**[Table 18]**

| **Example** | **Structure** | **Example** | **Structure** | **Example** | **Structure** |
|---|---|---|---|---|---|
| **205** | 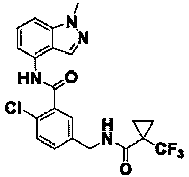 | **206** | 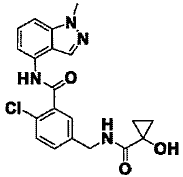 | **207** | 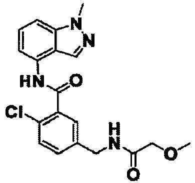 |
| **208** | 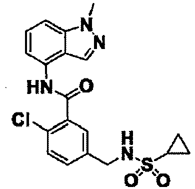 | **209** | 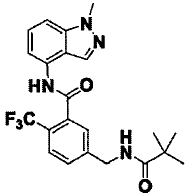 | **210** | 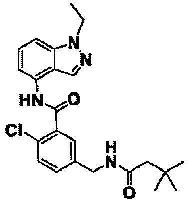 |
| **211** | 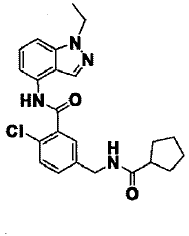 | **212** | 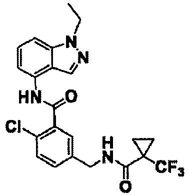 | **213** | 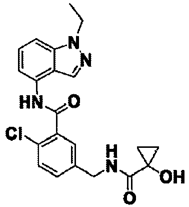 |
| **214** | 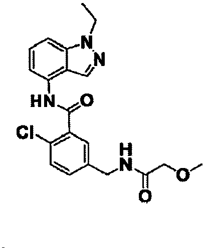 | **215** | 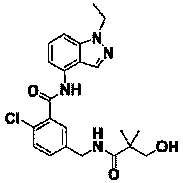 | **216** | 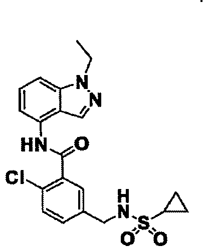 |

**[Table 19]**

| **Example** | **Structure** | **Example** | **Structure** | **Example** | **Structure** |
|---|---|---|---|---|---|
| **217** | 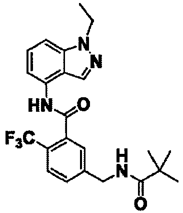 | **218** | 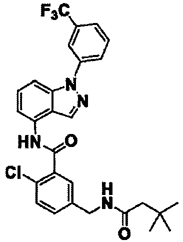 | **219** | 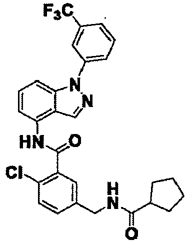 |
| **220** | 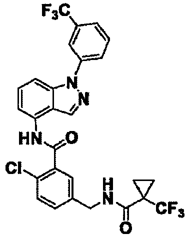 | **221** | 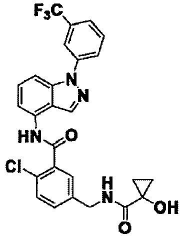 | **222** | 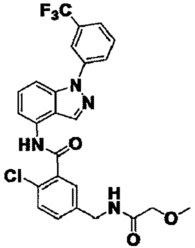 |
| **223** | 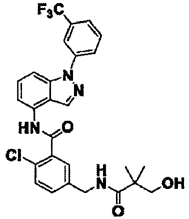 | **224** | 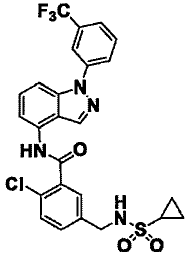 | **225** | 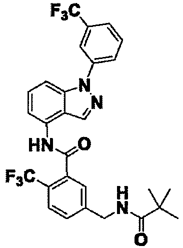 |
| **226** | 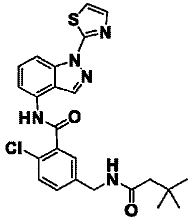 | **227** | 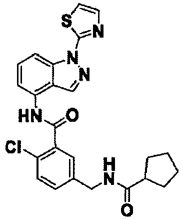 | **228** | 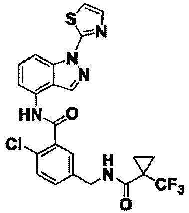 |

**[Table 20]**

| **Example** | **Structure** | **Example** | **Structure** | **Example** | **Structure** |
|---|---|---|---|---|---|
| **229** | 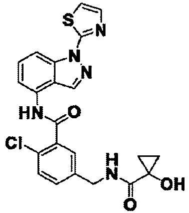 | **230** | 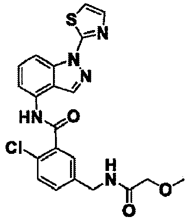 | **231** | 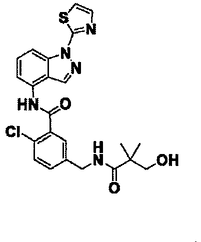 |
| **232** | 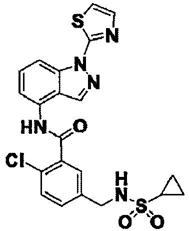 | **233** | 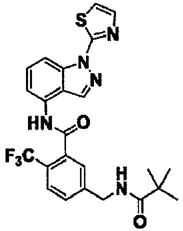 | **234** | 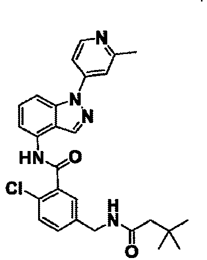 |
| **235** | 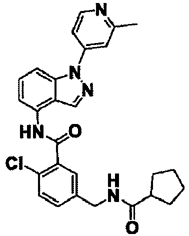 | **236** | 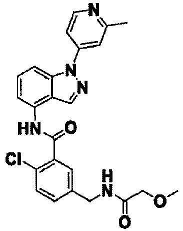 | **237** | 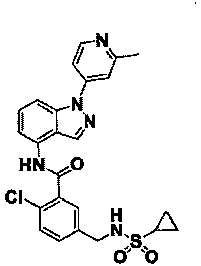 |
| **238** | 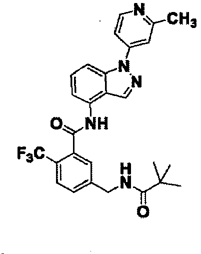 | **239** | 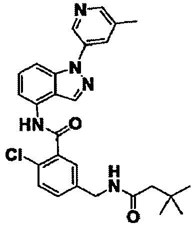 | **240** | 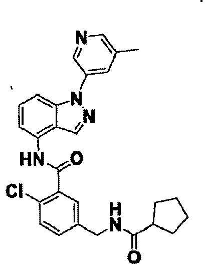 |

**[Table 21]**

| **Example** | **Structure** | **Example** | **Structure** | **Example** | **Structure** |
|---|---|---|---|---|---|
| **241** | 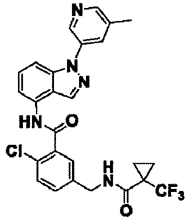 | **242** | 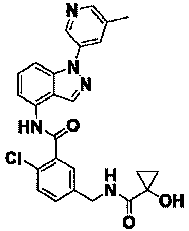 | **243** | 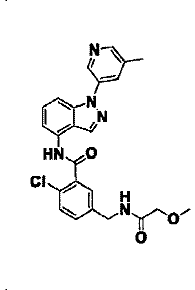 |
| **244** | 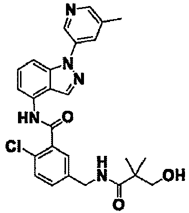 | **245** | 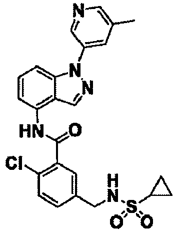 | **246** | 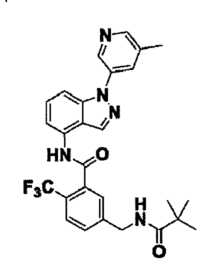 |
| **247** | 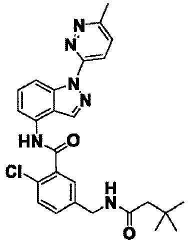 | **248** | 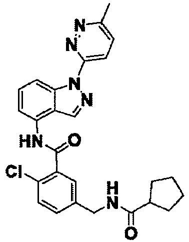 | **249** | 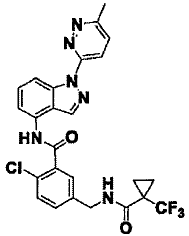 |
| **250** | 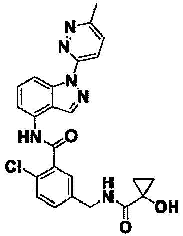 | **251** | 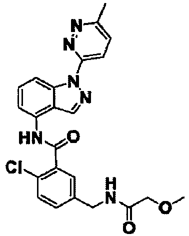 | **252** | 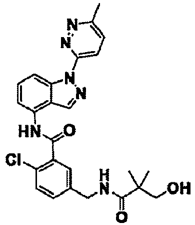 |

**[Table 22]**

| **Example** | **Structure** | **Example** | **Structure** | **Example** | **Structure** |
|---|---|---|---|---|---|
| **253** | 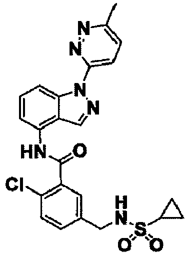 | **254** | 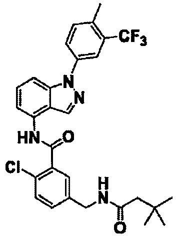 | **255** | 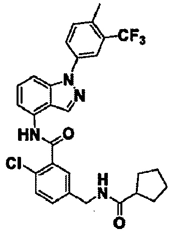 |
| **256** | 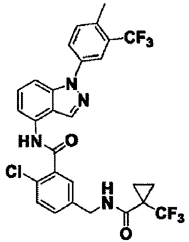 | **257** | 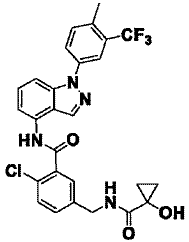 | **258** | 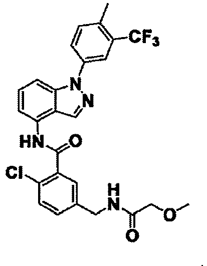 |
| **259** | 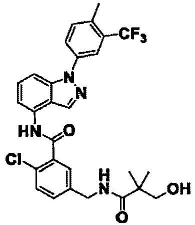 | **260** | 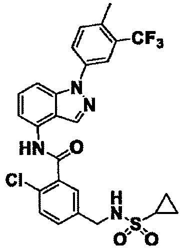 | **261** | 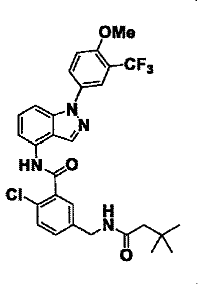 |
| **262** | 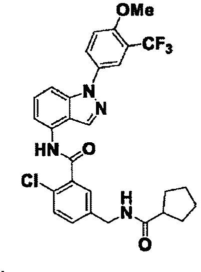 | **263** | 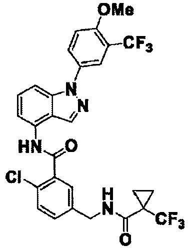 | **264** | 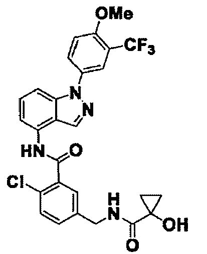 |

**[Table 23]**

| **Example** | **Structure** | **Example** | **Structure** | **Example** | **Structure** |
|---|---|---|---|---|---|
| **265** | 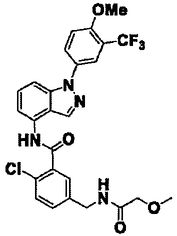 | **266** | 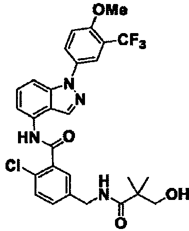 | **267** | 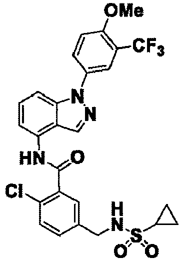 |
| **268** | 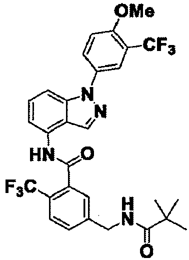 | **269** | 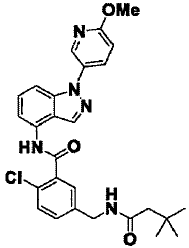 | **270** | 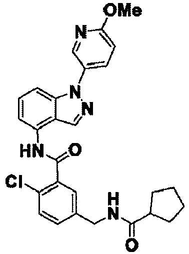 |
| **271** | 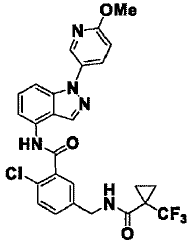 | **272** | 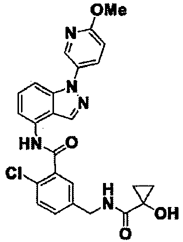 | **273** | 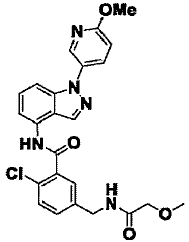 |
| **274** | 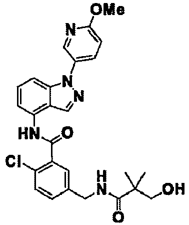 | **275** | 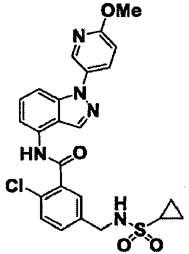 | **276** | 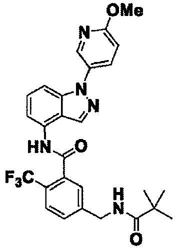 |

**[Table 24]**

| **Example** | **Structure** | **Example** | **Structure** | **Example** | **Structure** |
|---|---|---|---|---|---|
| **277** | 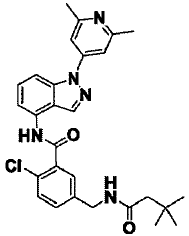 | **278** | 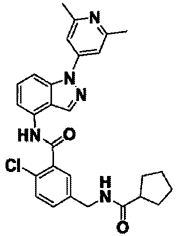 | **279** | 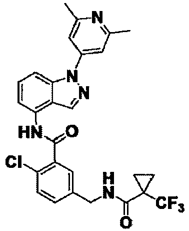 |
| **280** | 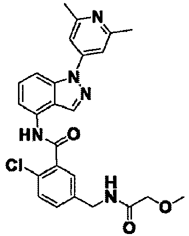 | **281** | 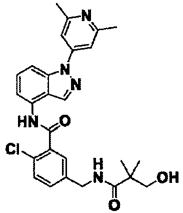 | **282** | 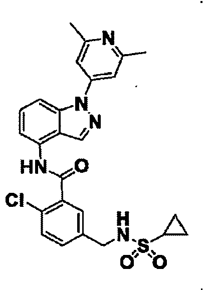 |
| **283** | 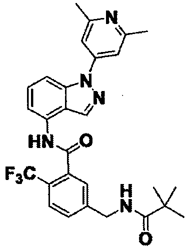 | **284** | 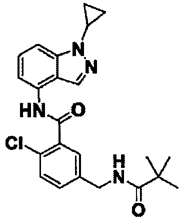 | **285** | 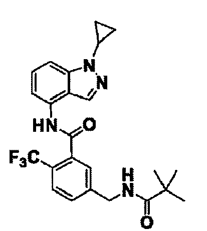 |

### Test Example 1: mPGES-1 Inhibitory Activity Test

mPGES-1 microsomal specimens were prepared by transiently transfecting CHO-K1 cells (DS Pharma Biomedical Co. Ltd) with a plasmid encoding human mPGES-1 cDNA using FuGENE 6 Transfection Reagent (Promega). The prepared mPGES-1 microsome specimens were diluted in potassium phosphate buffer of pH 7.4 comprising 2.5 mM reduced glutathione (Sigma-Aldrich), DMSO solution of a test compound or DMSO was added to a final concentration of 1% of DMSO, and then incubated at 4°C for 20 minutes. The enzymatic reaction was then initiated by adding a solution of PGH2 (Cayman chemical) substrate of a final concentration 1 µM and incubated at 4°C for 60 seconds. The reaction was terminated by adding a solution of ferric chloride (STREM CHEMICALS) and citric acid (Wako Pure Chemical Industries) salt to a final concentration of 5 mg/mL and 250 mM, respectively. The formed PGE2 was quantified using the HTRF kit (Cisbio International). Solutions without the test compound were used as positive controls, and solutions without the test compound and microsomal specimens were used as negative controls. 100% activity was defined as production of PGE2 in the positive control minus that in the negative control. The percentage inhibition of production of PGE2 at final concentrations of 10 nM and 100 nM of the test compound was then calculated, or IC50 values were determined using standard methods.

### Test Example 2: Inhibition test of PGE2 and PGF2α production using human A549 cells

100 µL of RPMI medium (Sigma) comprising 10% FBS and human A549 cells was seeded in a 96-well plate to 2 × 10⁴ cells per well, and incubated overnight. The medium was then removed and washed with phosphate-buffered saline, and added to a DMSO solution of the test compound or RPMI medium comprising 3% FBS with DMSO to a final concentration of 0.1% DMSO. After incubation for 60 minutes, IL-1β (5 ng/well, BioLegend) was added to the solution. After incubation at 37°C for 24 h, PGE2 in the medium was quantified using the HTRF kit (Cisbio International) and PGF2α was also quantified using the EIA kit (Cayman chemical). Solutions without the test compound were used as positive controls, and solutions without the test compound and IL-1β were used as negative controls. 100% activity was defined as production of PGE2 and PGF2α in the positive control minus those in the negative control. IC50 values were then determined using standard methods.

The results of Test Examples 1 and 2 are shown in Tables 25 to 31 ("-" in the tables indicates not applicable).

**[Table 25]**

| Example | mPGES-1 inhibition IC₅₀(nM) | A549 cell inhibition of PGE2 production IC₅₀(nM) | Example | mPGES-1 inhibition IC₅₀(nM) | A549 cell inhibition of PGE2 production IC₅₀(nM) |
|---|---|---|---|---|---|
| 1 | 1.5 | 4.5 | 31 | 1.6 | 5.5 |
| 2 | 0.2 | 6.3 | 32 | 23.0 | 12.1 |
| 3 | 1.2 | 0.7 | 33 | 18.0 | 41.4 |
| 4 | 18.8 | - | 34 | 31.0 | 20.7 |
| 5 | 9.6 | 688 | 35 | 116 | 933 |
| 6 | 1.3 | 0.6 | 36 | 1.4 | 1.7 |
| 7 | 1.8 | 1.6 | 37 | 8.9 | 2.5 |
| 8 | 3.9 | 11.3 | 38 | 5.7 | 7.5 |
| 9 | 2.2 | 4.1 | 39 | 7.5 0 | 5.1 |
| 10 | 10.0 | 31.2 | 40 | 19.1 | 60.2 |
| 11 | 2.9 | 3.4 | 41 | 1.0 | 13.7 |
| 12 | 2.7 | 1.1 | 42 | 391 | - |
| 13 | 2.0 | 505 | 43 | 2.3 | 13.5 |
| 14 | 1.1 | 0.4 | 44 | 7.2 | 151 |
| 1 5 | 5.6 | 7.8 | 45 | 1.2 | 37.6 |
| 16 | 6.3 | 9.7 | 46 | 3.0 | 14.3 |
| 17 | 17.0 | 1.6 | 47 | 8.0 | 8.5 |
| 18 | 84.7 | 52.4 | 48 | 3.3 | 4.0 |
| 19 | 6.1 | 1.0 | 49 | 9.9 | 0.9 |
| 20 | <1.0 | 2.8 | 50 | 14.7 | 3.5 |
| 21 | 3.8 | 68.0 | 51 | 5.0 | 112 |
| 22 | 3.3 | 12.8 | 52 | 3.6 | 20.9 |
| 23 | 5.2 | 1.5 | 53 | 2.6 | 1.8 |
| 24 | 57.1 | 46.4 | 54 | 9.5 | 6.2 |
| 25 | 3.9 | 8.6 | 55 | 1.3 | 1.8 |
| 26 | 1.6 | 2.3 | 56 | 23.6 | 183 |
| 27 | 10.0 | 4.4 | 57 | 19.8 | 5.7 |
| 28 | 1.9 | 5.8 | 58 | 5.9 | 5.9 |
| 29 | 4.5 | 6.7 | 59 | 34.2 | 24.4 |
| 30 | 3.5 | 2.6 | 60 | 6.7 | 10.4 |

**[Table 26]**

| Example | mPGES-1 inhibition IC₅₀(nM) | A549 cell inhibition of PGE2 production IC₅₀(nM) | Example | mPGES-1 inhibition IC₅₀(nM) | A549 cell inhibition of PGE2 production IC₅₀(nM) |
|---|---|---|---|---|---|
| 61 | 3.5 | 2.9 | 91 | <1.0 | 5.1 |
| 62 | 2.4 | 3.6 | 92 | 5.4 | 4.2 |
| 63 | 1.0 | 13.7 | 94 | 10.0 | 9.2 |
| 64 | 1.4 | 1.9 | 95 | 1.2 | 2.7 |
| 65 | 2.2 | 3.6 | 97 | 0.7 | 5.3 |
| 66 | 0.2 | 4.0 | 98 | 9.0 | 1.6 |
| 67 | 3.7 | 5.1 | 99 | 1.3 | 4.1 |
| 68 | 6.7 | 2.0 | 100 | 1.0 | 1.9 |
| 69 | 3.8 | 2.1 | 101 | 4.7 | 8.1 |
| 70 | 4.3 | 1.0 | 102 | 3.1 | 20.7 |
| 71 | 0.1 | 0.6 | 103 | 2.7 | 4.0 |
| 72 | 6.2 | 11.5 | 104 | 99.9 | 80.6 |
| 73 | 1.2 | 3.3 | 105 | 1.3 | 11.1 |
| 74 | 1.3 | 15.6 | 106 | 175 | 884 |
| 75 | 71.1 | 260 | 107 | 10 | 399 |
| 76 | 2.4 | 5.2 | 108 | 11.1 | 20.6 |
| 77 | 1.1 | 1.0 | 109 | 2.04 | 119 |
| 78 | 10.4 | 9.8 | 111 | 5.5 | 40.6 |
| 79 | 1.0 | 3.4 | 112 | 12.9 | 91.1 |
| 80 | 1.1 | 5.2 | 113 | 1.2 | 20.2 |
| 81 | 14.9 | 0.3 | | | |
| 82 | 10.0 | 0.3 | | | |
| 83 | 1.5 | 5.3 | | | |
| 84 | 0.8 | 4.9 | | | |
| 85 | 8.6 | 6.0 | | | |
| 86 | 15.7 | 101 | | | |
| 87 | 4.7 | 72.0 | | | |
| 88 | 1.5 | 68.2 | | | |
| 89 | 3.5 | 20.7 | | | |
| 90 | 2.2 | 17.7 | | | |

**[Table 27]**

| Example | mPGES-1 inhibition (%) | | A549 cell inhibition of PGE2 production IC₅₀(nM) |
|---|---|---|---|
| | 10nM | 100nM | |
| 114 | 87 | - | 1.9 |
| 115 | 61 | - | 1.2 |
| 116 | 68 | 100 | 0.6 |
| 121 | 48 | - | 10.0 |
| 122 | 100 | - | 2.9 |
| 123 | 98 | - | 5.1 |
| 125 | 62 | - | 3.5 |
| 126 | 100 | - | 9.8 |
| 127 | 64 | - | 4.7 |
| 129 | 67 | - | 1.2 |
| 132 | 86 | - | 1.5 |
| 138 | 61 | 100 | 6.0 |
| 141 | 54 | 100 | 59.4 |
| 143 | 92 | 100 | 17.4 |
| 144 | 73 | 100 | 1.8 |
| 145 | 92 | - | 6.3 |
| 147 | 85 | - | 0.9 |
| 148 | 70 | 100 | 4.2 |
| 149 | 94 | - | 3.2 |
| 150 | 72 | - | 5.9 |
| 151 | 100 | - | 14.3 |

**[Table 28]**

| Example | mPGES-1 inhibition (%) | | A549 cell inhibition of PGE2 production IC₅₀(nM) |
|---|---|---|---|
| | 10nM | 100nM | |
| 152 | 58 | - | 7.6 |
| 153 | 100 | - | 1.0 |
| 154 | 58 | 89 | 17.6 |
| 155 | 100 | - | 5.9 |
| 156 | 63 | 86 | 7.1 |
| 157 | 100 | - | 14.6 |
| 158 | 49 | 100 | 1.6 |
| 159 | 55 | 100 | 9.1 |
| 164 | 69 | 100 | 28.4 |
| 165 | 80 | 96 | 8.6 |
| 167 | 60 | 85 | 1.0 |
| 168 | 53 | 92 | 1.2 |
| 171 | - | 100 | 9.6 |
| 172 | 100 | | 5.6 |
| 173 | 54 | 86 | 6.7 |
| 174 | 85 | 86 | 1.9 |
| 179 | 59 | 95 | 4.4 |
| 180 | 75 | - | 40.6 |
| 188 | 68 | - | 102 |
| 189 | 80 | - | 23.6 |
| 192 | 70 | 90 | 31.4 |

**[Table 29]**

| Example | mPGES-1 inhibition (%) | | A549 cell inhibition of PGE2 production IC₅₀(nM) |
|---|---|---|---|
| | 10nM | 100nM | |
| 193 | 71 | - | 33.0 |
| 194 | 62 | 83 | 0.7 |
| 195 | 65 | 84 | 0.8 |
| 196 | 60 | 78 | 2.0 |
| 200 | 69 | 103 | 0.9 |
| 201 | 85 | 97 | 20.1 |
| 202 | 59 | 79 | 20.2 |
| 204 | 57 | 85 | 43.6 |
| 207 | 59 | - | 303 |
| 208 | 63 | 68 | 799 |
| 209 | 76 | 90 | 38.4 |
| 210 | 64 | - | 379 |
| 211 | 84 | - | 65.8 |
| 212 | 71 | - | 17.3 |
| 217 | 79 | 80 | 14.7 |
| 219 | 74 | 93 | 1.0 |
| 220 | 74 | 90 | <1.0 |
| 221 | 65 | 100 | 2.6 |
| 222 | 61 | - | 3.3 |
| 223 | 78 | - | 0.6 |
| 224 | 83 | - | <1.00 |

**[Table 30]**

| Example | mPGES-1 inhibition (%) | | A549 cell inhibition of PGE2 production IC₅₀(nM) |
|---|---|---|---|
| | 10nM | 100nM | |
| 227 | 81 | - | 10.0 |
| 228 | 77 | - | 12.9 |
| 235 | 53 | 88 | 6.1 |
| 246 | 78 | 89 | 3.5 |
| 253 | 67 | 75 | 299 |
| 254 | 100 | 99 | 0.7 |
| 256 | 88 | 98 | 0.3 |
| 257 | 94 | 96 | 4.3 |
| 258 | 84 | 100 | 3.2 |
| 259 | 92 | 92 | 0.5 |
| 260 | 89 | 101 | 1.0 |
| 261 | 95 | 100 | 1.4 |
| 262 | 99 | 94 | 2.0 |
| 263 | 89 | 85 | 0.3 |
| 264 | 94 | 97 | 5.1 |
| 265 | 95 | 100 | 2.7 |
| 270 | 64 | 88 | 3.5 |
| 271 | 50 | 90 | 3.0 |
| 275 | 66 | 91 | 3.9 |
| 276 | 68 | 94 | 3.0 |
| 278 | 72 | 90 | 3.1 |

**[Table 31]**

| Example | mPGES-1 inhibition (%) | | A549 cell inhibition of PGE2 production IC₅₀(nM) |
|---|---|---|---|
| | 10nM | 100nM | |
| 283 | 72 | 96 | 1.9 |
| 284 | 79 | - | 9.6 |
| 285 | 75 | - | 7.7 |

### Test Example 3: Inhibition test of production of PGE2 using human whole blood

The human whole blood assay was carried out according to Brideau et al. (Inflamm. Res., vol. 45, p. 68, 1996). Newly collected venous blood from volunteers was drawn into heparin-filled tubes. These subjects had no appearance of external inflammatory symptoms and had not taken an NSAID for at least 7 days prior to blood collection. A DMSO solution of the test compound or DMSO (both of them having 0.25% DMSO final concentration) was added to the blood and incubated at 37°C for 20 minutes. Then, bacterial LPS (E. coli serotype 0111: B4 diluted in phosphate buffered saline) was added at a final concentration of 100 µg/mL and incubated at 37°C for 24 h. After incubation for 24 h, the blood was centrifuged at 2000 rpm for 5 min at 4°C, and PGE2 in the supernatant was quantified using the HTRF kit (Cisbio International product catalog #62P2APEC). Solutions without the test compound were used as positive controls, and solutions without the test compound and LPS were used as negative controls. 100% activity was defined as production of PGE2 in the positive control minus production of PGE2 in the negative control. IC50 values were then determined using standard methods. The compounds of the present invention also inhibited PGE2 production in human whole blood.

### Test Example 4: Pharmacokinetic study in rats

The test compound was suspended in 0.5% methylcellulose solution to prepare a 10 mg/5 mL dose solution. Female SD rats under fasting conditions were orally administered 10 mg/kg of the test compound and blood was collected from the jugular vein at 1, 2, 4, and 6 hours after administration. The blood was centrifuged to separate the plasma, and the plasma concentration of the test compound was determined by HPLC after the plasma was deproteinized.

### Test Example 5: Evaluation of the effect on the suppression of urinary output in a guinea pig model of water-loaded polyuria

The water-loaded polyuria model, commonly used as a model for polyuria, was used to evaluate the inhibitory effect of the test compounds on urinary output in the following manner. Female Hartley guinea pigs (Japan SLC Co., Ltd.) were acclimated in a metabolic cage (Natsume Seisakusho Co., Ltd.) in a fasted condition from the evening of one day before the test. On the following day, 20 mL/kg of distilled water was administered orally as a water load, and then the guinea pigs were placed back in the metabolic cage and the total urinary output up to 3 hours after the water load was downloaded into a personal computer via an electronic balance (GX-200, A&D Corporation) and a data collection and analysis system (PowerLab^{®}, ADInstruments), and then analyzed using analyzing software (LabChart^{®}, ADInstruments). Test compounds dissolved in 0.5% methylcellulose solution (vehicle) or vehicle only were administered orally immediately before water loading depending on the kinetics of the compound. Based on the total urinary output at 3 hours after administration of the test compound and vehicle groups, the percentage inhibition of urinary output of the test compound group (%) was calculated relative to the urinary output of the vehicle group.

**[Table 32]**

| Example | Suppression (%) |
|---|---|
| Example 59 | 21.2 |
| Example 88 | 34.5 |
| Example 69 | 57.8 |
| Example 70 | 25.1 |
| Example 125 | 58.0 |
| Example 276 | 35.1 |

### Test Example 6: Evaluation of effect on urinary volume suppression in a monkey water-loaded polyuria model

The urinary volume suppression effect of the test compounds was evaluated in a water-loaded polyuria model using monkeys, whose urinary function is considered to be more similar to that of humans. Female cynomolgus monkeys were fasted from the evening of one day before the test. After oral administration of 50 mL/kg of distilled water as a water load on the following day as acclimation, the monkeys were placed in a metabolic cage and the total urinary output up to 6 hours after the water load was downloaded into the computer system for data acquisition via an electronic balance (GX-6100R, A&D Corporation) and analyzed (hardware: Data Sciences International, analysis software: NOTOCORD SYSTEMS). Acclimation was carried out three times in total. Evaluation of test compounds was conducted in the same manner as for acclimation, and test compounds dissolved in 0.5% methylcellulose solution (vehicle) or vehicle only were administered orally either 2 hours before or immediately before water loading, depending on the kinetics of the compounds.

### Test Example 7: Evaluation of Sodium Concentration in Plasma in a Rat Water-Loaded Polyuria Model

The water-loaded polyuria model of rat was used to evaluate the effect of the test compounds on lowering plasma sodium concentrations. Female SD rats (Japan SLC Co., Ltd.) were fasted from the evening of one day before the test. At 2 or 3 hours after oral administration of 25 mL/kg of distilled water as water load, blood was collected using a heparinized syringe. Plasma was obtained by centrifuging the resulting blood at 4°C for 10 minutes at 1000 rpm. Sodium concentration in plasma was measured using a fully automated electrolyte analyzer (PVA-EXII, A&T). Test compounds dissolved in 0.5% methylcellulose solution (vehicle) or vehicle only were administered orally either 2 hours before or immediately before water loading, depending on the kinetics of the compounds.

Desmopressin, an existing drug for the treatment of nocturnal polyuria, is known to cause hyponatremia as a side effect. In this study, we examined the effect of the compounds of the present invention on sodium concentration in plasma and found that the compounds had no effect on sodium concentration in plasma even when exposed to four times of the effective dose of drug.

| | |
|---|---|
| Formulation 1: 80 mg tablet for oral administration | |
| Compound of Example 1 | 5.0 mg |
| Corn starch | 46.6 mg |
| Cellulose, crystalline | 24.0 mg |
| Methylcellulose | 4.0 mg |
| Magnesium stearate | 0.4 mg |

The mixed powder in this ratio is tableted to form oral tablet by the usual method.

### [Industrial applicability]

The compounds of the present invention or pharmaceutically acceptable salts thereof exhibit mPGES-1 inhibitory activity and are applicable in the manufacture of a medicament for the treatment or prevention of diseases involving mPGES-1.

## Claims

1. A compound represented by the Formula [1]: 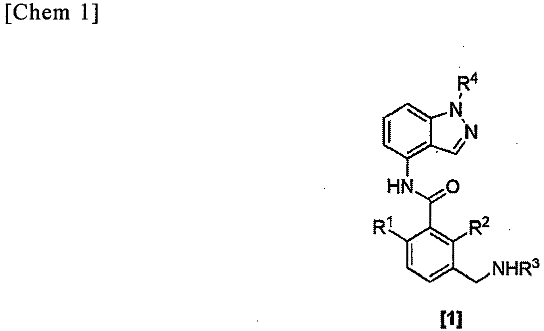 wherein,
R¹ and R² each independently represent hydrogen, halogen, or optionally substituted alkyl;
R³ represents -SO₂R⁵ or -COR⁵
R⁴ represents optionally substituted alkyl, optionally substituted cycloalkyl, optionally substituted aryl, optionally substituted heteroaryl, optionally substituted saturated heterocyclic group, or optionally substituted arylalkyl; and
R⁵ represents optionally substituted alkyl, optionally substituted cycloalkyl, optionally substituted aryl, optionally substituted heteroaryl, optionally substituted saturated heterocyclic group, optionally substituted alkynyl, or optionally substituted arylalkyl;
wherein said heteroaryl or saturated heterocyclic groups is bound at a carbon atom on the ring, or a pharmaceutically acceptable salt thereof, or a solvate thereof.

2. The compound according to claim 1,
wherein
the alkyl in R¹ and R² is optionally substituted by 1 to 3 halogens;
the heteroaryl in R⁴ is optionally substituted by 1 to 3 groups independently selected from the group consisting of:
(1) alkoxy optionally substituted by 1 to 3 halogens,
(2) cyano,
(3) halogen,
(4) alkyl optionally substituted by (i) 1 to 3 halogens, (ii) hydroxy or (iii) alkoxy, and
(5) cycloalkyl;
the saturated heterocyclic group in R⁴ is optionally substituted by 1 to 3 groups independently selected from the group consisting of alkyl, alkoxycarbonyl and alkylcarbonyl;
the alkyl in R⁴ is optionally substituted by a saturated heterocyclic group or cycloalkyl;
the aryl in R⁴ is optionally substituted by 1 to 3 groups independently selected from the group consisting of:
(1) alkoxy optionally substituted by 1 to 3 halogens,
(2) cyano,
(3) halogen,
(4) alkyl optionally substituted by (i) 1 to 3 halogens, (ii) hydroxy or (iii) alkoxy, and
(5) cycloalkyl;
the arylalkyl in R⁴ is optionally substituted by alkyl or alkoxy optionally substituted by 1 to 3 halogens;
the saturated heterocyclic group in R⁵ is optionally substituted by 1 to 3 groups independently selected from the group consisting of alkyl and alkoxycarbonyl;
the alkyl in R⁵ is optionally substituted by 1 to 3 groups independently selected from the group consisting of hydroxy, alkoxy, cycloalkyl, monoalkylamino, dialkylamino and halogen; and
the cycloalkyl in R⁵ is optionally substituted by 1 to 3 groups independently selected from the group consisting of:
(1) hydroxy,
(2) cyano, and
(3) alkyl optionally substituted by 1 to 3 halogens,
or a pharmaceutically acceptable salt thereof, or a solvate thereof.

3. The compound according to claim 1 or 2,
wherein
the aryl in R⁴ is phenyl optionally substituted by 1 to 3 groups independently selected from the group consisting of:
(1) alkoxy which is optionally substituted by 1 to 3 halogens,
(2) cyano,
(3) halogen,
(4) alkyl optionally substituted by (i) 1 to 3 halogens, (ii) hydroxy or (iii) alkoxy, and
(5) cycloalkyl,
the heteroaryl in R⁴ is imidazolyl, thiazolyl, pyridyl, pyridazinyl or pyrimidinyl, each of which is optionally substituted by 1 to 3 groups independently selected from the group consisting of:
(1) alkoxy which is optionally substituted by 1 to 3 halogens,
(2) cyano,
(3) halogen,
(4) alkyl optionally substituted by (i) 1 to 3 halogens, (ii) hydroxy, or (iii) alkoxy, and
(5) cycloalkyl; and
the saturated heterocyclic group in R⁴ is piperidinyl or tetrahydropyranyl optionally substituted by alkyl, alkoxycarbonyl or alkylcarbonyl,
or a pharmaceutically acceptable salt thereof, or a solvate thereof.

4. The compound according to any one of claims 1-3,
wherein
the aryl in R⁵ is phenyl;
the heteroaryl in R⁵ is furyl; and
the saturated heterocyclic group in R⁵ is piperidinyl, tetrahydrofuryl or tetrahydropyranyl, each of which is optionally substituted by 1 to 3 groups independently selected from the group consisting of alkyl and alkoxycarbonyl,
or a pharmaceutically acceptable salt thereof, or a solvate thereof.

5. The compound according to any one of claims 1-4, wherein R⁴ is alkyl having 1 to 6 carbons optionally substituted by a saturated heterocyclic group or cycloalkyl, or a pharmaceutically acceptable salt, or a solvate thereof.

6. The compound according to any one of claims 1-4, wherein R⁴ is cycloalkyl having 3 to 10 carbons, or a pharmaceutically acceptable salt thereof, or a solvate thereof.

7. The compound according to any one of claims 1-4,
wherein
R⁴ is phenyl optionally substituted by 1 to 3 groups independently selected from the group consisting of:
(1) alkoxy optionally substituted by 1 to 3 halogens,
(2) cyano,
(3) halogen,
(4) alkyl optionally substituted by (i) 1 to 3 halogens, (ii) hydroxy or (iii) alkoxy, and
(5) cycloalkyl,
or a pharmaceutically acceptable salt thereof, or a solvate thereof.

8. The compound according to any one of claims 1-4,
wherein
R⁴ is imidazolyl, thiazolyl, pyridyl, pyridazinyl or pyrimidinyl, each of which is optionally substituted by 1 to 3 groups independently selected from the group consisting of:
(1) alkoxy optionally substituted by 1 to 3 halogens,
(2) cyano,
(3) halogen,
(4) alkyl optionally substituted by (i) 1 to 3 halogens, (ii) hydroxy or (iii) alkoxy, and
(5) cycloalkyl,
or a pharmaceutically acceptable salt thereof, or a solvate thereof.

9. The compound according to any one of claims 1-4, wherein R⁴ is piperidinyl or tetrahydropyranyl, each of which is optionally substituted by 1 to 3 groups independently selected from the group consisting of alkyl, alkoxycarbonyl and alkylcarbonyl, or a pharmaceutically acceptable salt thereof, or a solvate thereof.

10. The compound of any one of claims 1-4, wherein R⁴ is phenylalkyl, which is optionally substituted by alkyl or alkoxy of 1 to 6 carbons, each of which is optionally substituted by 1 to 3 halogens, or a pharmaceutically acceptable salt thereof, or a solvate thereof.

11. The compound of any one of claims 1-10, wherein R⁵ is alkyl having 1-6 carbons, which is optionally substituted by 1 to 3 groups independently selected from the group consisting of hydroxy, alkoxy, cycloalkyl, monoalkylamino, dialkylamino, and halogen, or a pharmaceutically acceptable salt thereof, or a solvate thereof.

12. The compound according to any one of claims 1-10, wherein R⁵ is cycloalkyl having 3 to 10 carbons optionally substituted by 1 to 3 groups independently selected from the group consisting of (1) hydroxy, (2) cyano and (3) alkyl optionally substituted by 1 to 3 halogens, or a pharmaceutically acceptable salt thereof, or a solvate thereof.

13. The compound according to any one of claims 1-10, wherein R⁵ is optionally substituted phenyl, or a pharmaceutically acceptable salt thereof, or a solvate thereof.

14. The compound according to any one of claims 1-10, wherein R⁵ is optionally substituted furyl, or a pharmaceutically acceptable salt thereof, or a solvate thereof.

15. The compound according to any one of claims 1-10, wherein R⁵ is piperidinyl, tetrahydrofuryl or tetrahydropyranyl, each of which is optionally substituted by 1 to 3 groups independently selected from the group consisting of alkyl and alkoxycarbonyl, or a pharmaceutically acceptable salt thereof, or a solvate thereof.

16. The compound according to any one of claims 1-10, wherein R⁵ is optionally substituted alkynyl having 2 to 6 carbons, or a pharmaceutically acceptable salt thereof, or a solvate thereof.

17. The compound of any one of claims 1-10, wherein R⁵ is optionally substituted phenylalkyl, or a pharmaceutically acceptable salt thereof, or a solvate thereof.

18. The compound according to any one of claims 1-17, wherein R¹ is halogen or alkyl having 1 to 6 carbons optionally substituted by 1 to 3 halogens, or a pharmaceutically acceptable salt thereof, or a solvate thereof.

19. The compound according to any one of claims 1-18, wherein R² is hydrogen or halogen, or a pharmaceutically acceptable salt thereof, or a solvate thereof.

20. The compound of claim 1 selected from the group consisting of the following compounds:
(1) 2-Chloro-5-{[(cyclopropylcarbonyl)amino]methyl)-N-{1-[4-(trifluoromethyl)phenyl]-1H-indazol-4-yl}benzamide,
(2) 2-Chloro-5-{[(cyclopropylcarbonyl)amino]methyl}-N-(1-phenyl-1H-indazol-4-yl)benzamide,
(3) 2-Chloro-5- {[(cyclopropylcarbonyl)amino]methyl} -N- {1 -[3-(trifluoromethyl)phenyl]- 1H-indazol-4-yl} benzamide,
(4) 2-Chloro-5-{[(cyclopropylcarbonyl)amino]methyl}-N-[1-(pyridin-3-yl)-1H-indazol-4-yl]benzamide,
(5) 2-Chloro-5-{[(cyclopropylcarbonyl)amino]methyl}-N-[1-(pyridin-4-yl)-1H-indazol-4-yl]benzamide,
(6) 2-Chloro-5-{[(cyclopropylcarbonyl)amino]methyl}-N-[1-(4-methylphenyl)-1H-indazol-4-yl]benzamide,
(7) 2-Chloro-5-{[(cyclopropylcarbonyl)amino]methyl}-N-[1-(3-methylphenyl)-1H-indazol-4-yl]benzamide,
(8) 2-Chloro-5-{[(cyclopropylcarbonyl)amino]methyl}-N-[1-(pyridin-2-yl)-1H-indazol-4-yl]benzamide,
(9) 2-Chloro-5-{[(cyclopropylcarbonyl)amino]methyl}-N-[1-(2-methylphenyl)-1H-indazol-4-yl]benzamide,
(10) 2-Chloro-5-{[(cyclopropylcarbonyl)amino]methyl}-N-[1-(6-methylpyridin-3-yl)-1H-indazol-4-yl]benzamide,
(11) 2-Chloro-5-{[(cyclopropylcarbonyl)amino]methyl}-N-{1-[5-(trifluoromethyl)pyridin-3-yl]-1H-indazol-4-yl}benzamide,
(12) 2-Chloro-5-{[(cyclopropylcarbonyl)amino]methyl}-N-{1-[6-(trifluoromethyl)pyridin-3-yl]-1H-indazol-4-yl}benzamide,
(13) 2-Chloro-5-{[(cyclopropylcarbonyl)amino]methyl}-N-[1-(2-methoxyphenyl)-1H-indazol-4-yl]benzamide,
(14) N-[1-(4-tert-butylphenyl)-1H-indazol-4-yl]-2-chloro-5-{[(cyclopropylcarbonyl)amino]-methyl} benzamide,
(15) 2-Chloro-N-[1-(3-chloro-2-methylphenyl)-1H-indazol-4-yl]-5-{[(cyclopropylcarbonyl)-amino]methyl} benzamide,
(16) 3-{[(Cyclopropylcarbonyl)amino]methyl}-2,6-difluoro-N-{1-[3-(trifluoromethyl)phenyl]-1H-indazol-4-yl}benzamide,
(17) 2-Chloro-5-{[(trifluoroacetyl)amino]methyl}-N-{1-[4-(trifluoromethyl)phenyl]-1H-indazol-4-yl}benzamide,
(18) 5-[(Acetylamino)methyl]-2-chloro-N-{1-[4-(trifluoromethyl)phenyl]-1H-indazol-4-yl}benzamide,
(19) 2-Chloro-5-[(propanoylamino)methyl]-N-{1-[4-(trifluoromethyl)phenyl]-1H-indazol-4-yl}benzamide,
(20) 2-Chloro-5-{[(3,3-dimethylbutanoyl)amino]methyl}-N-{1-[4-(trifluoromethyl)phenyl]-1H-indazol-4-yl} benzamide,
(21) 2-Chloro-5-{[(methoxyacetyl)amino]methyl}-N-{1-[4-(trifluoromethyl)phenyl]-1H-indazol-4-yl}benzamide,
(22) 2-Chloro-5-{[(phenylcarbonyl)amino]methyl}-N-{1-[4-(trifluoromethyl)phenyl]-1H-indazol-4-yl}benzamide,
(23) 2-Chloro-5-{[(cyclopropylsulfonyl)amino]methyl}-N-{1-[4-(trifluoromethyl)phenyl]-1H-indazol-4-yl}benzamide,
(24) 2-Chloro-5-{[(N,N-dimethylglycyl)amino]methyl}-N-{1-[4-(trifluoromethyl)phenyl]-1H-indazol-4-yl} benzamide,
(25) 2-Chloro-5-({[(1-cyanocyclopropyl)carbonyl]amino}methyl)-N-{1-[4-(trifluoromethyl)-phenyl]-1H-indazol-4-yl}benzamide,
(26) 2-Chloro-5-[({[1-(trifluoromethyl)cyclopropyl]carbonyl)amino)methyl]-N-{1-[4-(trifluoromethyl)phenyl]-1H-indazol-4-yl}benzamide,
(27) 2-Chloro-5-({[(1-hydroxycyclopropyl)carbonyl]amino}methyl)-N-{1-[4-(trifluoromethyl)-phenyl]-1H-indazol-4-yl}benzamide,
(28) 2-Chloro-5-{[(3-hydroxy-2,2-dimethylpropanoyl)amino]methyl}-N-{1-[4-(trifluoromethyl)-phenyl]-1H-indazol-4-yl}benzamide,
(29) 2-Chloro-5-{[(cyclobutylcarbonyl)amino]methyl}-N-{1-[4-(trifluoromethyl)phenyl]-1H-indazol-4-yl}benzamide,
(30) 2-Chloro-5-{[(cyclopentylcarbonyl)amino]methyl}-N-{1-[4-(trifluoromethyl)phenyl]-1H-indazol-4-yl}benzamide,
(31) 2-Chloro-5-{[(cyclohexylcarbonyl)amino]methyl}-N-{ 1-[4-(trifluoromethyl)phenyl]-1H-indazol-4-yl}benzamide,
(32) N-[4-chloro-3-({1-[4-(trifluoromethyl)phenyl]-1H-indazol-4-yl}carbamoyl)benzyl]tricyclo[3.3.1.^{13,7}]decane-1-carboxamide,
(33) N-[4-chloro-3-({ 1-[4-(trifluoromethyl)phenyl]-1H-indazol-4-yl}carbamoyl)benzyl]tetrahydrofuran-2-carboxamide,
(34) N-[4-chloro-3-({1-[4-(trifluoromethyl)phenyl]-1H-indazol-4-yl}carbamoyl)benzyl]tetrahydrofuran-3-carboxamide,
(35) 2-Chloro-5-{[(N,N-dimethyl-β-alanyl)amino]methyl}-N-{ 1-[4-(trifluoromethyl)phenyl]-1H-indazol-4-yl} benzamide,
(36) tert-Butyl 4-{[4-chloro-3-({ 1-[4-(trifluoromethyl)phenyl]-1H-indazol-4-yl}carbamoyl)benzyl]carbamoyl}piperidine-1-carboxylate,
(37) 2-Chloro-N-{1-[4-(trifluoromethyl)phenyl]-1H-indazol-4-yl)-5-1[(3,3,3-trifluoropropanoyl)amino]methyl}benzamide,
(38) 2-Chloro-5-{[(cyclopropylacetyl)amino]methyl)-N-{1-[4-(trifluoromethyl)phenyl]-1H-indazol-4-yl} benzamide,
(39) 2-Chloro-5-({[trans-(4-hydroxycyclohexyl)carbonyl]amino}methyl)-N-{1-[4-(trifluoromethyl)phenyl]-1H-indazol-4-yl}benzamide,
(40) 2-Chloro-5-({[cis-(4-hydroxycyclohexyl)carbonyl]amino}methyl)-N-{1-[4-(trifluoromethyl)phenyl]-1H-indazol-4-yl}benzamide,
(41) 2-Chloro-5-{[(2-ethyl-2-hydroxybutanoyl)amino]methyl}-N-{ 1-[4-(trifluoromethyl)phenyl]-1H-indazol-4-yl}benzamide,
(42) 5-[(2-Butynoylamino)methyl]-2-chloro-N-{ 1-[4-(trifluoromethyl)phenyl]-1H-indazol-4-yl}benzamide,
(43) 5-[(Butanoylamino)methyl]-2-chloro-N-{1-[4-(trifluoromethyl)phenyl]-1H-indazol-4-yl}benzamide,
(44) 2-Chloro-5-{[(phenylacetyl)amino]methyl)-N-{1-[4-(trifluoromethyl)phenyl]-1H-indazol-4-yl}benzamide,
(45) N-[4-chloro-3-({1-[4-(trifluoromethyl)phenyl]-1H-indazol-4-yl}carbamoyl)benzyl]furan-2-carboxamide,
(46) 2-Chloro-5-{[(cyclopentylacetyl)amino]methyl}-N-{1-[4-(trifluoromethyl)phenyl]-1H-indazol-4-yl}benzamide,
(47) 2-Chloro-5-{[(3-methoxypropanoyl)amino]methyl}-N-{1-[4-(trifluoromethyl)phenyl]-1H-indazol-4-yl}benzamide,
(48) 2-Chloro-5-{[(2,2-dimethylpropanoyl)amino]methyl}-N-[1-(4-methoxyphenyl)-1H-indazol-4-yl]benzamide,
(49) 2-Chloro-5-{[(2,2-dimethylpropanoyl)amino]methyl}-N-[1-(3-methoxyphenyl)-1H-indazol-4-yl]benzamide,
(50) 2-Chloro-5-{[(2,2-dimethylpropanoyl)amino]methyl}-N-{1-[4-(trifluoromethyl)phenyl]-1H-indazol-4-yl}benzamide
(51) 2-Chloro-5-{[(2,2-dimethylpropanoyl)amino]methyl}-N-11-[4-(trifluoromethoxy)phenyl]-1H-indazol-4-yl}benzamide
(52) 2-Chloro-5-{[(2,2-dimethylpropanoyl)amino]methyl}-N-[1-(2-methylpyrimidin-5-yl)-1H-indazol-4-yl]benzamide,
(53) 2-Chloro-N-[1-(3-chloro-4-methylphenyl)-1H-indazol-4-yl]-5-([(2,2-dimethylpropanoyl)amino]methyl)benzamide,
(54) 2-Chloro-5-{[(2,2-dimethylpropanoyl)amino]methyl}-N-[1-(6-methylpyridin-3-yl)-1H-indazol-4-yl]benzamide,
(55) 2-Chloro-N-[1-(4-cyanophenyl)-1H-indazol-4-yl]-5-([(2,2-dimethylpropanoyl)amino]methyl)benzamide,
(56) 2-Chloro-5-{[(2,2-dimethylpropanoyl)amino]methyl}-N-[1-(2-methylpyridin-3-yl)-1H-indazol-4-yl]benzamide,
(57) 2-Chloro-5-{[(2,2-dimethylpropanoyl)amino]methyl}-N-{1-[5-(trifluoromethyl)pyridin-3-yl]-1H-indazol-4-yl}benzamide,
(58) 2-Chloro-5-{[(2,2-dimethylpropanoyl)amino]methyl}-N-[1-(2-methylpyridin-4-yl)-1H-indazol-4-yl]benzamide,
(59) 2-Chloro-5-{[(2,2-dimethylpropanoyl)amino]methyl}-N-[1-(6-methylpyridazin-3-yl)-1H-indazol-4-yl]benzamide,
(60) 2-Chloro-5-{[(2,2-dimethylpropanoyl)amino]methyl}-N-[1-(5-methylpyridin-3-yl)-1H-indazol-4-yl]benzamide,
(61) 2-Chloro-5-{[(2,2-dimethylpropanoyl)amino]methyl}-N-[1-(5-methoxypyridin-3-yl)-1H-indazol-4-yl]benzamide,
(62) 2-Chloro-5-{[(2,2-dimethylpropanoyl)amino]methyl}-N-{1-[4-methoxy-3-(trifluoromethyl)phenyl]-1H-indazol-4-yl}benzamide,
(63) 2-Chloro-5-{[(2,2-dimethylpropanoyl)amino]methyl}-N-[1-(6-methoxypyridin-3-yl)-1H-indazol-4-yl]benzamide,
(64) 2-Chloro-N-[1-(5-cyclopropylpyridin-3-yl)-1H-indazol-4-yl]-5-{[(2,2-dimethylpropanoyl)amino]methyl} benzamide,
(65) 2-Chloro-5-{[(2,2-dimethylpropanoyl)amino]methyl}-N-[1-(4-ethoxyphenyl)-1H-indazol-4-yl]benzamide,
(66) 2-Chloro-5-{[(2,2-dimethylpropanoyl)amino]methyl}-N-[1-(3-ethoxyphenyl)-1H-indazol-4-yl]benzamide,
(67) 2-Chloro-N-[1-(3,4-dimethoxyphenyl)-1H-indazol-4-yl]-5-{[(2,2-dimethylpropanoyl)amino]methyl}benzamide,
(68) 2-Chloro-5-{[(2,2-dimethylpropanoyl)amino]methyl}-N-{1-[3-(propan-2-yloxy)phenyl]-1H-indazol-4-yl} benzamide,
(69) 2-Chloro-N-[1-(6-cyclopropylpyridin-3-yl)-1H-indazol-4-yl]-5-{[(2,2-dimethylpropanoyl)amino]methyl} benzamide,
(70) 2-Chloro-N-[1-(2-cyclopropylpyridin-4-yl)-1H-indazol-4-yl]-5-{[(2,2-dimethylpropanoyl)amino]methyl} benzamide,
(71) 2-Chloro-5-{[(2,2-dimethylpropanoyl)amino]methyl}-N-{1-[3-(trifluoromethoxy)phenyl]-1H-indazol-4-yl}benzamide,
(72) 2-Chloro-N-[1-(6-cyanopyridin-3-yl)-1H-indazol-4-yl]-5-([(2,2-dimethylpropanoyl)amino]methyl)benzamide,
(73) 2-Chloro-5-{[(2,2-dimethylpropanoyl)amino]methyl}-N-[1-(2-methoxypyridin-4-yl)-1H-indazol-4-yl]benzamide,
(74) 2-Chloro-5-{[(2,2-dimethylpropanoyl)amino]methyll-N-11-[6-(methoxymethyl)pyridin-3-yl]-1H-indazol-4-yl}benzamide,
(75) 2-Chloro-5-{[(2,2-dimethylpropanoyl)amino]methyl}-N-{1-[6-(hydroxymethyl)pyridin-3-yl]-1H-indazol-4-yl}benzamide,
(76) 2-Chloro-5-{[(2,2-dimethylpropanoyl)amino]methyl}-N-[1-(2,6-dimethylpyridin-4-yl)-1H-indazol-4-yl]benzamide,
(77) 2-Chloro-5-{[(2,2-dimethylpropanoyl)amino]methyl}-N-{1-[6-(trifluoromethyl)pyridin-3-yl]-1H-indazol-4-yl}benzamide,
(78) 2-Chloro-5-{[(2,2-dimethylpropanoyl)amino]methyl}-N-{1-[5-(methoxymethyl)pyridin-3-yl]-1H-indazol-4-yl}benzamide,
(79) 2-Chloro-5-{[(2,2-dimethylpropanoyl)amino]methyl}-N-{1-[2-methyl-4-(trifluoromethoxy)phenyl]-1H-indazol-4-yl}benzamide,
(80) 2-Chloro-5-{[(2,2-dimethylpropanoyl)amino]methyl}-N-[1-(1,3-thiazol-2-yl)-1H-indazol-4-yl]benzamide,
(81) 2-Chloro-N-{1-[3-(difluoromethoxy)phenyl]-1H-indazol-4-yl}-5-{[(2,2-dimethylpropanoyl)amino]methyl}benzamide,
(82) 2-Chloro-5-{[(2,2-dimethylpropanoyl)amino]methyl}-N-{1-[3-methyl-4-(trifluoromethoxy)phenyl]-1H-indazol-4-yl}benzamide,
(83) 2-Chloro-5-{[(2,2-dimethylpropanoyl)amino]methyl}-N-[1-(1,3-thiazol-4-yl)-1H-indazol-4-yl]benzamide,
(84) 2-Chloro-5-{[(2,2-dimethylpropanoyl)amino]methyl}-N-{1-[6-(2-methoxypropan-2-yl)pyridin-3-yl]-1H-indazol-4-yl}benzamide,
(85) 2-Chloro-5-{[(2,2-dimethylpropanoyl)amino]methyl}-N-{1-[6-(2-fluoropropan-2-yl)pyridin-3-yl]-1H-indazol-4-yl}benzamide,
(86) 2-Chloro-5-{[(2,2-dimethylpropanoyl)amino]methyl}-N-{1-[6-(ethoxymethyl)pyridin-3-yl]-1H-indazol-4-yl}benzamide,
(87) 2-Chloro-N-[1-(6-methylpyridin-3-yl)-1H-indazol-4-yl]-5-{[(trifluoroacetyl)amino]methyl}benzamide,
(88) 5-{[(2,2-Dimethylpropanoyl)amino]methyl}-N-[1-(6-methylpyridin-3-yl)-1H-indazol-4-yl]-2-(trifluoromethyl)benzamide,
(89) 2-Chloro-5-{[(2,2-dimethylpropanoyl)amino]methyl}-N-[1-(3-methoxybenzyl)-1H-indazol-4-yl]benzamide,
(90) 2-Chloro-5-{[(2,2-dimethylpropanoyl)amino]methyl}-N-[1-(2-methoxybenzyl)-1H-indazol-4-yl]benzamide,
(91) 2-Chloro-5-{[(2,2-dimethylpropanoyl)amino]methyl}-N-[1-(2,4,6-trimethylbenzyl)-1H-indazol-4-yl]benzamide,
(92) 2-Chloro-5-{[(2,2-dimethylpropanoyl)amino]methyl}-N-{1-[4-(trifluoromethyl)benzyl]-1H-indazol-4-yl}benzamide,
(94) 2-Chloro-5-{[(2,2-dimethylpropanoyl)amino]methyl}-N- f 1-[3-(trifluoromethyl)benzyl]-1H-indazol-4-yl}benzamide,
(95) 2-Chloro-5-{[(2,2-dimethylpropanoyl)amino]methyl}-N-[1-(2-phenylethyl)-1H-indazol-4-yl]benzamide,
(97) 2-Chloro-N-(1-cyclopentyl-1H-indazol-4-yl)-5-{[(2,2-dimethylpropanoyl)amino]methyl} benzamide,
(98) 2-Chloro-N-(1-cyclohexyl-1H-indazol-4-yl)-5-{[(2,2-dimethylpropanoyl)amino]methyl}benzamide,
(99) 2-Chloro-N-[1-(cyclobutylmethyl)-1H-indazol-4-yl]-5-{[(2,2-dimethylpropanoyl)amino]methyl}benzamide,
(100) 2-Chloro-N-[1-(cyclohexylmethyl)-1H-indazol-4-yl]-5-{[(2,2-dimethylpropanoyl)amino]methyl}benzamide,
(101) 2-Chloro-5-{[(2,2-dimethylpropanoyl)amino]methyl}-N-[1-(3-phenylpropyl)-1H-indazol-4-yl]benzamide,
(102) 2-Chloro-5-{[(2,2-dimethylpropanoyl)amino]methyl}-N-[1-(propan-2-yl)-1H-indazol-4-yl]benzamide,
(103) tert-Butyl 4-(4-{[(2-chloro-5-{[(2,2-dimethylpropanoyl)amino]methyl}phenyl)carbonyl]amino}-1H-indazol-1-yl)piperidine-1-carboxylate,
(104) 2-Chloro-5-{[(2,2-dimethylpropanoyl)amino]methyl}-N-[1-(tetrahydro-2H-pyran-4-yl)-1H-indazol-4-yl]benzamide,
(105) 2-Chloro-5-{[(2,2-dimethylpropanoyl)amino]methyl}-N-[1-(tetrahydro-2H-pyran-2-ylmethyl)-1H-indazol-4-yl]benzamide,
(106) N-[1-(1-acetylpiperidin-4-yl)-1H-indazol-4-yl]-2-chloro-5-{[(2,2-dimethylpropanoyl)amino]methyl} benzamide,
(107) 2-Chloro-5-{[(2,2-dimethylpropanoyl)amino]methyl}-N-{1-[2-(morpholin-4-yl)ethyl]-1H-indazol-4-yl} benzamide,
(108) 2-Chloro-5-{[(2,2-dimethylpropanoyl)amino]methyl}-N-[1-(4-phenylbutyl)-1H-indazol-4-yl]benzamide,
(109) N-(1-benzyl-1H-indazol-4-yl)-2-chloro-5-{[(cyclopropylcarbonyl)amino]methyl} benzamide,
(111) 2-Chloro-5-{[(2,2-dimethylpropanoyl)amino]methyl}-N-(1-methyl-1H-indazol-4-yl)benzamide,
(112) 2-Chloro-5-{[(cyclopropylcarbonyl)amino]methyl}-N-(1-methyl-1H-indazol-4-yl)benzamide,
(113) 2-Chloro-5-{[(2,2-dimethylpropanoyl)amino]methyl}-N-(1-ethyl-1H-indazol-4-yl)benzamide,
(114) 2-Chloro-5-{[(3,3-dimethylbutanoyl)amino]methyl}-N-{1-[4-(trifluoromethoxy)phenyl]-1H-indazol-4-yl}benzamide,
(115) 2-Chloro-5-{[(cyclopentylcarbonyl)amino]methyl}-N-{1-[4-(trifluoromethoxy)phenyl]-1H-indazol-4-yl}benzamide,
(116) 2-Chloro-N-{1-[4-(trifluoromethoxy)phenyl]-1H-indazol-4-yl}-5-[({[1-(trifluoromethyl)cyclopropyl]carbonyl}amino)methyl]benzamide,
(117) 2-Chloro-5-({[(1-hydroxycyclopropyl)carbonyl]amino}methyl)-N-{1-[4-(trifluoromethoxy)phenyl]-1H-indazol-4-yl}benzamide,
(118) 2-Chloro-5-{[(methoxyacetyl)amino]methyl}-N-{1-[4-(trifluoromethoxy)phenyl]-1H-indazol-4-yl} benzamide,
(119) 2-Chloro-5-{[(3-hydroxy-2,2-dimethylpropanoyl)amino]methyl}-N-{1-[4-(trifluoromethoxy)phenyl]-1H-indazol-4-yl}benzamide,
(120) 2-Chloro-5-{[(cyclopropylsulfonyl)amino]methyl}-N-{1-[4-(trifluoromethoxy)phenyl]-1H-indazol-4-yl} benzamide,
(121) 2-Chloro-5-1[(3,3-dimethylbutanoyl)amino]methyl)-N-{1-[3-(trifluoromethoxy)phenyl]-1H-indazol-4-yl}benzamide,
(122) 2-Chloro-5-{[(cyclopentylcarbonyl)amino]methyl}-N-{1-[3-(trifluoromethoxy)phenyl]-1H-indazol-4-yl} benzamide,
(123) 2-Chloro-N-{1-[3-(trifluoromethoxy)phenyl]-1H-indazol-4-yl}-5-[({[1-(trifluoromethyl)cyclopropyl]carbonyl}amino)methyl]benzamide,
(124) 2-Chloro-5-{[(methoxyacetyl)amino]methyl}-N-{1-[3-(trifluoromethoxy)phenyl]-1H-indazol-4-yl}benzamide,
(125) 2-Chloro-5-{[(3-hydroxy-2,2-dimethylpropanoyl)amino]methyl}-N-{1-[3-(trifluoromethoxy)phenyl]-1H-indazol-4-yl}benzamide,
(126) 2-Chloro-5-{[(cyclopropylsulfonyl)amino]methyl}-N-{1-[3-(trifluoromethoxy)phenyl]-1H-indazol-4-yl}benzamide,
(127) 2-Chloro-N-[1-(3-chloro-4-methylphenyl)-1H-indazol-4-yl]-5-{[(3,3-dimethylbutanoyl)amino]methyl}benzamide,
(128) 2-Chloro-N-[1-(3-chloro-4-methylphenyl)-1H-indazol-4-yl]-5-{[(cyclopentylcarbonyl)amino]methyl}benzamide,
(129) 2-Chloro-N-[1-(3-chloro-4-methylphenyl)-1H-indazol-4-yl]-5-[({[1-(trifluoromethyl)cyclopropyl]carbonyl} amino)methyl]benzamide,
(130) 2-Chloro-N-[1-(3-chloro-4-methylphenyl)-1H-indazol-4-yl]-5-({[(1-hydroxycyclopropyl)carbonyl]amino}methyl)benzamide,
(131) 2-Chloro-N-[1-(3-chloro-4-methylphenyl)-1H-indazol-4-yl]-5-{[(methoxyacetyl)amino]methyl} benzamide,
(132) 2-Chloro-N-[1-(3-chloro-4-methylphenyl)-1H-indazol-4-yl]-5-{[(cyclopropylsulfonyl)amino]methyl}benzamide,
(133) 2-Chloro-5-{[(3,3-dimethylbutanoyl)amino]methyl}-N-{1-[3-methyl-4-(trifluoromethoxy)phenyl]-1H-indazol-4-yl}benzamide,
(134) 2-Chloro-5-{[(cyclopentylcarbonyl)amino]methyl}-N-{1-[3-methyl-4-(trifluoromethoxy)phenyl]-1H-indazol-4-yl}benzamide,
(135) 2-Chloro-5-{[(methoxyacetyl)amino]methyl}-N-{1-[3-methyl-4-(trifluoromethoxy)phenyl]-1H-indazol-4-yl}benzamide,
(136) 2-Chloro-5-{[(3-hydroxy-2,2-dimethylpropanoyl)amino]methyl}-N-{1-[3-methyl-4-(trifluoromethoxy)phenyl]-1H-indazol-4-yl}benzamide,
(137) 2-Chloro-5-{[(cyclopropylsulfonyl)amino]methyl}-N-{1-[3-methyl-4-(trifluoromethoxy)phenyl]-1H-indazol-4-yl}benzamide,
(138) 2-Chloro-N-{1-[3-(difluoromethoxy)phenyl]-1H-indazol-4-yl}-5-{[(3,3-dimethylbutanoyl)amino]methyl} benzamide,
(139) 2-Chloro-5-{[(cyclopentylcarbonyl)amino]methyl}-N-{1-[3-(difluoromethoxy)phenyl]-1H-indazol-4-yl} benzamide,
(140) 2-Chloro-N-{1-[3-(difluoromethoxy)phenyl]-1H-indazol-4-yl}-5-[({[1-(trifluoromethyl)cyclopropyl]carbonyl}amino)methyl]benzamide,
(141) 2-Chloro-N-{1-[3-(difluoromethoxy)phenyl]-1H-indazol-4-yl}-5-({[(1-hydroxycyclopropyl)carbonyl]amino}methyl)benzamide,
(142) 2-Chloro-N-{1-[3-(difluoromethoxy)phenyl]-1H-indazol-4-yl}-5-{[(methoxyacetyl)amino]methyl} benzamide,
(143) 2-Chloro-N-{1-[3-(difluoromethoxy)phenyl]-1H-indazol-4-yl}-5-1[(3-hydroxy-2,2-dimethylpropanoyl)amino]methyl}benzamide,
(144) 2-Chloro-5-{[(cyclopropylsulfonyl)amino]methyl}-N-{1-[3-(difluoromethoxy)phenyl]-1H-indazol-4-yl} benzamide
(145) 2-Chloro-N-[1-(4-chlorophenyl)-1H-indazol-4-yl]-5-{[(3,3-dimethylbutanoyl)amino]methyl} benzamide
(146) 2-Chloro-N-[1-(4-chlorophenyl)-1H-indazol-4-yl]-5-{[(cyclopentylcarbonyl)amino]methyl} benzamide,
(147) 2-Chloro-N-[1-(4-chlorophenyl)-1H-indazol-4-yl]-5-[({[1-(trifluoromethyl)cyclopropyl]carbonyl}amino)methyl]benzamide,
(148) 2-Chloro-N-[1-(4-chlorophenyl)-1H-indazol-4-yl]-5-{[(methoxyacetyl)amino]methyl}benzamide,
(149) 2-Chloro-N-[1-(4-chlorophenyl)-1H-indazol-4-yl]-5-{[(3-hydroxy-2,2-dimethylpropanoyl)amino]methyl} benzamide,
(150) 2-Chloro-N-[1-(4-chlorophenyl)-1H-indazol-4-yl]-5-([(cyclopropylsulfonyl)amino]methyl)benzamide,
(151) 2-Chloro-5-{[(3,3-dimethylbutanoyl)amino]methyl}-N-[1-(4-fluorophenyl)-1H-indazol-4-yl]benzamide,
(152) 2-Chloro-5-{[(cyclopentylcarbonyl)amino]methyl}-N-[1-(4- fluorophenyl)-1H-indazol-4-yl]benzamide,
(153) 2-Chloro-N-[1-(4-fluorophenyl)-1H-indazol-4-yl]-5-[({[1-(trifluoromethyl)cyclopropyl]carbonyl}amino)methyl]benzamide,
(154) 2-Chloro-N-[1-(4-fluorophenyl)-1H-indazol-4-yl]-5-({[(1-hydroxycyclopropyl)carbonyl]amino}methyl)benzamide,
(155) 2-Chloro-N-[1-(4-fluorophenyl)-1H-indazol-4-yl]-5-([(methoxyacetyl)amino]methyl)benzamide,
(156) 2-Chloro-N-[1-(4-fluorophenyl)-1H-indazol-4-yl]-5-{[(3-hydroxy-2,2-dimethylpropanoyl)amino]methyl}benzamide,
(157) 2-Chloro-5-{[(cyclopropylsulfonyl)amino]methyl}-N-[1-(4-fluorophenyl)-1H-indazol-4-yl]benzamide,
(158) 2,6-Dichloro-3-{[(2,2-dimethylpropanoyl)amino]methyl}-N-[1-(4-fluorophenyl)-1H-indazol-4-yl]benzamide,
(159) 2-Chloro-5-{[(3,3-dimethylbutanoyl)amino]methyl}-N-[1-(2-methoxypyridin-4-yl)-1H-indazol-4-yl]benzamide,
(160) 2-Chloro-5-{[(cyclopentylcarbonyl)amino]methyl}-N-[1-(2-methoxypyridin-4-yl)-1H-indazol-4-yl]benzamide,
(161) 2-Chloro-N-[1-(2-methoxypyridin-4-yl)-1H-indazol-4-yl]-5-[({[1-(trifluoromethyl)cyclopropyl]carbonyl}amino)methyl]benzamide,
(162) 2-Chloro-5-({[(1-hydroxycyclopropyl)carbonyl]amino}methyl)-N-[1-(2-methoxypyridin-4-yl)-1H-indazol-4-yl]benzamide,
(163) 2-Chloro-5-{[(methoxyacetyl)amino]methyl}-N-[1-(2-methoxypyridin-4-yl)-1H-indazol-4-yl]benzamide,
(164) 2-Chloro-5-{[(3-hydroxy-2,2-dimethylpropanoyl)amino]methyl}-N-[1-(2-methoxypyridin-4-yl)-1H-indazol-4-yl]benzamide,
(165) 2-Chloro-5-{[(cyclopropylsulfonyl)amino]methyl}-N-[1-(2-methoxypyridin-4-yl)-1H-indazol-4-yl]benzamide,
(166) 2-Chloro-5-{[(3,3-dimethylbutanoyl)amino]methyl)-N-{1-[5-(trifluoromethyl)pyridin-3-yl]-1H-indazol-4-yl}benzamide,
(167) 2-Chloro-5-{[(cyclopentylcarbonyl)amino]methyl}-N-{1-[5-(trifluoromethyl)pyridin-3-yl]-1H-indazol-4-yl}benzamide,
(168) 2-Chloro-5-[({[1-(trifluoromethyl)cyclopropyl]carbonyl}amino)methyl]-N-{1-[5-(trifluoromethyl)pyridin-3-yl]-1H-indazol-4-yl}benzamide,
(169) 2-Chloro-5-({[(1-hydroxycyclopropyl)carbonyl]amino}methyl)-N-{1-[5-(trifluoromethyl)pyridin-3-yl]-1H-indazol-4-yl}benzamide,
(170) 2-Chloro-5-{[(methoxyacetyl)amino]methyl}-N-{1-[5-(trifluoromethyl)pyridin-3-yl]-1H-indazol-4-yl} benzamide,
(171) 2-Chloro-5-{[(3-hydroxy-2,2-dimethylpropanoyl)amino]methyl}-N-{1-[5-(trifluoromethyl)pyridin-3-yl]-1H-indazol-4-yl}benzamide,
(172) 2-Chloro-5-{[(cyclopropylsulfonyl)amino]methyl}-N-{1-[5-(trifluoromethyl)pyridin-3-yl]-1H-indazol-4-yl}benzamide,
(173) 2-Chloro-5-{[(3,3-dimethylbutanoyl)amino]methyl}-N-{1-[6-(trifluoromethyl)pyridin-3-yl]-1H-indazol-4-yl} benzamide,
(174) 2-Chloro-5-{[(cyclopentylcarbonyl)amino]methyl}-N-{1-[6-(trifluoromethyl)pyridin-3-yl]-1H-indazol-4-yl}benzamide,
(175) 2-Chloro-5-[({[1-(trifluoromethyl)cyclopropyl]carbonyl}amino)methyl]-N-{1-[6-(trifluoromethyl)pyridin-3-yl]-1H-indazol-4-yl}benzamide,
(176) 2-Chloro-5-({[(1-hydroxycyclopropyl)carbonyl]amino}methyl)-N-{1-[6-(trifluoromethyl)pyridin-3-yl]-1H-indazol-4-yl}benzamide,
(177) 2-Chloro-5-{[(methoxyacetyl)amino]methyl}-N-{1-[6-(trifluoromethyl)pyridin-3-yl]-1H-indazol-4-yl}benzamide,
(178) 2-Chloro-5-{[(3-hydroxy-2,2-dimethylpropanoyl)amino]methyl)-N-{1-[6-(trifluoromethyl)pyridin-3-yl]-1H-indazol-4-yl}benzamide,
(179) 2-Chloro-5-{[(cyclopropylsulfonyl)amino]methyl}-N-{1-[6-(trifluoromethyl)pyridin-3-yl]-1H-indazol-4-yl}benzamide,
(180) 2-Chloro-5-{[(cyclopentylcarbonyl)amino]methyl}-N-[1-(propan-2-yl)-1H-indazol-4-yl]benzamide,
(181) 2-Chloro-N-[1-(propan-2-yl)-1H-indazol-4-yl]-5-[({[1-(trifluoromethyl)cyclopropyl]carbonyl}amino)methyl]benzamide,
(182) 2-Chloro-5-{[(methoxyacetyl)amino]methyl}-N-[1-(propan-2-yl)-1H-indazol-4-yl]benzamide,
(183) 2-Chloro-5-{[(3-hydroxy-2,2-dimethylpropanoyl)amino]methyl}-N-[1-(propan-2-yl)-1H-indazol-4-yl]benzamide,
(184) 2-Chloro-5-{[(cyclopropylsulfonyl)amino]methyl}-N-[1-(propan-2-yl)-1H-indazol-4-yl]benzamide,
(185) 2-Chloro-5-({[(1-hydroxycyclopropyl)carbonyl]amino}methyl)-N-[1-(propan-2-yl)-1H-indazol-4-yl]benzamide,
(186) 2-Chloro-5-{[(3,3-dimethylbutanoyl)amino]methyl}-N-[1-(propan-2-yl)-1H-indazol-4-yl]benzamide,
(187) 2-Chloro-5-{[(3,3-dimethylbutanoyl)amino]methyl}-N-[1-(1,3-thiazol-4-yl)-1H-indazol-4-yl]benzamide,
(188) 2-Chloro-5-{[(cyclopentylcarbonyl)amino]methyl}-N-[1-(1,3-thiazol-4-yl)-1H-indazol-4-yl]benzamide,
(189) 2-Chloro-N-[1-(1,3-thiazol-4-yl)-1H-indazol-4-yl]-5-[({[1-(trifluoromethyl)cyclopropyl]carbonyl}amino)methyl]benzamide,
(190) 2-Chloro-5-({[(1-hydroxycyclopropyl)carbonyl]amino}methyl)-N-[1-(1,3-thiazol-4-yl)-1H-indazol-4-yl]benzamide,
(191) 2-Chloro-5-{[(methoxyacetyl)amino]methyl}-N-[1-(1,3-thiazol-4-yl)-1H-indazol-4-yl]benzamide,
(192) 2-Chloro-5-{[(3-hydroxy-2,2-dimethylpropanoyl)amino]methyl}-N-[1-(1,3-thiazol-4-yl)-1H-indazol-4-yl]benzamide,
(193) 2-Chloro-5-{[(cyclopropylsulfonyl)amino]methyl}-N-[1-(1,3-thiazol-4-yl)-1H-indazol-4-yl]benzamide,
(194) 5-{[(2,2-Dimethylpropanoyl)amino]methyl}-N-{1-[4-(trifluoromethoxy)phenyl]-1H-indazol-4-yl}-2-(trifluoromethyl)benzamide,
(195) 5-{[(2,2-Dimethylpropanoyl)amino]methyl}-N-{1-[3-(trifluoromethoxy)phenyl]-1H-indazol-4-yl}-2-(trifluoromethyl)benzamide,
(196) 5-{[(2,2-Dimethylpropanoyl)amino]methyl}-2-(trifluoromethyl)-N-{1-[6-(trifluoromethyl)pyridin-3-yl]-1H-indazol-4-yl}benzamide,
(197) 5-{[(2,2-Dimethylpropanoyl)amino]methyl}-N-[1-(2-methoxypyridin-4-yl)-1H-indazol-4-yl]-2-(trifluoromethyl)benzamide,
(198) N-[1-(4-chlorophenyl)-1H-indazol-4-yl]-5-{[(2,2-dimethylpropanoyl)amino]methyl}-2-(trifluoromethyl)benzamide,
(199) 5-{[(2,2-Dimethylpropanoyl)amino]methyl}-N-{1-[3-methyl-4-(trifluoromethoxy)phenyl]-1H-indazol-4-yl}-2-(trifluoromethyl)benzamide,
(200) N-[1-(3-chloro-4-methylphenyl)-1H-indazol-4-yl]-5-{[(2,2-dimethylpropanoyl)amino]methyl}-2-(trifluoromethyl)benzamide,
(201) 5-{[(2,2-Dimethylpropanoyl)amino]methyl}-N-[1-(1,3-thiazol-4-yl)-1H-indazol-4-yl]-2-(trifluoromethyl)benzamide,
(202) 5-{[(2,2-Dimethylpropanoyl)amino]methyl}-N-[1-(propan-2-yl)-1H-indazol-4-yl]-2-(trifluoromethyl)benzamide,
(203) 2-Chloro-5-{[(3,3-dimethylbutanoyl)amino]methyl}-N-(1-methyl-1H-indazol-4-yl)benzamide,
(204) 2-Chloro-5-{[(cyclopentylcarbonyl)amino]methyl}-N-(1-methyl-1H-indazol-4-yl)benzamide,
(205) 2-Chloro-N-(1-methyl-1H-indazol-4-yl)-5-[({[1-(trifluoromethyl)cyclopropyl]carbonyl} amino)methyl]benzamide,
(206) 2-Chloro-5-({[(1-hydroxycyclopropyl)carbonyl]amino}methyl)-N-(1-methyl-1H-indazol-4-yl)benzamide,
(207) 2-Chloro-5-{[(methoxyacetyl)amino]methyl}-N-(1-methyl-1H-indazol-4-yl)benzamide,
(208) 2-Chloro-5-{[(cyclopropylsulfonyl)amino]methyl}-N-(1-methyl-1H-indazol-4-yl)benzamide,
(209) 5-{[(2,2-Dimethylpropanoyl)amino]methyl}-N-(1-methyl-1H-indazol-4-yl)-2-(trifluoromethyl)benzamide,
(210) 2-Chloro-5-{[(3,3-dimethylbutanoyl)amino]methyl}-N-(1-ethyl-1H-indazol-4-yl)benzamide,
(211) 2-Chloro-5-{[(cyclopentylcarbonyl)amino]methyl}-N-(1-ethyl-1H-indazol-4-yl)benzamide,
(212) 2-Chloro-N-(1-ethyl-1H-indazol-4-yl)-5-[({[1-(trifluoromethyl)cyclopropyl]carbonyl} amino)methyl]benzamide,
(213) 2-Chloro-N-(1-ethyl-1H-indazol-4-yl)-5-({[(1-hydroxycyclopropyl)carbonyl]amino}methyl)benzamide,
(214) 2-Chloro-N-(1-ethyl-1H-indazol-4-yl)-5-{[(methoxyacetyl)amino]methyl}benzamide,
(215) 2-Chloro-N-(1-ethyl- 1H-indazol-4-yl)-5-{[(3-hydroxy-2,2-dimethylpropanoyl)amino]methyl}benzamide,
(216) 2-Chloro-5-{[(cyclopropylsulfonyl)amino]methyl}-N-(1-ethyl-1H-indazol-4-yl)benzamide,
(217) 5-{[(2,2-Dimethylpropanoyl)amino]methyl}-N-(1-ethyl-1H-indazol-4-yl)-2-(trifluoromethyl)benzamide,
(218) 2-Chloro-5-{[(3,3-dimethylbutanoyl)amino]methyl}-N-{1-[3-(trifluoromethyl)phenyl]-1H-indazol-4-yl}benzamide,
(219) 2-Chloro-5-{[(cyclopentylcarbonyl)amino]methyl}-N-{1-[3-(trifluoromethyl)phenyl]-1H-indazol-4-yl}benzamide,
(220) 2-Chloro-5-[({[1-(trifluoromethyl)cyclopropyl]carbonyl}amino)methyl]-N-{1-[3-(trifluoromethyl)phenyl]-1H-indazol-4-yl}benzamide,
(221) 2-Chloro-5-({[(1-hydroxycyclopropyl)carbonyl]amino}methyl)-N-{1-[3-(trifluoromethyl)phenyl]-1H-indazol-4-yl}benzamide,
(222) 2-Chloro-5-{[(methoxyacetyl)amino]methyl}-N-{1-[3-(trifluoromethyl)phenyl]-1H-indazol-4-yl}benzamide,
(223) 2-Chloro-5-{[(3-hydroxy-2,2-dimethylpropanoyl)amino]methyl}-N-{1-[3-(trifluoromethyl)phenyl]-1H-indazol-4-yl}benzamide,
(224) 2-Chloro-5-{[(cyclopropylsulfonyl)amino]methyl}-N-{1-[3-(trifluoromethyl)phenyl]-1H-indazol-4-yl}benzamide,
(225) 5-{[(2,2-Dimethylpropanoyl)amino]methyl}-2-(trifluoromethyl)-N-{1-[3-(trifluoromethyl)phenyl]-1H-indazol-4-yl}benzamide,
(226) 2-Chloro-5-{[(3,3-dimethylbutanoyl)amino]methyl}-N-[1-(1,3-thiazol-2-yl)-1H-indazol-4-yl]benzamide,
(227) 2-Chloro-5-{[(cyclopentylcarbonyl)amino]methyl}-N-[1-(1,3-thiazol-2-yl)-1H-indazol-4-yl]benzamide,
(228) 2-Chloro-N-[1-(1,3-thiazol-2-yl)-1H-indazol-4-yl]-5-[({[1-(trifluoromethyl)cyclopropyl]carbonyl}amino)methyl]benzamide,
(229) 2-Chloro-5-({[(1-hydroxycyclopropyl)carbonyl]amino}methyl)-N-[1-(1,3-thiazol-2-yl)-1H-indazol-4-yl]benzamide,
(230) 2-Chloro-5-{[(methoxyacetyl)amino]methyl}-N-[1-(1,3-thiazol-2-yl)-1H-indazol-4-yl]benzamide,
(231) 2-Chloro-5-{[(3-hydroxy-2,2-dimethylpropanoyl)amino]methyl}-N-[1-(1,3-thiazol-2-yl)-1H-indazol-4-yl]benzamide,
(232) 2-Chloro-5-{[(cyclopropylsulfonyl)amino]methyl}-N-[1-(1,3-thiazol-2-yl)-1H-indazol-4-yl]benzamide,
(233) 5-{[(2,2-Dimethylpropanoyl)amino]methyl}-N-[1-(1,3-thiazol-2-yl)-1H-indazol-4-yl]-2-(trifluoromethyl)benzamide,
(234) 2-Chloro-5-{[(3,3-dimethylbutanoyl)amino]methyl}-N-[1-(2-methylpyridin-4-yl)-1H-indazol-4-yl]benzamide,
(235) 2-Chloro-5-{[(cyclopentylcarbonyl)amino]methyl}-N-[1-(2-methylpyridin-4-yl)-1H-indazol-4-yl]benzamide,
(236) 2-Chloro-5-{[(methoxyacetyl)amino]methyl}-N-[1-(2-methylpyridin-4-yl)-1H-indazol-4-yl]benzamide,
(237) 2-Chloro-5-{[(cyclopropylsulfonyl)amino]methyl}-N-[1-(2-methylpyridin-4-yl)-1H-indazol-4-yl]benzamide,
(238) 5-{[(2,2-Dimethylpropanoyl)amino]methyl}-N-[1-(2-methylpyridin-4-yl)-1H-indazol-4-yl]-2-(trifluoromethyl)benzamide,
(239) 2-Chloro-5-{[(3,3-dimethylbutanoyl)amino]methyl}-N-[1-(5-methylpyridin-3-yl)-1H-indazol-4-yl]benzamide,
(240) 2-Chloro-5-{[(cyclopentylcarbonyl)amino]methyl}-N-[1-(5-methylpyridin-3-yl)-1H-indazol-4-yl]benzamide,
(241) 2-Chloro-N-[1-(5-methylpyridin-3-yl)-1H-indazol-4-yl]-5-[({[1-(trifluoromethyl)cyclopropyl]carbonyl}amino)methyl]benzamide,
(242) 2-Chloro-5-({[(1-hydroxycyclopropyl)carbonyl]amino}methyl)-N-[1-(5-methylpyridin-3-yl)-1H-indazol-4-yl]benzamide,
(243) 2-Chloro-5-{[(methoxyacetyl)amino]methyl}-N-[1-(5-methylpyridin-3-yl)-1H-indazol-4-yl]benzamide,
(244) 2-Chloro-5-{[(3-hydroxy-2,2-dimethylpropanoyl)amino]methyl}-N-[1-(5-methylpyridin-3-yl)-1H-indazol-4-yl]benzamide,
(245) 2-Chloro-5-{[(cyclopropylsulfonyl)amino]methyl}-N-[1-(5-methylpyridin-3-yl)-1H-indazol-4-yl]benzamide,
(246) 5-{[(2,2-Dimethylpropanoyl)amino]methyl}-N-[1-(5-methylpyridin-3-yl)-1H-indazol-4-yl]-2-(trifluoromethyl)benzamide,
(247) 2-Chloro-5-{[(3,3-dimethylbutanoyl)amino]methyl}-N-[1-(6-methylpyridazin-3-yl)-1H-indazol-4-yl]benzamide,
(248) 2-Chloro-5-{[(cyclopentylcarbonyl)amino]methyl}-N-[1-(6-methylpyridazin-3-yl)-1H-indazol-4-yl]benzamide,
(249) 2-Chloro-N-[1-(6-methylpyridazin-3-yl)-1H-indazol-4-yl]-5-[({[1-(trifluoromethyl)cyclopropyl]carbonyl}amino)methyl]benzamide,
(250) 2-Chloro-5-({[(1-hydroxycyclopropyl)carbonyl]amino}methyl)-N-[1-(6-methylpyridazin-3-yl)-1H-indazol-4-yl]benzamide,
(251) 2-Chloro-5-{[(methoxyacetyl)amino]methyl}-N-[1-(6-methylpyridazin-3-yl)-1H-indazol-4-yl]benzamide,
(252) 2-Chloro-5-{[(3-hydroxy-2,2-dimethylpropanoyl)amino]methyl}-N-[1-(6-methylpyridazin-3-yl)-1H-indazol-4-yl]benzamide,
(253) 2-Chloro-5-{[(cyclopropylsulfonyl)amino]methyl}-N-[1-(6-methylpyridazin-3-yl)-1H-indazol-4-yl]benzamide,
(254) 2-Chloro-5-{[(3,3-dimethylbutanoyl)amino]methyl}-N-{1-[4-methyl-3-(trifluoromethyl)phenyl]-1H-indazol-4-yl}benzamide,
(255) 2-Chloro-5-{[(cyclopentylcarbonyl)amino]methyl}-N-{1-[4-methyl-3-(trifluoromethyl)phenyl]-1H-indazol-4-yl}benzamide,
(256) 2-Chloro-N-{1-[4-methyl-3-(trifluoromethyl)phenyl]-1H-indazol-4-yl}-5-[({[1-(trifluoromethyl)cyclopropyl]carbonyl} amino)methyl]benzamide,
(257) 2-Chloro-5-({[(1-hydroxycyclopropyl)carbonyl]amino}methyl)-N-{1-[4-methyl-3-(trifluoromethyl)phenyl]-1H-indazol-4-yl}benzamide,
(258) 2-Chloro-5-{[(methoxyacetyl)amino]methyl}-N-{1-[4-methyl-3-(trifluoromethyl)phenyl]-1H-indazol-4-yl}benzamide,
(259) 2-Chloro-5-{[(3-hydroxy-2,2-dimethylpropanoyl)amino]methyl}-N-{1-[4-methyl-3-(trifluoromethyl)phenyl]-1H-indazol-4-yl}benzamide,
(260) 2-Chloro-5-{[(cyclopropylsulfonyl)amino]methyl}-N-{1-[4-methyl-3-(trifluoromethyl)phenyl]-1H-indazol-4-yl}benzamide,
(261) 2-Chloro-5-{[(3,3-dimethylbutanoyl)amino]methyl}-N-{1-[4-methoxy-3-(trifluoromethyl)phenyl]-1H-indazol-4-yl}benzamide,
(262) 2-Chloro-5-{[(cyclopentylcarbonyl)amino]methyl}-N-{1-[4-methoxy-3-(trifluoromethyl)phenyl]-1H-indazol-4-yl}benzamide,
(263) 2-Chloro-N-{1-[4-methoxy-3-(trifluoromethyl)phenyl]-1H-indazol-4-yl}-5-[({[1-(trifluoromethyl)cyclopropyl]carbonyl}amino)methyl]benzamide,
(264) 2-Chloro-5-({[(1-hydroxycyclopropyl)carbonyl]amino}methyl)-N-{1-[4-methoxy-3-(trifluoromethyl)phenyl]-1H-indazol-4-yl}benzamide,
(265) 2-Chloro-5-{[(methoxyacetyl)amino]methyl}-N-{1-[4-methoxy-3-(trifluoromethyl)phenyl]-1H-indazol-4-yl}benzamide,
(266) 2-Chloro-5-{[(3-hydroxy-2,2-dimethylpropanoyl)amino]methyll-N-11-[4-methoxy-3-(trifluoromethyl)phenyl]-1H-indazol-4-yl}benzamide,
(267) 2-Chloro-5-{[(cyclopropylsulfonyl)amino]methyl}-N-{1-[4-methoxy-3-(trifluoromethyl)phenyl]-1H-indazol-4-yl}benzamide,
(268) 5-{[(2,2-Dimethylpropanoyl)amino]methyl}-N-{1-[4-methoxy-3-(trifluoromethyl)phenyl]-1H-indazol-4-yl}-2-(trifluoromethyl)benzamide,
(269) 2-Chloro-5-{[(3,3-dimethylbutanoyl)amino]methyl}-N-[1-(6-methoxypyridin-3-yl)-1H-indazol-4-yl]benzamide,
(270) 2-Chloro-5-{[(cyclopentylcarbonyl)amino]methyl}-N-[1-(6-methoxypyridin-3-yl)-1H-indazol-4-yl]benzamide,
(271) 2-Chloro-N-[1-(6-methoxypyridin-3-yl)-1H-indazol-4-yl]-5-[({[1-(trifluoromethyl)cyclopropyl]carbonyl} amino)methyl]benzamide,
(272) 2-Chloro-5-({[(1-hydroxycyclopropyl)carbonyl]amino}methyl)-N-[1-(6-methoxypyridin-3-yl)-1H-indazol-4-yl]benzamide,
(273) 2-Chloro-5-{[(methoxyacetyl)amino]methyl}-N-[1-(6-methoxypyridin-3-yl)-1H-indazol-4-yl]benzamide,
(274) 2-Chloro-5-{[(3-hydroxy-2,2-dimethylpropanoyl)amino]methyl}-N-[1-(6-methoxypyridin-3-yl)-1H-indazol-4-yl]benzamide,
(275) 2-Chloro-5-{[(cyclopropylsulfonyl)amino]methyl}-N-[1-(6-methoxypyridin-3-yl)-1H-indazol-4-yl]benzamide,
(276) 5-{[(2,2-Dimethylpropanoyl)amino]methyl}-N-[1-(6-methoxypyridin-3-yl)-1H-indazol-4-yl]-2-(trifluoromethyl)benzamide,
(277) 2-Chloro-5-{[(3,3-dimethylbutanoyl)amino]methyl}-N-[1-(2,6-dimethylpyridin-4-yl)-1H-indazol-4-yl]benzamide,
(278) 2-Chloro-5-{[(cyclopentylcarbonyl)amino]methyl}-N-[1-(2,6-dimethylpyridin-4-yl)-1H-indazol-4-yl]benzamide,
(279) 2-Chloro-N-[1-(2,6-dimethylpyridin-4-yl)-1H-indazol-4-yl]-5-[({[1-(trifluoromethyl)cyclopropyl]carbonyl}amino)methyl]benzamide,
(280) 2-Chloro-N-[1-(2,6-dimethylpyridin-4-yl)-1H-indazol-4-yl]-5-{[(methoxyacetyl)amino]methyl}benzamide,
(281) 2-Chloro-N-[1-(2,6-dimethylpyridin-4-yl)-1H-indazol-4-yl]-5-{[(3-hydroxy-2,2-dimethylpropanoyl)amino]methyl}benzamide,
(282) 2-Chloro-5-{[(cyclopropylsulfonyl)amino]methyl}-N-[1-(2,6-dimethylpyridin-4-yl)-1H-indazol-4-yl]benzamide,
(283) 5-{[(2,2-Dimethylpropanoyl)amino]methyl}-N-[1-(2,6-dimethylpyridin-4-yl)-1H-indazol-4-yl]-2-(trifluoromethyl)benzamide,
(284) 2-Chloro-5-{[(2,2-dimethylpropanoyl)amino]methyl}-N-(1-cyclopropyl-1H-indazol-4-yl)benzamide,
(285) 5-{[(2,2-Dimethylpropanoyl)amino]methyl)-N-(1-cyclopropyl-1H-indazol-4-yl)-2-(trifluoromethyl)benzamide,
(286) 2,6-Dichloro-3-{[(2,2-dimethylpropanoyl)amino]methyl}-N-{1-[4-(trifluoromethoxy)phenyl]-1H-indazol-4-yl}benzamide,
(287) 2-Chloro-5-({[(1-hydroxycyclopropyl)carbonyl]amino}methyl)-N-{1-[3-(trifluoromethoxy)phenyl]-1H-indazol-4-yl}benzamide,
(288) 2,6-Dichloro-3-{[(2,2-dimethylpropanoyl)amino]methyl}-N-{1-[3-(trifluoromethoxy)phenyl]-1H-indazol-4-yl}benzamide,
(289) 2-Chloro-N-[1-(3-chloro-4-methylphenyl)-1H-indazol-4-yl]-5-{[(3-hydroxy-2,2-dimethylpropanoyl)amino]methyl}benzamide,
(290) 2,6-Dichloro-N-[1-(3-chloro-4-methylphenyl)-1H-indazol-4-yl]-3-{[(2,2-dimethylpropanoyl)amino]methyl} benzamide,
(291) 2-Chloro-N-{1-[3-methyl-4-(trifluoromethoxy)phenyl]-1H-indazol-4-yl}-5-[(1[1-(trifluoromethyl)cyclopropyl]carbonyl}amino)methyl]benzamide,
(292) 2-Chloro-5-({[(1-hydroxycyclopropyl)carbonyl]amino}methyl)-N-{1-[3-methyl-4-(trifluoromethoxy)phenyl]-1H-indazol-4-yl}benzamide,
(293) 2,6-Dichloro-3-1[(2,2-dimethylpropanoyl)amino]methyl}-N-{1-[3-methyl-4-(trifluoromethoxy)phenyl]-1H-indazol-4-yl}benzamide,
(294) 2,6-Dichloro-N-{ 1-[3-(difluoromethoxy)phenyl]-1H-indazol-4-yl}-3-{[(2,2-dimethylpropanoyl)amino]methyl} benzamide,
(295) 2-Chloro-N-[1-(4-chlorophenyl)-1H-indazol-4-yl]-5-({[(1-hydroxycyclopropyl)carbonyl]amino}methyl)benzamide,
(296) 2,6-Dichloro-N-[1-(4-chlorophenyl)-1H-indazol-4-yl]-3-{[(2,2-dimethylpropanoyl)amino]methyl}benzamide,
(297) 2,6-Dichloro-3-{[(2,2-dimethylpropanoyl)amino]methyl}-N-[1-(2-methoxypyridin-4-yl)-1H-indazol-4-yl]benzamide,
(298) 2,6-Dichloro-3-{[(2,2-dimethylpropanoyl)amino]methyl}-N-{1-[5-(trifluoromethyl)pyridin-3-yl]-1H-indazol-4-yl}benzamide,
(299) 2,6-Dichloro-3-{[(2,2-dimethylpropanoyl)amino]methyl}-N-{1-[6-(trifluoromethyl)pyridin-3-yl]-1H-indazol-4-yl}benzamide,
(300) 2,6-Dichloro-3-{[(2,2-dimethylpropanoyl)amino]methyl}-N-[1-(propan-2-yl)-1H-indazol-4-yl]benzamide,
(301) 2,6-Dichloro-3-{[(2,2-dimethylpropanoyl)amino]methyl}-N-[1-(1,3-thiazol-4-yl)-1H-indazol-4-yl]benzamide,
(302) 5-{[(2,2-Dimethylpropanoyl)amino]methyl}-2-(trifluoromethyl)-N-{1-[5-(trifluoromethyl)pyridin-3-yl]-1H-indazol-4-yl}benzamide,
(303) 5-{[(2,2-Dimethylpropanoyl)amino]methyl}-N-[1-(4-fluorophenyl)-1H-indazol-4-yl]-2-(trifluoromethyl)benzamide,
(304) N-{1-[3-(Difluoromethoxy)phenyl]-1H-indazol-4-yl}-5-{[(2,2-dimethylpropanoyl)amino]methyl}-2-(trifluoromethyl)benzamide,
(305) 2-Chloro-5-{[(3-hydroxy-2,2-dimethylpropanoyl)amino]methyl}-N-(1-methyl-1H-indazol-4-yl)benzamide,
(306) 2,6-Dichloro-3-{[(2,2-dimethylpropanoyl)amino]methyl}-N-(1-methyl-1H-indazol-4-yl)benzamide,
(307) 2,6-Dichloro-3-{[(2,2-dimethylpropanoyl)amino]methyl}-N-(1-ethyl-1H-indazol-4-yl)benzamide,
(308) 2,6-Dichloro-3-{[(2,2-dimethylpropanoyl)amino]methyl}-N-{1-[3-(trifluoromethyl)phenyl]-1H-indazol-4-yl}benzamide,
(309) 2-Chloro-5-{[(3,3-dimethylbutanoyl)amino]methyl}-N-{1-[2-methyl-4-(trifluoromethoxy)phenyl]-1H-indazol-4-yl}benzamide,
(310) 2-Chloro-5-{[(cyclopentylcarbonyl)amino]methyl}-N-{1-[2-methyl-4-(trifluoromethoxy)phenyl]-1H-indazol-4-yl}benzamide,
(311) 2-Chloro-N-{1-[2-methyl-4-(trifluoromethoxy)phenyl]-1H-indazol-4-yl}-5-[({[1-(trifluoromethyl)cyclopropyl]carbonyl} amino)methyl]benzamide,
(312) 2-Chloro-5-({[(1-hydroxycyclopropyl)carbonyl]amino}methyl)-N-{1-[2-methyl-4-(trifluoromethoxy)phenyl]-1H-indazol-4-yl}benzamide,
(313) 2-Chloro-5-{[(methoxyacetyl)amino]methyll-N-11-[2-methyl-4-(trifluoromethoxy)phenyl]-1H-indazol-4-yl}benzamide,
(314) 2-Chloro-5-{[(3-hydroxy-2,2-dimethylpropanoyl)amino]methyl}-N-{1-[2-methyl-4-(trifluoromethoxy)phenyl]-1H-indazol-4-yl}benzamide,
(315) 2-Chloro-5-{[(cyclopropylsulfonyl)amino]methyl}-N-{1-[2-methyl-4-(trifluoromethoxy)phenyl]-1H-indazol-4-yl}benzamide,
(316) 2,6-Dichloro-3-{[(2,2-dimethylpropanoyl)amino]methyl}-N-{1-[2-methyl-4-(trifluoromethoxy)phenyl]-1H-indazol-4-yl}benzamide,
(317) 5-{[(2,2-Dimethylpropanoyl)amino]methyl)-N-11-[2-methyl-4-(trifluoromethoxy)phenyl]-1H-indazol-4-yl}-2-(trifluoromethyl)benzamide,
(318) 2,6-Dichloro-3-{[(2,2-dimethylpropanoyl)amino]methyl}-N-[1-(1,3-thiazol-2-yl)-1H-indazol-4-yl]benzamide,
(319) 2-Chloro-N-[1-(2-methylpyridin-4-yl)-1H-indazol-4-yl]-5-[({[1-(trifluoromethyl)cyclopropyl]carbonyl}amino)methyl]benzamide,
(320) 2-Chloro-5-({[(1-hydroxycyclopropyl)carbonyl]amino}methyl)-N-[1-(2-methylpyridin-4-yl)-1H-indazol-4-yl]benzamide,
(321) 2-Chloro-5-{[(3-hydroxy-2,2-dimethylpropanoyl)amino]methyl}-N-[1-(2-methylpyridin-4-yl)-1H-indazol-4-yl]benzamide-4-yl]benzamide,
(322) 2,6-Dichloro-3-{[(2,2-dimethylpropanoyl)amino]methyl}-N-[1-(2-methylpyridin-4-yl)-1H-indazol-4-yl]benzamide,
(323) 2,6-Dichloro-3-{[(2,2-dimethylpropanoyl)amino]methyl}-N-[1-(5-methylpyridin-3-yl)-1H-indazol-4-yl]benzamide,
(324) 2,6-Dichloro-3-{[(2,2-dimethylpropanoyl)amino]methyl}-N-[1-(6-methylpyridazin-3-yl)-1H-indazol-4-yl]benzamide,
(325) 5-{[(2,2-Dimethylpropanoyl)amino]methyl}-N-[1-(6-methylpyridazin-3-yl)-1H-indazol-4-yl]-2-(trifluoromethyl)benzamide,
(326) 2,6-Dichloro-3-{[(2,2-dimethylpropanoyl)amino]methyl}-N-{1-[4-methyl-3-(trifluoromethyl)phenyl]-1H-indazol-4-yl}benzamide,
(327) 5-{[(2,2-Dimethylpropanoyl)amino]methyl}-N-{1-[4-methyl-3-(trifluoromethyl)phenyl]-1H-indazol-4-yl}-2-(trifluoromethyl)benzamide,
(328) 2,6-Dichloro-3-{[(2,2-dimethylpropanoyl)amino]methyl}-N-{1-[4-methoxy-3-(trifluoromethyl)phenyl]-1H-indazol-4-yl}benzamide,
(329) 2,6-Dichloro-3-{[(2,2-dimethylpropanoyl)amino]methyl}-N-[1-(6-methoxypyridin-3-yl)-1H-indazol-4-yl]benzamide,
(330) 2-Chloro-N-[1-(2,6-dimethylpyridin-4-yl)-1H-indazol-4-yl]-5-({[(1-hydroxycyclopropyl)carbonyl]amino}methyl)benzamide,
(331) 2,6-Dichloro-3-{[(2,2-dimethylpropanoyl)amino]methyl}-N-[1-(2,6-dimethylpyridin-4-yl)-1H-indazol-4-yl]benzamide,
(332) 2-Chloro-N-[1-(2-cyclopropylpyridin-4-yl)-1H-indazol-4-yl]-5-{[(3,3-dimethylbutanoyl)amino]methyl}benzamide,
(333) 2-Chloro-5-{[(cyclopentylcarbonyl)amino]methyl}-N-[1-(2-cyclopropylpyridin-4-yl)-1H-indazol-4-yl]benzamide,
(334) 2-Chloro-N-[1-(2-cyclopropylpyridin-4-yl)-1H-indazol-4-yl]-5-[({[1-(trifluoromethyl)cyclopropyl]carbonyl}amino)methyl]benzamide,
(335) 2-Chloro-N-[1-(2-cyclopropylpyridin-4-yl)-1H-indazol-4-yl]-5-({[(1-hydroxycyclopropyl)carbonyl]amino}methyl)benzamide,
(336) 2-Chloro-N-[1-(2-cyclopropylpyridin-4-yl)-1H-indazol-4-yl]-5-{[(methoxyacetyl)amino]methyl} benzamide,
(337) 2-Chloro-N-[1-(2-cyclopropylpyridin-4-yl)-1H-indazol-4-yl]-5-{[(3-hydroxy-2,2-dimethylpropanoyl)amino]methyl} benzamide,
(338) 2-Chloro-N-[1-(2-cyclopropylpyridin-4-yl)-1H-indazol-4-yl]-5-{[(cyclopropylsulfonyl)amino]methyl}benzamide,
(339) 2,6-Dichloro-N-[1-(2-cyclopropylpyridin-4-yl)-1H-indazol-4-yl]-3-{[(2,2-dimethylpropanoyl)amino]methyl}benzamide, and
(340) N-[1-(2-Cyclopropylpyridin-4-yl)-1H-indazol-4-yl]-5-{[(2,2-dimethylpropanoyl)amino]methyl}-2-(trifluoromethyl)benzamide,
or a pharmaceutically acceptable salt thereof, or a solvate thereof.

21. A pharmaceutical composition comprising the compound of any one of claims 1 to 20, or a pharmaceutically acceptable salt thereof, or a solvate thereof.

22. An inhibitory agent for mPGES-1, comprising the compound of any one of claims 1 to 20, or a pharmaceutically acceptable salt thereof, or a solvate thereof.

23. An inhibitory agent for the production of PGE2, comprising the compound of any one of claims 1 to 20, or a pharmaceutically acceptable salt thereof, or a solvate thereof.

24. A preventive or therapeutic agent for diseases involving mPGES-1, comprising the compound of any one of claims 1 to 20, or a pharmaceutically acceptable salt thereof, or a solvate thereof.

25. A preventive or therapeutic agent for diseases involving PGE2, comprising the compound of any one of claims 1 to 20, or a pharmaceutically acceptable salt thereof, or a solvate thereof.

26. A preventive or therapeutic agent comprising the compound according to any one of claims 1 to 20 or a pharmaceutically acceptable salt thereof, or a solvate thereof, wherein the agent is used for preventing or treating inflammatory bowel disease, irritable bowel syndrome, migraine, headache, lumbago, lumbar spinal stenosis, herniated disc, temporomandibular joint disorder, cervico-omo-brachial syndrome, cervical spondylosis, endometriosis, adenomyosis, premature labor, threatened premature labor, dysmenorrhea, overactive bladder, nocturia, interstitial cystitis, neurodegenerative disease, psoriasis, rheumatoid arthritis, rheumatic fever, fibromyalgia, neuralgia, complex regional pain syndrome, myofascial disorder, viral infection, bacterial infection, fungal infection, burn injury, postoperative, post-trauma, and post-tooth extraction inflammation and pain, malignant tumor, atherosclerosis, stroke, gout, osteoarthritis, juvenile idiopathic arthritis, tenosynovitis, ligament ossification, systemic lupus erythematosus, vasculitis, pancreatitis, nephritis, conjunctivitis, iritis, scleritis, uveitis, wound healing, dermatitis, eczema, osteoporosis, asthma, chronic obstructive pulmonary disease, pulmonary fibrosis, allergic diseases, familial adenomatous polyposis, scleroderma, bursitis, uterine fibroids, prostatitis, depression, neurogenic bladder, nocturnal polyuria, diurnal polyuria, nocturnal enuresis, central diabetes insipidus, nephrogenic diabetes insipidus, urinary incontinence, prostatic hyperplasia, chronic prostatitis, or pain in cancer.

27. A preventive or therapeutic agent comprising the compound according to any one of claims 1 to 20, or a pharmaceutically acceptable salt thereof, or a solvate thereof, wherein the agent is used for preventing or treating nocturnal polyuria, nocturia, overactive bladder, neurogenic bladder, nocturnal enuresis, prostatic hyperplasia, central diabetes insipidus, nephrogenic diabetes insipidus, chronic prostatitis, interstitial cystitis, or urinary incontinence.
